# EUROPEAN PATENT SPECIFICATION

(11) **EP 2 554 672 B1**
(45) Date of publication and mention of the grant of the patent: **19.11.2014**
(21) Application number: 11765752.8
(22) Date of filing: 31.03.2011
(51) Int. Cl.: C12N 15/09, C12N 15/115, C12Q 1/68, G01N 33/50, G01N 33/53, C12N 15/10

(54) **NUCLEIC ACID STRUCTURE, METHOD FOR PRODUCING COMPLEX USING SAME, AND SCREENING METHOD**
NUKLEINSÄURESTRUKTUR, VERFAHREN ZUR HERSTELLUNG EINES KOMPLEXES DAMIT UND SCREENINGVERFAHREN DAFÜR
STRUCTURE D'ACIDE NUCLÉIQUE, PROCÉDÉ POUR PRODUIRE UN COMPLEXE L'EMPLOYANT ET PROCÉDÉ DE CRIBLAGE

(30) Priority: 01.04.2010 JP 2010085545
(43) Date of publication of application: 06.02.2013
(73) Proprietor: NEC Solution Innovators, Ltd., Tokyo 136-8627 (JP); Tsuji, Shotaro, Tokyo 157-0072 (JP); Tsuji, Makiko, Tokyo 157-0072 (JP); Ohtsu, Takashi, Kanagawa 227-0055 (JP)
(72) Inventor: TSUJI Shotaro, Tokyo 157-0072 (JP); TSUJI Makiko, Tokyo 157-0072 (JP); OHTSU Takashi, Yokohama-shi Kanagawa 227-0055 (JP); KATOU, Shintarou, Tokyo 136-8627 (JP); AKITOMI, Jou, Tokyo 136-8627 (JP); WAGA, Iwao, Tokyo 136-8627 (JP)
(74) Representative: Betten & Resch
(86) International application number: PCT/JP2011/058249
(87) International publication number: WO 2011/125852

(56) References cited:
- EP-A1- 1 394 250
- TSUJI S ET AL.: 'RNA aptamer binding to polyhistidine-tag.' BIOCHEMICAL AND BIOPHYSICAL RESEARCH COMMUNICATIONS vol. 386, 2009, pages 227 - 231, XP026467450
- WURSTER SE ET AL.: 'Characterization of anti-NF-KB RNA aptamer-binding specificity in vitro and in the yeast three-hybrid system.' NUCLEIC ACIDS RESEARCH vol. 37, no. 18, 2009, pages 6214 - 6224, XP055069955
- BUTTER F ET AL.: 'Unbiased RNA-protein interaction screen by quantitative proteomics.' PROCEEDINGS OF THE NATIONAL ACADEMY OF SCIENCES OF USA vol. 106, no. 26, 2009, pages 10626 - 10631, XP055069956
- MAIRAL T ET AL.: 'Aptamers: molecular tools for analytical applications.' ANALYTICAL AND BIOANALYTICAL CHEMISTRY vol. 390, 2008, pages 989 - 1007, XP019584708
- SRISAWAT C ET AL.: 'Streptavidin aptamers: affinity tags for the study of RNAs and ribonucleoproteins.' RNA vol. 7, 2001, pages 632 - 641, XP002278262
- GREEN LS ET AL.: 'Aptamers as reagents for high-throughput screening.' BIOTECHNIQUES vol. 30, no. 5, 2001, pages 1094, 1095, 1098, 1100, 1102, 1104, 1106 - 1108, 1110, XP001153135

## Description

### Technical Field

The present invention relates to a nucleic acid construct and a complex formation method and a screening method using the same.

### Background Art

Binding molecules that specifically recognize targets and bind thereto are used widely, for example, in medical fields such as examinations, diagnoses, and treatments and are very important tools in the analysis of a disease and a disease state. Among them, the monoclonal antibodies are researched most widely because they have good specificity and affinity to targets and are favorable in stability and in terms of cost. However, in immunization to ordinary animals, it is difficult to form antibodies for low molecular antigens or antigens that are stored across species at a high degree, and antibodies that are specific to targets are not always available. In fact, there is the problem that, in spite of the fact that effective markers related to diseases have been reported, the markers cannot be applied to diagnoses and treatments because there are no specific antibodies.

Hence, in these years, methods of artificially forming binding molecules in place of forming antibodies using animals have been reported. For example, with respect to peptidic binding molecules, a phage display method, a liposome method, an *in vitro* virus method (an mRNA display method) using a puromycin probe, a peptide array method, and the like can be employed (Non-Patent Documents 1, 2, and 3). In whichever method, with respect to targets whose antibodies cannot be obtained by immunization, binding molecules can be separated by artificial selection.

However, for example, these methods have problems such as use of special reagents and apparatus, efficiency, and cost. Among the aforementioned methods, for example, a phage display method is relatively implemented. However, since the phage display method still requires technical know-how, the result is affected by the experience and knowledge of the experimenter, and it is not easy for everyone to perform the phage display method.

### Related Art Document

### [Non-Patent Document]

[Non-Patent Document 1] Smith, G. P. et al., Science, Vol. 228: pp. 1315-1317, 1985
[Non-Patent Document 2] Mathcakis, L. C. et al., Proc. Natl. Acad. Sci. USA, Vol. 91: pp. 9022-9026, 1994
[Non-Patent Document 3] Keefe, A.D.et al., Nature, Vol.410: pp. 715-718, 2001

EP1394250 provides a genotype-phenotype linkage by interaction between an RNA-binding protein and its encoding transcript.

### Summary of Invention

### Problem to be Solved by the Invention

Hence, the present invention is intended to provide a new tool for simply screening a candidate molecule that is bindable to a target and a screening method for screening a candidate molecule using the tool.

### Means for Solving Problem

The nucleic acid construct of the present invention is a nucleic acid construct that includes:
a cloning region for being inserted with an encoding nucleic acid of arbitrary peptide;
an encoding nucleic acid of a peptide tag, wherein the peptide tag is a histidine tag; and
an encoding nucleic acid of an aptamer that is bindable to the peptide tag, wherein the nucleic acid construct is for forming a complex of a fusion transcript having a base sequence that includes the encoding nucleic acid of arbitrary peptide, the encoding nucleic acid of a peptide tag, and the encoding nucleic acid of an aptamer that is bindable to the peptide tag and a fusion translation that includes the arbitrary peptide and the peptide tag.

The formation method of the present invention is a complex formation method for forming a complex of a fusion transcript having a base sequence that includes an encoding nucleic acid of arbitrary peptide, an encoding nucleic acid of a peptide tag, and an encoding nucleic acid of an aptamer that is bindable to the peptide tag and a fusion translation that includes the arbitrary peptide and the peptide tag that includes the step of:
expressing the nucleic acid construct of the present invention, the nucleic acid construct including:
   a cloning region for being inserted with an encoding nucleic acid oi arbitrary peptide;
   an encoding nucleic acid of a peptide tag; and
   an encoding nucleic acid of an aptamer that is bindable to the peptide tag, the encoding nucleic acid of arbitrary peptide being inserted into the cloning region, wherein the peptide tag is a histidine tag.

The screening method of the present invention is a screening method for screening peptide that is bindable to a target or an encoding nucleic acid of the peptide that includes the steps of:
(A) forming a complex by the complex formation method of the present invention;
(B) bringing the complex into contact with the target; and
(C) recovering the complex that is bound to the target.

The complex formation kit of the present invention is a complex formation kit used for the complex formation method of the present invention that includes the nucleic acid construct of the present invention. Further, the screening kit of the present invention is a screening kit used for the screening method of the present invention that includes the nucleic acid construct of the present invention.

### Effects of the Invention

The nucleic acid construct of the present invention can form the complex of the fusion transcript and the fusion translation utilizing the binding between the transcribed aptamer and the translated peptide tag. In the complex, the fusion transcript includes the transcript of the encoding sequence of the arbitrary peptide and the fusion translation includes the arbitrary peptide. Therefore, in the case where the complex binds to a target, for example, the arbitrary peptide that is bindable to the target can be identified through the identification of the transcript in the complex. In this manner, according to the present invention, simply by inserting the encoding nucleic acid of arbitrary peptide into the nucleic acid construct of the present invention to form a complex and recovering the complex that is bound to the target, the peptide that is bindable to the target and its encoding nucleic acid can be identified without difficulty. Accordingly, for example, it can be said that the present invention provides very useful tools and methods for the screening of a binding molecule relative to a target in medical fields and the like.

### Brief Description of Drawings

[FIG. 1] FIG. 1 is a sensorgram of the aptamers in Examples of the present invention.
[FIG. 2] FIG. 2 is a sensorgram of the aptamer shot 47 in Examples of the present invention.
[FIG. 3] FIG. 3 is a schematic view showing the predicted secondary structures of the aptamers in Examples of the present invention.
[FIG. 4] FIG. 4 is a schematic view showing the secondary structure of the aptamer #47s in Examples of the present invention.
[FIG. 5] FIG. 5 is a sensorgram of the aptamers in Examples of the present invention.
[FIG. 6] FIG. 6 is a schematic view showing the structures of the fusion proteins in Examples of the present invention.
[FIG. 7] FIG. 7 is a graph showing the binding of the aptamer shot 47 to the fusion proteins in Examples of the present invention.
[FIG. 8] FIG. 8 is a graph showing the binding of the aptamer shot 47 to the fusion protein in Examples of the present invention.
[FIG. 9] FIG. 9 is a graph showing the binding of the aptamer shot 47sss to the fusion protein in Examples of the present invention.
[FIG. 10] FIG. 10 is a photograph of the result of the blotting showing the binding between the aptamer shot 47 and the fusion proteins in Examples of the present invention.
[FIG. 11] FIG. 11 is a photograph of the result of the blotting showing the binding between the aptamer shot 47 and the His-MIF in Examples of the present invention.
[FIG. 12] FIGs. 12A to 12I show the outline of the steps in an example of the screening method of the present invention.
[FIG. 13] FIG. 13 is an electrophoresis photograph of the amplification product of the RNA recovered by the screening in Examples of the present invention.
[FIG. 14] FIG. 14 is a graph showing the binding of the lysates of round 1 and round 2 to the targets in Examples of the present invention.
[FIG. 15] FIG. 15 is an electrophoresis photograph of the amplification product using the RNA that encodes the complex protein that is bound to the immobilized antibody as a template in Examples of the present invention.

### Description of Embodiments

In the present invention, hereinafter, the aptamer that is bindable to a peptide tag, which according to the present invention is a histidine tag, is also referred to as the "tag aptamer" or the "aptamer" and the encoding nucleic acid of the aptamer is also referred to as the "tag aptamer-encoding nucleic acid" or the "aptamer-encoding nucleic acid". The encoding nucleic acid of a peptide tag is also referred to as the "peptide tag-encoding nucleic acid" or the "tag-encoding nucleic acid". The encoding nucleic acid of arbitrary peptide to be inserted into the cloning region is also referred to as the "arbitrary encoding nucleic acid" or the "random encoding nucleic acid".

In the present invention, an antisense strand refers to one of the strands of a double stranded nucleic acid that can be a template of transcription and a sense strand refers to the other of the strands that is a complementary strand of the antisense strand and is not to be served as a template of transcription. Since the transcript is complementary to the antisense strand, it has the same sequence as the sense strand (T is replaced by U). The "fusion transcript" is, for example, RNA and specifically mRNA. The "fusion translation" is, for example, fusion peptide and includes the meaning of fusion protein. In the present invention, the "peptide" refers to the substance in which at least two amino acid residues are bound, for example. In the present invention, for example, the peptide includes the meaning of so-called polypeptide and the polypeptide includes the meaning of so-called oligopeptide. Generally, the oligopeptide is peptide having about at the most 10 amino acid residues, for example. In the present invention, there is no particular limitation on the number of the amino acid residues of the peptide. In the present invention, the 5' side is also referred to as the upstream side and the 3' side is also referred to as the downstream side.

### <Nucleic acid construct>

As described above, the nucleic acid construct of the present invention is a nucleic acid construct that includes a cloning region for being inserted with the arbitrary encoding nucleic acid; the peptide tag-encoding nucleic acid; and an encoding nucleic acid of an aptamer that is bindable to the peptide tag. The nucleic acid construct is for forming a complex of a fusion transcript having a base sequence that includes the arbitrary encoding nucleic acid, the peptide tag-encoding nucleic acid, and the aptamer-encoding nucleic acid and a fusion translation that includes the arbitrary peptide and the peptide tag.

The nucleic acid construct of the present invention can transcribe a fusion transcript having a base sequence that includes the arbitrary encoding nucleic acid to be inserted into the cloning region, the peptide tag-encoding nucleic acid, and the aptamer-encoding nucleic acid, and can translate a fusion translation that includes the arbitrary peptide and the peptide tag, for example.

The nucleic acid construct of the present invention may be a single stranded-nucleic acid or a double stranded-nucleic acid, for example, and the latter is preferable. Although the terms, the "sense strand" and the "antisense strand" are used in the description of the present invention, this does not limit the nucleic acid construct of the present invention to a double stranded-nucleic acid. These terms are used for making it clear whether a nucleic acid is described as an antisense strand that is to be used as a template of transcription or as its complementary strand when the sequences of the respective encoding nucleic acids, the positional relationship thereof, and the like are described, for example.

In the nucleic acid construct of the present invention, there is no particular limitation on the positional relationship between the cloning region, the peptide tag-encoding nucleic acid, and the aptamer-encoding nucleic acid. With respect to the positional relationship between the cloning region and the peptide tag-encoding nucleic acid, for example, in a sense strand, the peptide tag-encoding nucleic acid may be positioned at the 5' side of the cloning region or the peptide tag-encoding nucleic acid may be positioned at the 3' side of the cloning region, and the former is preferable. Further, there is no particular limitation on the positional relationship between the cloning region and the peptide tag-encoding nucleic acid and the aptamer-encoding nucleic acid. In a sense strand, for example, the aptamer-encoding nucleic acid may be positioned at the 5' side or at the 3' side of the cloning region and the peptide tag-encoding nucleic acid, and the latter is preferable.

As a specific example, with respect to the cloning region, the peptide tag-encoding nucleic acid, and the aptamer-encoding nucleic acid, for example, in a sense strand, the peptide tag-encoding nucleic acid, the cloning region, and the aptamer-encoding nucleic acid are preferably arranged from the 5' side or the 3' side in this order, and more preferably arranged from the 5' side in this order. That is, preferably, the nucleic acid construct of the present invention includes the peptide tag-encoding nucleic acid at the 5' side of the cloning region and includes the aptamer-encoding nucleic acid at the 3' side of the cloning region, for example. Further, preferably, the aptamer-encoding nucleic acid is contained in a stem-loop structure at a transcription termination site or in the vicinity of a transcription termination site, for example.

Preferably, in the nucleic acid construct of the present invention, the cloning region includes a recognition site of a restriction enzyme, for example. There is no limitation at all on the recognition site of the restriction enzyme, and can be designed based on the recognition sites of various restriction enzymes. With respect to the nucleic acid construct of the present invention, when the arbitrary encoding nucleic acid is inserted into the cloning region, for example, one section may be cleaved for being inserted with arbitrary encoding nucleic acid or plural sections may be cleaved for being inserted with the arbitrary encoding nucleic acids. In the latter case, by the cleavage of plural sections, a partial sequence or the entire sequence of the cloning region may be removed, for example. That is, when the arbitrary encoding nucleic acid is inserted into the cloning region, for example, the arbitrary encoding nucleic acid may be inserted by cleaving plural sections to remove a partial sequence or the entire sequence of the cloning region. In such a case, the cloning region preferably includes a stuffer sequence, for example. There is no particular limitation on the base sequence of the stuffer sequence.

In the nucleic acid construct of the present invention, there is no particular limitation on the arbitrary encoding nucleic acid to be inserted into the cloning region. The arbitrary encoding sequence may be a base sequence designed randomly or a base sequence that encodes a target amino acid sequence, for example. Specifically, examples thereof include nucleic acid libraries such as a cDNA library and a random DNA library that includes random base sequences. There is no particular limitation on the length of the arbitrary encoding nucleic acid, and the arbitrary encoding nucleic acid has, for example, a base length of multiples of 3. The lower limit of the length is, for example, 12 bases, preferably 21 bases, more preferably 42 bases, and still more preferably 60 bases; the upper limit of the length is, for example, 3000 bases, preferably 501 bases, more preferably 120 bases; and the range thereof is, for example, 21 to 3000 bases, preferably 42 to 501 bases, and more preferably 60 to 120 bases. In the case where the library of the arbitrary encoding nucleic acid is formed and this library is inserted into the nucleic acid construct, the arbitrary encoding nucleic acid preferably includes a consensus sequence common among the arbitrary encoding nucleic acids in the library and a random sequence different among the arbitrary encoding nucleic acids in the library, for example. In the case where the arbitrary encoding nucleic acid includes the random sequence, the random sequence has, for example, a length from 21 to 180 bases, preferably from 39 to 120 bases, and more preferably from 60 to 99 bases.

In the case where the nucleic acid library that includes a random base sequence is inserted as the arbitrary encoding nucleic acid, the random base sequence is preferably designed such that the expression probability of stop codon becomes low in the middle of the sequence, for example. Therefore, with respect to the random base sequence in a sense strand, for example, the 3^{rd} base of the codon is preferably a base other than A. Specifically, the sequence of the codon is preferably "NNK". Here, N is A, G, C, T, or U and K is G, T, or U. Further, for preventing the expression of the stop codon, with respect to the random base sequence in a sense strand, for example, the 1^{st} base of the codon can be a base other than T. Specifically, the sequence of the codon can be "VNK". Here, V is A, G, or C.

With respect to the nucleic acid construct of the present invention, for example, the arbitrary encoding nucleic acid may be inserted into the cloning region before use, or the arbitrary encoding nucleic acid may be inserted into the cloning region at the time of use. In the former case, the arbitrary encoding nucleic acid is arranged so as to be a reading frame according to the amino acid sequence of the arbitrary peptide, for example. Further, in the latter case, the arbitrary encoding nucleic acid is arranged so as to be a reading frame according to the amino acid sequence of the arbitrary peptide, for example.

Preferably, the arbitrary encoding nucleic acid includes a start codon, for example. Further, for example, the cloning region may include a start codon so that the start codon is arranged adjacent to the 5' side of the arbitrary encoding nucleic acid when the arbitrary encoding nucleic acid is inserted into the cloning region.

In the case where the nucleic acid construct of the present invention includes the cloning region at the 3' side of the peptide tag-encoding nucleic acid, the arbitrary encoding nucleic acid preferably includes a stop codon, for example. Specifically, for example, in the case where the peptide tag-encoding nucleic acid, the cloning region, and the aptamer-encoding nucleic acid are arranged from the 5' side in this order in a sense strand, the arbitrary encoding nucleic acid preferably includes a stop codon as described above because the translation of the aptamer-encoding nucleic acid can be prevented. The position of the stop codon in the arbitrary encoding nucleic acid is preferably adjacent to the 3' side of the codon relative to a C-terminal amino acid residue of the arbitrary peptide, for example. Thereby, for example, translation can be terminated at the stage where the arbitrary peptide has been translated. Further, for example, the cloning region may include a stop codon so that the stop codon is arranged adjacent to the 3' side of the arbitrary encoding nucleic acid when the arbitrary encoding nucleic acid is inserted into the cloning region.

On the other hand, in the case where the nucleic acid construct of the present invention includes the cloning region at the 5' side of the peptide tag-encoding nucleic acid, the arbitrary encoding nucleic acid preferably does not include a stop codon, for example. Specifically, for example, in the case where the cloning region, the peptide tag-encoding nucleic acid, and the aptamer-encoding nucleic acid are arranged from the 5' side in this order in a sense strand, the arbitrary encoding nucleic acid preferably does not include a stop codon for efficiently performing the translation of the peptide tag-encoding nucleic acid.

In the present invention, the peptide tag is a histidine tag and there is no particular limitation on the type of the aptamer that is bindable to a peptide tag as long as the peptide tag and the aptamer can be bound to each other. By selecting the peptide tag and the aptamer bindable thereto, when the fusion transcript and the fusion translation are formed, the complex can be formed by the binding between the peptide tag and the aptamer by the nucleic acid construct of the present invention. In the present invention, the "peptide tag" refers to peptide that is to be bound or added to a molecule as a marker, for example.

In the present invention, "bindable to a peptide tag" can also be described as "having a binding affinity to a peptide tag" or "having a binding activity to a peptide tag (peptide tag binding activity)", for example. The binding between the aptamer and the peptide tag can be determined, for example, by the surface plasmon resonance-molecular interaction analysis or the like. An apparatus such as Biacore X (product name, GE Healthcare UK Ltd.) can be used for the determination, for example. The binding activity of the aptamer to the peptide tag can be expressed, for example, by the dissociation constant between the aptamer and the peptide tag. In the present invention, there is no particular limitation on the dissociation constant of the aptamer.

For example, it is preferable that the aptamer is specifically bindable to the peptide tag and it is more preferable that the aptamer has a superior binding force to the peptide tag. The binding constant (K_{D}) of the aptamer to the peptide tag is preferably 1 × 10⁻⁹ mol/L or less, more preferably 5 × 10⁻¹⁰ mol/L, and still more preferably 1 × 10⁻¹⁰ mol/L, for example.

The peptide tag aptamer is bindable to a single peptide tag. In addition, the peptide tag aptamer is bindable to fusion peptide that contains the peptide tag via the peptide tag, for example. Examples of the fusion peptide include fusion peptide that contains the peptide tag at the N-terminal side and fusion peptide that contains the peptide tag at the C-terminal side. Further, the fusion peptide may contain other peptide.

According to the present invention, the peptide tag is a histidine tag. There is no particular limitation on the length of the peptide tag, and the length is preferably 6 to 330 or 6 to 30, more preferably 6 to 15, and still more preferably 8 to 15, for example.

In the nucleic acid construct of the present invention, the peptide tag-encoding nucleic acid is arranged so as to form a reading frame according to the amino acid sequence of the peptide tag, for example. Further, in the nucleic acid construct of the present invention, the peptide tag-encoding nucleic acid and the arbitrary encoding nucleic acid are arranged such that the peptide tag is added to the arbitrary peptide at the time of translation, for example. At the time of translation, for example, the peptide tag may be added directly to the arbitrary peptide or the peptide tag may be added indirectly to the arbitrary peptide via a linker such as peptide.

In the present invention, hereinafter, histidine is also referred to as "His", a histidine tag is also referred to as the "His tag", and an encoding nucleic acid of the His tag is also referred to as the "His tag-encoding nucleic acid". Further, an aptamer that is bindable to the His tag is also referred to as the "His tag aptamer" or the "aptamer", and an encoding nucleic acid of the His tag aptamer is referred to as the "His tag aptamer-encoding nucleic acid" or the "aptamer-encoding nucleic acid".

The His tag normally refers to plural continuous His, i.e., His peptide. In the present invention, for example, the His tag may be peptide consisting of His peptide of plural contiguous His. Also, the His tag may be peptide including the His peptide, i.e., peptide including an additional sequence at at least one of the N-terminal side and the C-terminal side of the His peptide. The additional sequence may be one amino acid residue or peptide consisting of at least two amino acid residues, for example. In the nucleic acid construct of the present invention, there is no particular limitation on the length of the His tag to be encoded with the His tag-encoding nucleic acid. The number of amino acid residues of the His tag is, for example, 6 to 30, preferably 6 to 15, and more preferably 8 to 15. The number of histidines of His peptide in the His tag is preferably 6 to 10 and more preferably 6 to 8, for example.

There is no particular limitation on the sequence of the His tag-encoding nucleic acid as long as the His tag-encoding nucleic acid includes a sequence that encodes His peptide (hereinafter, referred to as the "His peptide-encoding sequence"). Specifically, there is no particular limitation on the sequence as long as the His tag-encoding nucleic acid has codons of His continuously. Further, as described above, the His tag may further include the additional sequence besides His peptide. Therefore, for example, the His tag-encoding nucleic acid may include a sequence that encodes the additional sequence (hereinafter, referred to as the "additional encoding sequence") at least one of the 5' side and the 3' side of the His peptide-encoding sequence. There is no particular limitation on the additional encoding sequence.

A specific example of the additional encoding sequence at the 5' side of the His peptide-encoding sequence includes a sequence that includes a start codon. The sequence that includes a start codon may be a sequence that includes only the start codon or a sequence including the start codon and a sequence having a base length of multiples of 3. In the latter case, for example, the sequence having a base length of multiples of 3 is a sequence that encodes at least one amino acid residue and is adjacent to the 3' side of the start codon, for example.

Further, the additional encoding sequence at the 3' side of the His peptide-encoding sequence is preferably a sequence having a base length of multiples of 3, for example. Among them, in the case where the nucleic acid construct of the present invention includes the cloning region at the 5' side of a His tag-encoding nucleic acid, the additional encoding sequence at the 3' side preferably includes a stop codon, for example. Specifically, for example, in the case where the cloning region, the His tag-encoding nucleic acid, and the aptamer-encoding nucleic acid are arranged from the 5' side in this order in a sense strand, since the translation of the aptamer-encoding nucleic acid can be prevented, the additional encoding sequence preferably includes a stop codon as described above. On the other hand, in the case where the nucleic acid construct of the present invention includes the cloning region at the 3' side of the His tag-encoding nucleic acid, the additional encoding sequence at the 3' side preferably does not include a stop codon. Specifically, for example, in the case where the His tag-encoding nucleic acid, the cloning region, and the aptamer-encoding nucleic acid are arranged from the 5' side in this order in the sense strand, the additional encoding sequence at the 3' side preferably does not include a stop codon for efficiently performing the translation of the arbitrary encoding nucleic acid to be inserted into the cloning region.

In the nucleic acid construct of the present invention, there is no particular limitation on the aptamer-encoding nucleic acid as long as it is a nucleic acid that encodes the aptamer that is bindable to the peptide tag. Specific examples of the aptamer to be encoded with the aptamer-encoding nucleic acid will be described below.

In the nucleic acid construct of the present invention, as described above, the peptide tag-encoding nucleic acid and the arbitrary encoding nucleic acid to be inserted into the cloning region preferably each include a start codon. For example, only one of the encoding nucleic acids positioned at the 5' side may include a start codon.

The nucleic acid construct of the present invention may include at least two peptide tag-encoding nucleic acids, for example. In this case, one of the peptide tag-encoding nucleic acids is the aforementioned encoding nucleic acid of the peptide tag to which the aptamer can be bound. Hereinafter, this peptide tag-encoding nucleic acid is referred to as the "main peptide tag-encoding nucleic acid" and a peptide tag to be encoded with this encoding nucleic acid is referred to as the "main peptide tag". Examples of the main peptide tag include the aforementioned peptide tags and examples of the main peptide tag-encoding nucleic acid include the aforementioned peptide tag-encoding nucleic acids. Preferably, the main peptide tag is the His tag and the main peptide tag-encoding nucleic acid is the His tag-encoding nucleic acid. In the nucleic acid construct of the present invention, hereinafter, a peptide tag-encoding nucleic acid other than the main peptide tag-encoding nucleic acid is referred to as the "sub peptide tag-encoding nucleic acid" and a peptide tag to be encoded with this encoding nucleic acid is referred to as the "sub peptide tag". There are no particular limitations on the sub peptide tag and the sub peptide tag-encoding nucleic acid. An example of the sub peptide tag includes T7 gene 10 leader and an example of the sub peptide tag-encoding nucleic acid includes the encoding sequence of T7 gene 10 leader. In the case where the nucleic acid construct of the present invention includes the main peptide tag-encoding sequence and the sub peptide tag-encoding sequence, for example, a complex of a fusion transcript having a base sequence that includes the main peptide tag-encoding nucleic acid, the sub peptide tag-encoding nucleic acid, the arbitrary encoding nucleic acid, and the aptamer-encoding nucleic acid and a fusion translation that includes the main peptide tag, the sub peptide tag, and the arbitrary peptide is formed by the transcription and the translation of the nucleic acid construct of the present invention. There is no particular limitation on the position of the sub peptide tag-encoding nucleic acid. For example, the sub peptide tag-encoding nucleic acid is preferably adjacent to the main peptide tag-encoding nucleic acid in a sense strand. The sub peptide tag-encoding nucleic acid can be arranged either of the 5' side or the 3' side of the main peptide tag-encoding nucleic acid, and is more preferably arranged at the 3' side of the main peptide tag-encoding nucleic acid.

Specifically, for example, besides the His tag-encoding nucleic acid as the main peptide tag-encoding nucleic acid, the nucleic acid construct of the present invention may further include the sub peptide tag-encoding nucleic acid. In this case, a complex of a fusion transcript having a base sequence that includes the His tag-encoding nucleic acid, the sub peptide tag-encoding nucleic acid, the arbitrary encoding nucleic acid, and the aptamer-encoding nucleic acid and a fusion translation that includes the His tag, the sub peptide tag, and the arbitrary peptide is formed by the transcription and the translation of the nucleic acid construct of the present invention. The sub peptide tag is preferably T7 gene 10 leader and the nucleic acid construct of the present invention preferably includes the encoding sequence of T7 gene 10 leader as the sub peptide tag-encoding nucleic acid, for example. There is no particular limitation on the position of the sub peptide tag-encoding nucleic acid. For example, the sub peptide tag-encoding nucleic acid is preferably adjacent to the His tag-encoding nucleic acid in a sense strand. The sub peptide tag-encoding nucleic acid can be arranged either of the 5' side or the 3' side of the His tag-encoding nucleic acid, and is more preferably arranged at the 3' side of the His tag-encoding nucleic acid.

The nucleic acid construct of the present invention may further include a sequence that encodes a linker (hereinafter, referred to as the "linker-encoding sequence"), for example. The linker may be one amino acid residue or peptide consisting of at least two amino acid residues, for example. There is no particular limitation on the position of the linker-encoding sequence, and examples of the position include a site between the peptide tag-encoding nucleic acid and the cloning region, the inserted arbitrary encoding nucleic acid, or the aptamer-encoding nucleic acid and a site between the cloning region or the inserted arbitrary encoding nucleic acid and the aptamer-encoding nucleic acid.

When the transcription and the translation are performed using the nucleic acid construct of the present invention, as described above, the transcript (RNA aptamer) of the aptamer-encoding nucleic acid generated by the transcription is preferably not translated into peptide based on its sequence information. Hence, preferably, the nucleic acid construct of the present invention further includes a sequence for preventing the translation of the aptamer, for example. An example of the sequence for preventing the translation of the aptamer includes the aforementioned stop codon. In the case where the aptamer-encoding nucleic acid is arranged at the 3' side of the peptide tag-encoding nucleic acid and the arbitrary encoding nucleic acid in a sense strand, preferably, the sequence for preventing the translation of the aptamer is arranged between the aforementioned encoding nucleic acids (the peptide tag-encoding nucleic acid and the arbitrary encoding nucleic acid) and the aptamer-encoding nucleic acid, for example.

The nucleic acid construct of the present invention is, for example, composed of nucleosides. Specifically, the nucleic acid construct of the present invention is preferably composed of nucleotide residues that contain nucleosides. Examples of the nucleoside include ribonucleoside and deoxyribonucleoside. For example, the nucleic acid construct of the present invention may be composed of one of ribonucleosides and deoxynucleosides or may include both of them. Preferably, the nucleic acid construct of the present invention is DNA composed of deoxyribonucleosides, for example.

The nucleic acid construct of the present invention may include natural bases (non-artificial bases) and unnatural bases (artificial bases) as bases, for example. Examples of the natural base include A, C, G, T, U, and modified bases thereof. Examples of the modification include methylation, fluoration, amination, and thiation. Examples of the unnatural base include 2'-fluoropyrimidine and 2'-O-methylpyrimidine, and specific examples thereof include 2'-fluorouracil, 2'-aminouracil, 2'-O-methyl uracil, and 2-thiouracil. The nucleic acid construct of the present invention may include a natural nucleic acid and an unnatural nucleic acid as a nucleic acid. Examples of the natural nucleic acid include nucleotide residues including A, C, G, T, U, and modified bases thereof. Examples of the modification are the same as those described above. Examples of the unnatural nucleic acid include 2'-methylated-uracil nucleotide residue, 2'-methylated-cytosine nucleotide residue, 2'-fluorated-uracil nucleotide residue, 2'-fluorated-cytosine nucleotide residue, 2'-aminated-uracil nucleotide residue, 2'-aminated-cytosine nucleotide residue, 2'-thioated-uracil nucleotide residue, and 2'-thioated-cytosine nucleotide residue. The nucleic acid construct of the present invention may include a peptide nucleic acid (PNA), a locked nucleic acid (LNA), or the like, for example.

The nucleic acid construct of the present invention is preferably a vector, for example. Hereinafter, the vector is also referred to as the "expression vector". The expression vector can be constructed, for example, by inserting the cloning region, the peptide tag-encoding nucleic acid, and the aptamer-encoding nucleic acid into the basic skeleton of a vector. There is no particular limitation on the basic skeleton of a vector, and conventional vectors can be used, for example. Hereinafter, the basic skeleton of a vector is referred to as the "basic vector". In the case where the basic vector includes the cloning region and the peptide tag-encoding nucleic acid, for example, the expression vector can be constructed by inserting the aptamer-encoding nucleic acid into a desired site. Examples of the basic vector serving as the basic skeleton include a plasmid vector and a virus vector. Examples of the plasmid vector include *Escherichia coli* derived plasmid vectors such as pCold series (registered trademark, TAKARA BIO INC.), pET series (Merck, Invitrogen Corporation, etc.), pRSET series (Invitrogen Corporation), pBAD series (Invitrogen Corporation), pcDNA series (Invitrogen Corporation), pEF series (Invitrogen Corporation), pBR322, pBR325, pUC118, and pUC119; *Bacillus subtilis* derived plasmid vectors such as pUB110 and pTP5; and yeast derived plasmid vectors such as YEp13, YEp24, and YCp50. Further, examples of the virus vector include λ phage vectors such as Charon4A, Charon21A, EMBL3, EMBL4, λgt10, λgt11, and λZAP; filamentous phage vectors such as M13KE and pCANTAB5E; T7 phage vectors such as T7Select series; animal DNA virus vectors or RNA virus vectors such as retrovirus, vaccinia virus, and adenovirus; an insect virus vector such as baculovirus; and plant virus vectors. Among these basic vectors, for example, pCold, which is a cold shock expression vector, is preferable. Since such vectors can prevent the insolubilization of peptide expressed in a living cell such as *Escherichia coli* and can promote the solubilization thereof, the expressed peptide can be recovered without difficulty.

The nucleic acid construct of the present invention preferably includes a promoter such as a T7 promoter, a cold shock expression promoter (cspA promoter), a trp promoter, a lac promoter, a PL promoter, or a tac promoter so that the fusion translation can be expressed efficiently, for example. Besides this, for example, the nucleic acid construct of the present invention may include a terminator; a cis element such as an enhancer; a polyadenylation signal; a sequence of origin of replication (ori); a selection marker; a ribosome binding sequence such as an SD sequence or a KOZAK sequence; or a suppressor sequence. Examples of the selection marker include a dihydrofolate reductase gene, an ampicillin-resistant gene, and a neomycin-resistant gene.

In the nucleic acid construct of the present invention, as described above, the sequence of the aptamer-encoding nucleic acid is satisfied as long as it is a nucleic acid that encodes the aptamer that is bindable to the peptide tag.

There is no particular limitation on the sequence of the His tag aptamer as long as it is bindable to the His tag. There is no particular limitation on the dissociation constant of the His tag aptamer, and the dissociation constant is, for example, 1 × 10⁻⁹ mol/L or less. Since the dissociation constant (K_{d}) of an antibody to a His tag generally exceeds 1 × 10⁻⁹ mol/L, the His tag aptamer has a better binding affinity than the antibody. The dissociation constant of the His tag aptamer is preferably 5 × 10⁻¹⁰ mol/L or less and more preferably 1 × 10⁻¹⁰ mol/L or less.

The His tag aptamer is bindable to a single His tag. In addition, the His tag aptamer is bindable to fusion peptide that contains the His tag via the His tag, for example. Examples of the fusion peptide include fusion peptide that contains the His tag at the N-terminal side and fusion peptide that contains the His tag at the C-terminal side. Further, the fusion peptide may contain other peptide fragment.

Specific examples of the His tag aptamer will be described below. In the present invention, the aptamer is not limited to these examples. The His tag aptamer described below is, for example, an aptamer having a dissociation constant of 1 × 10⁻⁹ mol/L or less and has a better binding affinity to the His tag than an ordinary antibody. The sequences described below are the sequences of the His tag aptamer. For example, the sequence of the encoding nucleic acid of the His tag aptamer is complementary or homologous to the sequences of the His tag aptamer below and is a sequence in which U is replaced with T.

The His tag aptamer is any of the following nucleic acids (a), (b), (c), and (d), for example.
(a) a nucleic acid that includes the base sequence represented by SEQ ID NO: 17 GGUNₙAYUₘGGH (SEQ ID NO: 17)
   Here, N represents A, G, C, U, or T, n of Nₙ represents the number of N, which is an integer of 1 to 3, Y represents U, T, or C, m of Uₘ represents the number of U, which is an integer of 1 to 3, and H represents U, T, C, or A.
(b) a nucleic acid that includes a base sequence obtained by substitution, deletion, addition, or insertion of one or more bases in the base sequence shown in (a) and is bindable to the His peptide
(c) a nucleic acid that includes the base sequence represented by SEQ ID NO: 18 GGCGCCUUCGUGGAAUGUC (SEQ ID NO: 18)
(d) a nucleic acid that includes a base sequence obtained by substitution, deletion, addition, or insertion of one or more bases in the base sequence shown in (c) and is bindable to the His peptide

The His tag aptamer of the nucleic acid (a) can be any aptamer as long as it is a nucleic acid that includes the base sequence represented by SEQ ID NO: 17. Hereinafter, the base sequence represented by SEQ ID NO: 17 may also be referred to as the "binding motif sequence". In the binding motif sequence represented by SEQ ID NO: 17, N represents A, G, C, U, or T, in which A, G, C, or U is preferable, n of Nₙ represents the number of N, which is an integer of 1 to 3, Y represents U, T, or C, in which U or C is preferable, m of Uₘ represents the number ofU, which is an integer of 1 to 3, and H represents U, T, C, or A, in which U, C, or A is preferable. The binding motif sequence is a consensus sequence that can be seen in the base sequences represented by SEQ ID NOs: 1 to 16 that will be described later. In the binding motif sequence, there is no particular limitation on the number (n) of N in Nₙ. For example, the number of N may be 1 (N), 2 (NN), or 3 (NNN), and each N may be the same base or different base. In the binding motif sequence, there is no particular limitation on the number (m) of U in Uₘ. For example, the number of U may be 1 (U), 2 (UU), or 3 (UUU).

Examples of the His tag aptamer of the nucleic acid (a) include the following nucleic acids (a1) to (a4).

An example of the His tag aptamer of the nucleic acid (a) includes the following nucleic acid (a1),
(a1) a nucleic acid that includes a base sequence represented by any of SEQ ID NOs: 89 to 104.

The base sequences represented by these SEQ ID NOs each include the binding motif sequence represented by SEQ ID NO: 17. The aptamer of the nucleic acid (a1) may be a nucleic acid consisting of a base sequence represented by any of these SEQ ID NOs or a nucleic acid that includes a base sequence represented by any of these SEQ ID NOs, for example. The following Table 1 shows the base sequences represented by SEQ ID NOs: 89 to 104. In Table 1, the underlined parts each correspond to the binding motif sequence represented by SEQ ID NO: 17. Hereinafter, each aptamer in Table 1 may be indicated by the name described at the left side of each sequence (hereinafter, the same applies).

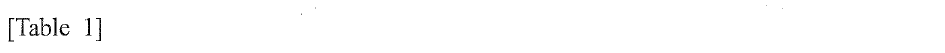

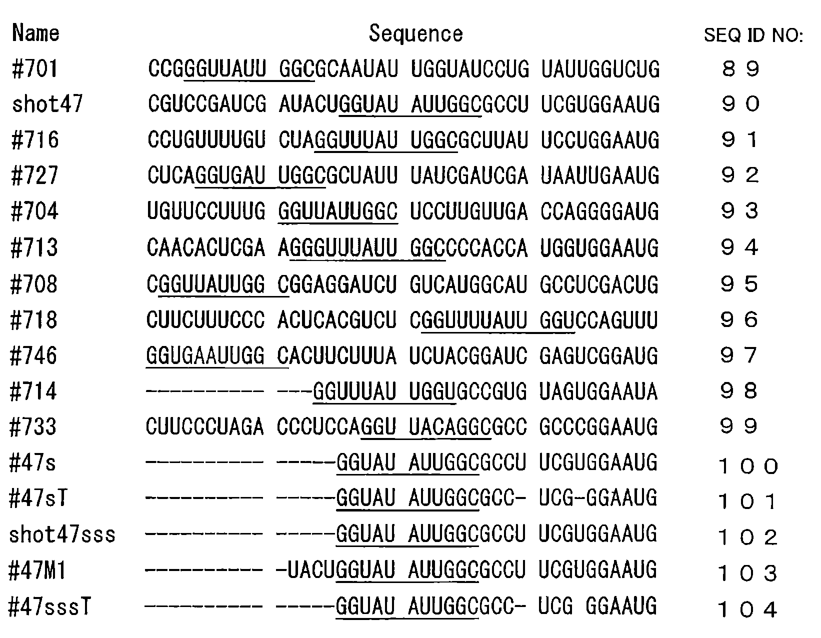

A specific example of the His tag aptamer of the nucleic acid (a1) includes the following nucleic acid (a1-1).

### (a1-1) a nucleic acid that includes a base sequence represented by any of SEQ ID NOs: 1 to 16

The base sequences represented by SEQ ID NOs: 1 to 16 respectively include the base sequences represented by SEQ ID NOs: 89 to 104. The aptamer of the nucleic acid (a1-1) may be a nucleic acid consisting of a base sequence represented by any of SEQ ID NOs: 1 to 16 or a nucleic acid that includes a base sequence represented by any of SEQ ID NOs: 1 to 16, for example. The following Table 2 shows the base sequences represented by SEQ ID NOs: 1 to 16. In Table 2, the underlined parts each correspond to the binding motif sequence represented by SEQ ID NO: 17. Hereinafter, in the present invention, each aptamer in Table 2 may be indicated by the name described at the left side of each sequence (hereinafter, the same applies).

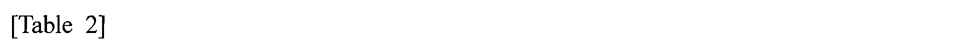

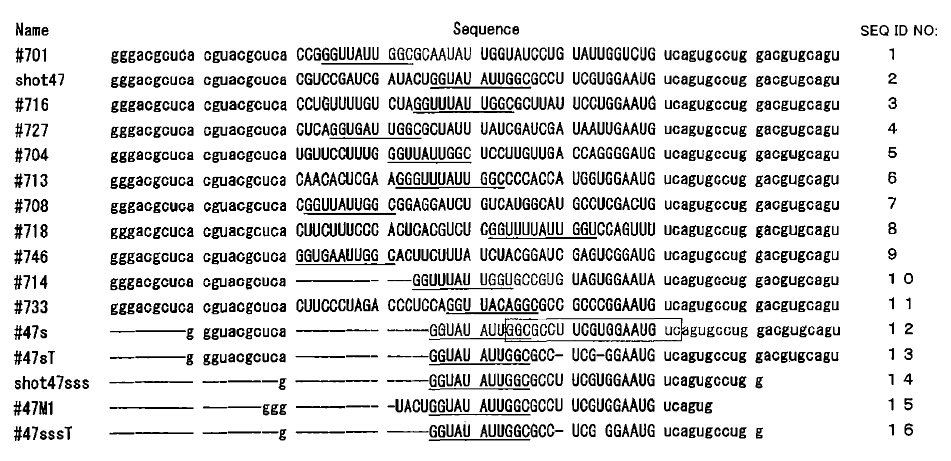

In addition, a specific example of the His tag aptamer of the nucleic acid (a) includes the following nucleic acid (a2).

### (a2) a nucleic acid that includes a base sequence represented by any of SEQ ID NOs: 105 to 114, 116 to 124, and 127 to 146

The base sequences represented by these SEQ ID NOs each include the binding motif sequence represented by SEQ ID NO: 17. The aptamer of the nucleic acid (a2) may be a nucleic acid consisting of a base sequence represented by any of these SEQ ID NOs or a nucleic acid that includes a base sequence represented by any of these SEQ ID NOs, for example. The following Tables 3 and 4 show the base sequences represented by SEQ ID NOs: 105 to 114, 116 to 124, and 127 to 146. In Tables 3 and 4, the underlined parts each correspond to the binding motif sequence represented by SEQ ID NO: 17. Hereinafter, in the present invention, each aptamer in Tables 3 and 4 may be indicated by the name described at the left side of each sequence (hereinafter, the same applies).

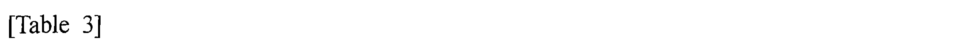

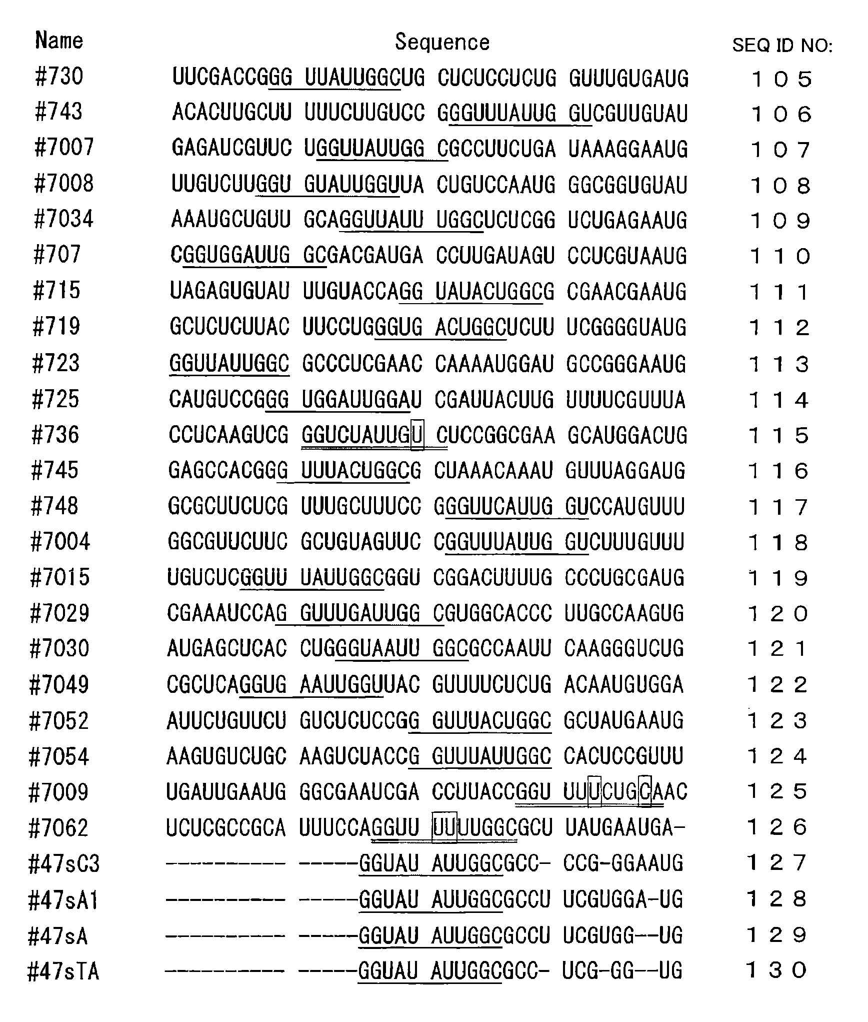

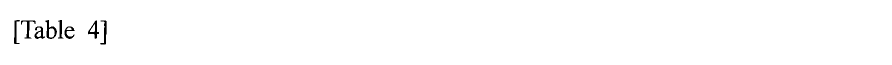

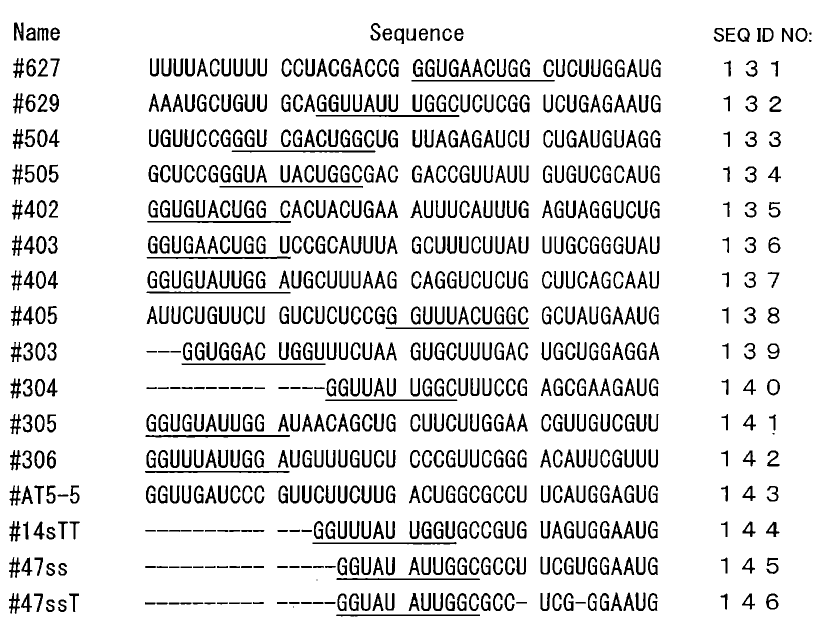

A specific example of the His tag aptamer of the nucleic acid (a2) includes the following nucleic acid (a2-1).

### (a2-1) a nucleic acid that includes a base sequence represented by any of SEQ ID NOs: 26 to 35, 37 to 45, 65 to 68, 19 to 25, and 48 to 56

The base sequences represented by SEQ ID NOs: 26 to 35, 37 to 45, 65 to 68, 19 to 25, and 48 to 56 respectively include the base sequences represented by SEQ ID NOs: 105 to 114, 116 to 124, and 127 to 146. The aptamer of the nucleic acid (a2-1) may be a nucleic acid consisting of a base sequence represented by any of these SEQ ID NOs or a nucleic acid that includes a base sequence represented by any of these SEQ ID NOs, for example. The following Tables 5 and 6 show the base sequences represented by SEQ ID NOs: 26 to 35, 37 to 45, 65 to 68, 19 to 25, and 48 to 56. In Tables 5 and 6, the underlined parts each correspond to the binding motif sequence represented by SEQ ID NO: 17. Hereinafter, in the present invention, each aptamer in Tables 5 and 6 may be indicated by the name described at the left side of each sequence (hereinafter, the same applies).

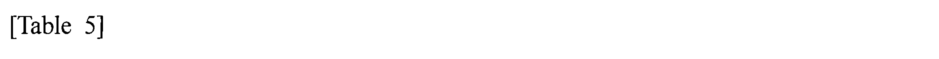

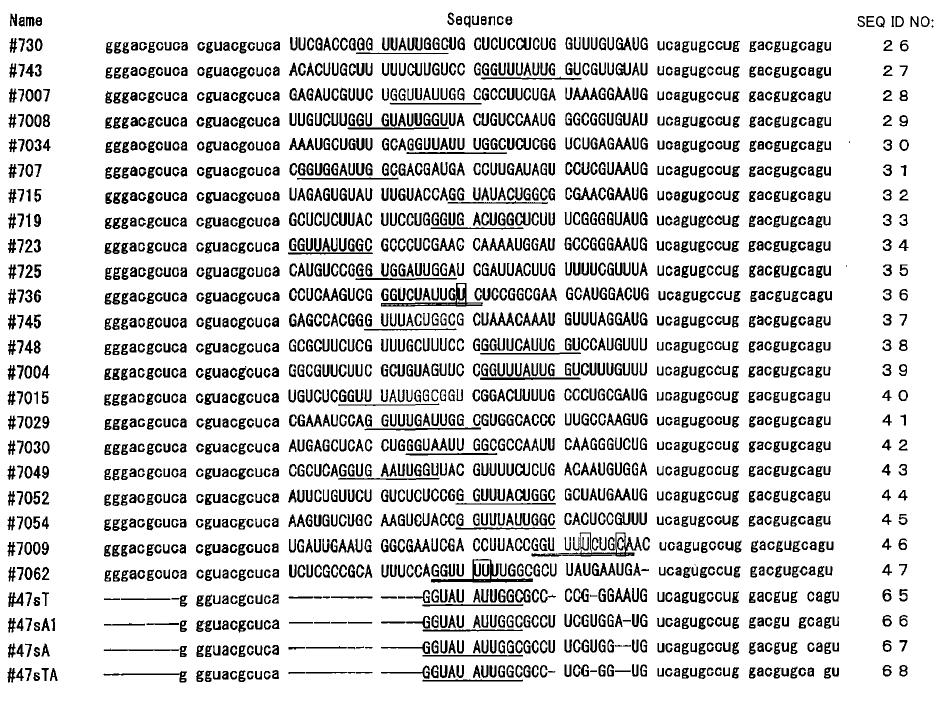

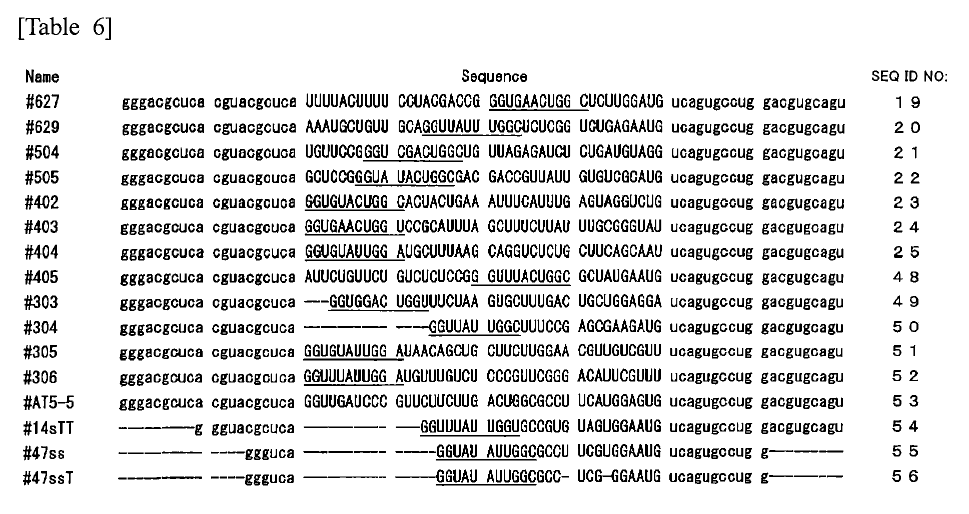

In addition, a specific example of the His tag aptamer of the nucleic acid (a) includes the following nucleic acid (a3).

### (a3) a nucleic acid that includes the base sequence represented by SEQ ID NO: 147 GGUNₙAYUₘGGHGCCUUCGUGGAAUGUC (SEQ ID NO: 147)

In the base sequence represented by SEQ ID NO: 147, "GGUNₙAYUₘGGH" corresponds to the binding motif sequence represented by SEQ ID NO: 17, which is as described above. Further, in the base sequence represented by SEQ ID NO: 147, "GGHGCCUUCGUGGAAUGUC" corresponds to the base sequence represented by SEQ ID NO: 18 that will be described below (here, H is C), which is as will be described below. The base sequence represented by SEQ ID NO: 18 is, for example, the base sequence of a region that forms a stem-loop structure in an aptamer, and is hereinafter also referred to as the "stem-loop motif sequence". In the base sequence represented by SEQ ID NO: 147, 3 bases at the 3' end of the binding motif sequence overlap with 3 bases at the 5' end of the stem-loop motif sequence.

An example of the base sequence represented by SEQ ID NO: 147 includes the base sequence represented by SEQ ID NO: 148.
GGUAUAUUGGCGCCUUCGUGGAAUGUC (SEQ ID NO: 148)

A specific example of the His tag aptamer of the nucleic acid (a3) includes the following nucleic acid (a3-1).

### (a3-1) a nucleic acid that includes a base sequence represented by any of SEQ ID NOs: 2, 12, 14, 15, and 55

The base sequences represented by SEQ ID NOs: 2, 12, 14, 15, and 55 each include the base sequence represented by SEQ ID NO: 147, specifically the base sequence represented by SEQ ID NO: 148. The aptamer of the nucleic acid (a3-1) may be a nucleic acid consisting of a base sequence represented by any of these SEQ ID NOs or a nucleic acid that includes a base sequence represented by any of these SEQ ID NOs, for example. The following Table 7 shows the base sequences represented by SEQ ID NOs: 2, 12, 14, 15, and 55. In Table 7, the underlined parts each correspond to the base sequence represented by SEQ ID NO: 17, and the regions enclosed in the boxes each correspond to the base sequence represented by SEQ ID NO: 18.

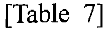

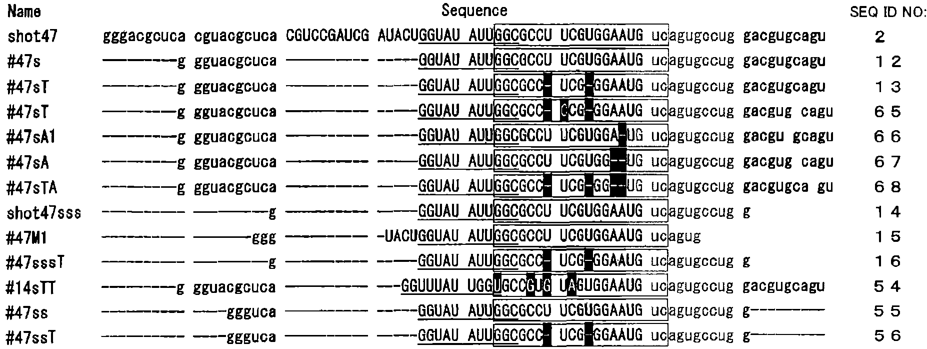

The His tag aptamer of the nucleic acid (b) is, as described above, a nucleic acid that includes a base sequence obtained by substitution, deletion, addition, or insertion of one or more bases in the base sequence shown in (a) and is bindable to the His peptide. There is no particular limitation on "one or more", and the number of bases is, for example, 1 to 5, preferably 1 to 4, more preferably 1 to 3, still more preferably 1 or 2, and particularly preferably 1 in the base sequence represented by SEQ ID NO: 17. Further, the aptamer of the nucleic acid (b) may be, for example, a nucleic acid that includes a base sequence obtained by substitution, deletion, addition, or insertion of one or more bases in a base sequence represented by any of the SEQ ID NOs mentioned for the aptamer of the nucleic acid (a) and is bindable to the His peptide. In this case, there is no particular limitation on "one or more", and the number of bases is, for example, 1 to 10, preferably 1 to 5, more preferably 1 to 4, still more preferably 1 to 3, particularly preferably 1 or 2, and most preferably 1 in each of the aforementioned base sequences. Further, the aptamer of the nucleic acid (b) may be, for example, a nucleic acid that includes a base sequence obtained by substitution, deletion, addition, or insertion of one or more bases in the full-length base sequence of the aptamer of the nucleic acid (a) and is bindable to the His peptide. In this case, there is no particular limitation on "one or more", and the number of bases is, for example, 1 to 10, preferably 1 to 5, more preferably 1 to 4, still more preferably 1 to 3, particularly preferably 1 or 2, and most preferably 1 in the full-length sequence.

There is no particular limitation on the base used for the substitution, addition, or insertion, and examples of the base include A, C, G, U, and T. Besides them, for example, a modified base, an artificial base, and the like can be employed. Examples of the modified base include 2'-fluoropyrimidine and 2'-O-methylpyrimidine. Further, for the substitution, addition, or insertion of a base, for example, a nucleoside or a nucleotide may be used, or a deoxynucleoside or a deoxynucleotide may be used. Besides them, for example, a peptide nucleic acid (PNA), a locked nucleic acid (LNA), and the like may be used.

Examples of the His tag aptamer of the nucleic acid (b) include nucleic acids that include the base sequences shown in Tables 3 and 5. Specific examples thereof include a nucleic acid that includes the base sequence represented by SEQ ID NO: 115 (#736) or SEQ ID NO: 36 (#736) and a nucleic acid consisting of the base sequence represented by SEQ ID NO: 115 (#736) or SEQ ID NO: 36 (#736). The base sequence represented by SEQ ID NO: 36 includes the base sequence represented by SEQ ID NO: 115. Further, examples of the His tag aptamer of the nucleic acid (b) include a nucleic acid that includes the base sequences represented by SEQ ID NO: 125 (#7009) and SEQ ID NO: 46 (#7009) or a nucleic acid consisting of the base sequences represented by SEQ ID NO: 125 (#7009) and SEQ ID NO: 46 (#7009). The base sequence represented by SEQ ID NO: 46 includes the base sequence represented by SEQ ID NO: 125. In Tables 3 and 5, the double underlined parts of these base sequences each correspond to the binding motif sequence represented by SEQ ID NO: 17 and the bases enclosed in the boxes are substituted bases each having a base sequence that is different from the base sequence represented by SEQ ID NO: 17. Further, examples of the His tag aptamer of the nucleic acid (b) include a nucleic acid that includes the base sequences represented by SEQ ID NO: 126 (#7062) and SEQ ID NO: 47 (#7062) or a nucleic acid consisting of the base sequences represented by SEQ ID NO: 126 (#7062) and SEQ ID NO: 47 (#7062). In Tables 3 and 5, the double underlined parts of these base sequences each correspond to the binding motif sequence represented by SEQ ID NO: 17 and one of the bases (UU) enclosed in the box is a substituted base that is different from A of the binding motif sequence represented by SEQ ID NO: 17. Moreover, examples of the His tag aptamer of the nucleic acid (b) include a nucleic acid consisting of the base sequences represented by SEQ ID NO: 143 (#AT5-5) and SEQ ID NO: 53 (#AT5-5) or a nucleic acid that includes the base sequences represented by SEQ ID NO: 143 (#AT5-5) and SEQ ID NO: 53 (#AT5-5).

The His tag aptamer may be, for example, the following nucleic acid (e) or (f).
(e) a nucleic acid that includes a base sequence having at least 60% homology in the base sequence shown in (a) and is bindable to the His peptide
(f) a nucleic acid that includes a base sequence that hybridizes to the base sequence shown in (a) under stringent conditions or the complementary base sequence thereof and is bindable to the His peptide

In the nucleic acid (e), the homology (hereinafter, also referred to as the "identity") is, for example, at least 70%, preferably at least 80%, more preferably at least 90%, still more preferably at least 95%, and particularly preferably at least 99%. The identity can be calculated using BLAST or the like under a default condition, for example. The aptamer of the nucleic acid (e) may be, for example, a nucleic acid that includes a base sequence having identity in the base sequence represented by SEQ ID NO: 17 in the aptamer of the nucleic acid (a) and is bindable to the His peptide. The aptamer of the nucleic acid (e) may be, for example, a nucleic acid that includes a base sequence having identity in any of the base sequences represented by the respective SEQ ID NOs mentioned for the aptamer of the nucleic acid (a) and is bindable to the His peptide. Further, the aptamer of the nucleic acid (e) may be, for example, a nucleic acid that includes a base sequence having identity in the full-length base sequence in the aptamer of the nucleic acid (a) and is bindable to the His peptide.

In the nucleic acid (f), "hybridizes under stringent conditions" refer, for example, to experimental conditions of hybridization well known to those skilled in the technical field. Specifically, "stringent conditions" refer to conditions in which identification can be performed by hybridizing at 60 to 68°C in the presence of 0.7 to 1 mol/L NaCl and then washing at 65 to 68°C using 0.1 to 2 times as much SSC solution, for example. 1 × SSC is consisting of 150 mmol/L NaCl and 15 mmol/L sodium citrate. The aptamer of the nucleic acid (f) may be, for example, a nucleic acid that includes a base sequence that hybridizes to the base sequence represented by SEQ ID NO: 17 in the aptamer of the nucleic acid (a) under stringent conditions or the complementary base sequence thereof and is bindable to the His peptide. The aptamer of the nucleic acid (f) may be, for example, a nucleic acid that includes a base sequence that hybridizes to any of the base sequences represented by the respective SEQ ID NOs mentioned for the aptamer of the nucleic acid (a) under stringent conditions or the complementary base sequence thereof and is bindable to the His peptide. Further, the aptamer of the nucleic acid (f) may be, for example, a nucleic acid that includes a base sequence that hybridizes to the full-length base sequence in the aptamer of the nucleic acid (a) under stringent conditions or the complementary base sequence thereof and is bindable to the His peptide.

Further, the His tag aptamer may be, for example, a nucleic acid that includes a partial sequence of any of the base sequences represented by the respective SEQ ID NOs mentioned for the aptamer of the nucleic acid (a) and is bindable to the His peptide. The partial sequence is, for example, a sequence consisting of contiguous plural bases, preferably a sequence consisting of contiguous 5 to 40 bases, more preferably a sequence consisting of contiguous 8 to 30 bases, and particularly preferably a sequence consisting of contiguous 10 to 12 bases.

The aptamer of the nucleic acid (c) is, as described above, a nucleic acid that includes the base sequence represented by SEQ ID NO: 18. The base sequence represented by SEQ ID NO: 18 is, as described above, for example, the base sequence of the region at which a stem-loop structure is formed in the aptamer.
GGCGCCUUCGUGGAAUGUC (SEQ ID NO: 18)

Example of the His tag aptamer of the nucleic acid (c) include nucleic acids that include the base sequences represented by SEQ ID NOs: 2, 12, 14, 15, and 55. The aptamer of the nucleic acid (c) may be a nucleic acid consisting of a base sequence represented by any of these SEQ ID NOs or a nucleic acid that includes a base sequence represented by any of these SEQ ID NOs, for example. These base sequences are shown in Table 7.

The His tag aptamer of the nucleic acid (d) is, as described above, a nucleic acid that includes a base sequence obtained by substitution, deletion, addition, or insertion of one or more bases in the base sequence shown in (c) and is bindable to the His peptide. There is no particular limitation on "one or more", and the number of bases is, for example, 1 to 5, preferably 1 to 4, more preferably 1 to 3, still more preferably 1 or 2, and particularly preferably 1 in the base sequence represented by SEQ ID NO: 18. Further, the aptamer of the nucleic acid (d) may be, for example, a nucleic acid that includes a base sequence obtained by substitution, deletion, addition, or insertion of one or more bases in any of the base sequences represented by SEQ ID NOs: 2, 12, 14, 15, and 55 and is bindable to the His peptide. In this case, there is no particular limitation on "one or more", and the number of bases is, for example, 1 to 10, preferably 1 to 5, more preferably 1 to 4, still more preferably 1 to 3, particularly preferably 1 or 2, and most preferably 1 in the aforementioned base sequences. Further, the aptamer of the nucleic acid (d) may be a nucleic acid that includes a base sequence obtained by substitution, deletion, addition, or insertion of one or more bases in the full-length base sequence of the aptamer of the nucleic acid (c) and is bindable to the His peptide. In this case, there is no particular limitation on "one or more", and the number of bases is, for example, 1 to 10, preferably 1 to 5, more preferably 1 to 4, still more preferably 1 to 3, particularly preferably 1 or 2, and most preferably 1 in the full-length sequence. Preferably, the aptamer of the nucleic acid (d) has a stem-loop structure that is virtually the same as the stem-loop structure formed by the base sequence represented by SEQ ID NO: 18, for example.

There is no particular limitation on the base used for the substitution, addition, or insertion, and examples of the base include A, C, G, U, and T. Besides them, for example, a modified base, an artificial base, and the like can be employed. Examples of the modified base include 2'-fluoropyrimidine and 2'-O-methylpyrimidine. Further, for the substitution, addition, or insertion of a base, for example, a nucleoside or a nucleotide may be used, or a deoxynucleoside or a deoxynucleotide may be used. Besides them, for example, a peptide nucleic acid (PNA), a locked nucleic acid (LNA), and the like may be used.

Example of the His tag aptamer of the nucleic acid (d) include nucleic acids consisting of the base sequences represented by SEQ ID NOs: 13, 65 to 68, 16, 54, and 56 or nucleic acids that include these base sequences, for example. These base sequences are shown in Table 7.

In the base sequences represented by SEQ ID NOs: 13, 65 to 68, 16, 54, and 56 shown in Table 7, the bases enclosed in the boxes are the same sites as the stem-loop motif sequence represented by SEQ ID NO: 18 and the bases in white letters enclosed in the black boxes are the sites deleted or substituted from the stem-loop motif sequence. In Table 7, the deleted sites are each indicated by "-". With respect to the aptamer of the nucleic acid (d), U at 7^{th} base and 11^{th} base and A at 15^{th} base are preferably maintained in the stem-loop motif sequence represented by SEQ ID NO: 18, for example.

The His tag aptamer may be, for example, the following nucleic acid (g) or (h).
(g) a nucleic acid that includes a base sequence having at least 60% identity in the base sequence shown in (c) and is bindable to the His peptide
(h) a nucleic acid that includes a base sequence that hybridizes to the base sequence shown in (c) under stringent conditions or the complementary base sequence thereof and is bindable to the His peptide

In the nucleic acid (g), the identity is, for example, at least 70%, preferably at least 80%, more preferably at least 90%, and still more preferably at least 95%, and particularly preferably at least 99%. The aptamer of the nucleic acid (g) may be, for example, a nucleic acid that includes a base sequence having identity in any of the base sequences represented by SEQ ID NOs: 2, 12, 14, 15, and 55 in the aptamer of the nucleic acid (c) and is bindable to the His peptide. The aptamer of the nucleic acid (g) may be, for example, a nucleic acid that includes a base sequence having identity in any of the base sequences represented by the respective SEQ ID NOs mentioned for the aptamer of the nucleic acid (c) and is bindable to the His peptide. Further, the aptamer of the nucleic acid (g) may be, for example, a nucleic acid that includes a base sequence having identity in the full-length base sequence in the aptamer of the nucleic acid (c) and is bindable to the His peptide. Preferably, the aptamer of the nucleic acid (g) has a stem-loop structure that is virtually the same as the stem-loop structure formed by the base sequence represented by SEQ ID NO: 18, for example.

In the nucleic acid (h), "hybridizes under stringent conditions" are, for example, as has been described above. The aptamer of the nucleic acid (h) may be, for example, a nucleic acid that includes a base sequence that hybridizes to any of the base sequences represented by SEQ ID NOs: 2, 12, 14, 15, and 55 in the aptamer of the nucleic acid (c) under stringent conditions or the complementary base sequence thereof and is bindable to the His peptide. The aptamer of the nucleic acid (h) may be, for example, a nucleic acid that includes a base sequence that hybridizes to any of the base sequences represented by the respective SEQ ID NOs mentioned for the aptamer of the nucleic acid (c) under stringent conditions or the complementary base sequence thereof and is bindable to the His peptide. Further, the aptamer of the nucleic acid (h) may be, for example, a nucleic acid that includes a base sequence that hybridizes to the full-length base sequence of the aptamer of the nucleic acid (c) under stringent conditions or the complementary base sequence thereof and is bindable to the His peptide. Preferably, the aptamer of the nucleic acid (h) has a stem-loop structure that is virtually the same as the stem-loop structure formed by the base sequence represented by SEQ ID NO: 18, for example.

Further, the His tag aptamer may be, for example, a nucleic acid that includes a partial sequence of any of the respective sequences mentioned for the aptamer of the nucleic acid (c) and is bindable to the His peptide. The partial sequence is, for example, a sequence consisting of contiguous plural bases, preferably a sequence consisting of contiguous 5 to 40 bases, more preferably a sequence consisting of contiguous 8 to 30 bases, and particularly preferably a sequence consisting of contiguous 10 to 12 bases.

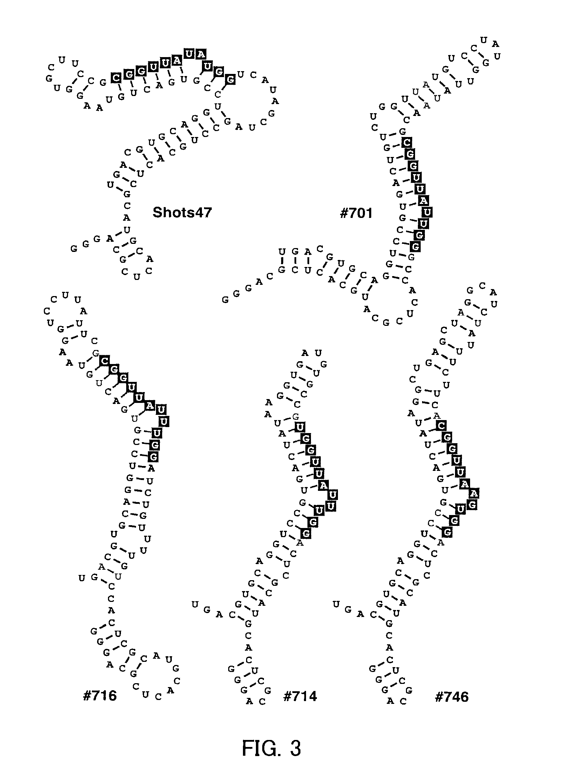

As examples of the aptamer, FIG. 3 shows the predicted secondary structures of the aptamer shot 47 (SEQ ID NO: 2), the aptamer #701 (SEQ ID NO: 1), the aptamer #716 (SEQ ID NO: 3), the aptamer #714 (SEQ ID NO: 10), and the aptamer #746 (SEQ ID NO: 9). In FIG. 3, the sequences in white letters enclosed in the black boxes each refer to the binding motif sequence represented by SEQ ID NO: 17, which is the consensus sequence among them. In FIG. 3, the binding motif sequence is positioned at the site in which the stem is bent. However, the present invention is not limited thereto.

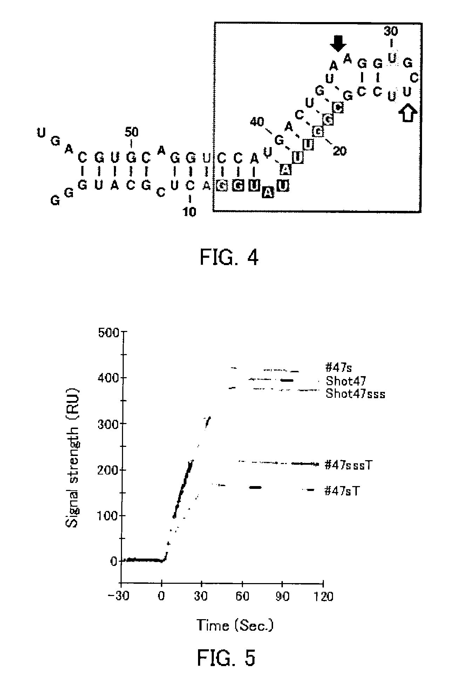

As examples of the aptamer, FIG. 4 shows the predicted secondary structure of the RNA aptamer shot 47 (SEQ ID NO: 2), the RNA aptamer #701 (SEQ ID NO: 1), the RNA aptamer #714 (SEQ ID NO: 10), and the RNA aptamer #746 (SEQ ID NO: 9). In FIG. 4, the sequence in white letters enclosed in the black box refers to the consensus sequence represented by SEQ ID NO: 17, and the consensus sequence is positioned at the site in which the stem is bent. However, the present invention is not limited thereto.

The aforementioned embodiment is not particularly limited. For example, an aptamer that includes a Y region, an X region, and a Y' region, wherein the Y region, the X region, and the Y' region are coupled from the 5' end can be employed as an example. In the aptamer of this embodiment, preferably, the X region includes any of the base sequences contained in the nucleic acids (a) to (h), for example. Specifically, for example, the X region preferably includes a base sequence represented by any of the SEQ ID NOs mentioned in Tables 1, 3, and 4.

Preferably, the aptamer includes a primer sequence capable of being annealed by a primer and a polymerase recognition sequence capable of being recognized by polymerase at at least one of the 5' side upstream of the X region, i.e., the Y region and the 3' side downstream of the X region, i.e., the Y' region, for example. By including the primer sequence and the polymerase recognition sequence in this manner, for example, the aptamer can be amplified by a reverse transcription reaction and/or a nucleic acid amplification reaction using a primer, a polymerase, and the like. The polymerase recognition sequence can be determined suitably according to the type of polymerase to be used for a nucleic acid amplification, for example. Preferably, the recognition sequence of polymerase is the recognition sequence of DNA-dependent RNA polymerase (hereinafter, also referred to as the "RNA polymerase recognition sequence"), for example. A specific example thereof includes a T7 promoter, which is the recognition sequence of T7 RNA polymerase. In the case where the aptamer of this embodiment is RNA, preferably, the Y region at the 5' end side includes the RNA polymerase recognition sequence and the primer sequence (hereinafter, also referred to as the "5' end side primer sequence") in this order, for example. Further, preferably, the X region is coupled to the 3' end side of the Y region. Furthermore, preferably, the Y' region is coupled to the 3' end side of the X region and the Y' region includes the primer sequence (hereinafter, also referred to as the "3' end side primer sequence"). Preferably, the 5' end side primer sequence in the RNA is a complementary sequence to the 3' end of a DNA antisense strand synthesized with the RNA as a template, i.e., a sequence similar to the primer that is bindable to the 3' end of the antisense strand, for example. Further, in the aptamer of this embodiment, for example, the Y region and the X region may be directly adjacent to each other and the X region and the Y' region may be directly adjacent to each other or the Y region and the X region may be indirectly adjacent to each other via an intervening sequence and the X region and the Y' region may be indirectly adjacent to each other via an intervening sequence. There arc no particular limitations on the Y region and the Y' region. For example, those skilled in the art can suitably set the Y region and the Y' region according to the type of primer to be used and the type of polymerase to be used.

The base sequence of the Y region and the base sequence of the Y' region are not particularly limited and can be determined appropriately. Examples of the Y region include a region consisting of the base sequence represented by SEQ ID NO: 149 and a region that includes the base sequence represented by SEQ ID NO: 149. Further, examples of the Y' region include a region consisting of the base sequence represented by SEQ ID NO: 150 and a region that includes the base sequence represented by SEQ ID NO: 150. These sequences are mere illustrative and do not limit the present invention.
GGGACGCUCA CGUACGCUCA (SEQ ID NO: 149)
UCAGUGCCUG GACGUGCAGU (SEQ ID NO: 150)

Specific examples of the aptamer that includes the Y region, the X region, and the Y' region include a nucleic acid consisting of any of the base sequences represented by the SEQ ID NOs mentioned in Tables 2, 5, and 6 and a nucleic acid that includes any of the base sequences represented by the SEQ ID NOs mentioned in Tables 2, 5, and 6. In Tables 2, 5, and 6, for example, lower-case sequences at the 5' end side each correspond to the Y region consisting of the base sequence represented by SEQ ID NO: 149, capital sequences each correspond to the X region, and lower-case sequences at the 3' end side each correspond to the Y' region consisting of the base sequence represented by SEQ ID NO: 150.

There is no particular limitation on the number of bases in the X region, and the number of bases is, for example, 10 to 60, preferably 15 to 50, and more preferably 20 to 40. There is no particular limitation on the number of bases in each of the Y region and Y' region, and the number of bases is, for example, 10 to 50, preferably 15 to 40, and more preferably 20 to 30. There is no particular limitation on the total number of bases of the aptamer, and the total number of bases is, for example, 20 to 160, preferably 30 to 120, and more preferably 40 to 100.

Besides the aforementioned examples, for example, the aptamer can be prepared, for example, by the SELEX method and the modified SELEX method (hereinafter, referred to as the "SELEX-T method") that will be described below. Note here that the present invention is not limited to the following methods.

The SELEX-T method includes the following steps (i) to (iv), for example.
(i) step of mixing an RNA pool and a substance that includes a His tag, e.g., fusion peptide to which a His tag is added (hereinafter, referred to as the "His tag-peptide")
(ii) step of separating RNA that binds to the His tag-peptide from the RNA pool
(iii) step of subjecting the separated RNA to a reverse transcription-polymerase chain reaction (RT-PCR) to synthesize a DNA product (cDNA)
(iv) step of synthesizing RNA from the DNA product by RNA polymerase

The RNA pool is, for example, an RNA group containing random sequences. The random sequence is, for example, a sequence consisting of 10 to 60 ofN (A, C, G, U, or T) and is preferably a sequence consisting of 30 to 50 ofN (A, C, G, U, or T). Further, RNA in the RNA pool may be the one in which a predetermined primer sequence and a predetermined promoter sequence (or their complementary sequences), are functionally coupled to the both ends of the random sequence, for example, in consideration of the RT-PCR step (iii) and the RNA synthesis step (iv) by RNA polymerase.

Hereinafter, an example of the SELEX-T method will be described. First, the RNA pool and the His tag-peptide are provided. For example, the RNA pool may be chemically synthesized using an automated nucleic acid synthesizer or may be synthesized by *in vitro* transcription from a DNA template. The His tag-peptide may be a His tag-peptide commercially available, a His tag-peptide isolated from a biological sample, or a His tag-peptide synthesized by transcription/translation *in vitro,* for example.

Next, the RNA pool is mixed with the His tag-peptide. At this time, the His tag-peptide may be immobilized on a carrier or a support. The His tag-peptide may be immobilized before mixing the RNA pool and the His tag-peptide or after mixing the RNA pool and the His tag-peptide, for example. For immobilizing the His tag-peptide, for example, the biotin-avidin binding, Ni²⁺-His tag binding or Co²⁺-His tag binding, covalent binding by a chemical crosslinker, nucleic acid hybridization, and the like may be employed. Examples of the carrier and the support include beads, chips, and resins. The conditions for mixing the RNA pool and the His tag-peptide are not limited as long as they can be specifically bound to each other, for example. Specifically, the temperature is, for example, 4 to 40°C and preferably 20 to 37°C; pH is, for example, 5.0 to 9.0 and preferably 6.5 to 7.5; the salt concentration is, for example, 50 to 500 mmol/L and preferably 100 to 150 mmol/L; and the treatment time is, for example, 10 minutes to 18 hours and preferably 30 minutes to 2 hours.

After mixing the RNA pool and the His tag-peptide, the complex of the RNA and the His tag-peptide formed is subjected to washing, elution, purification, or the like to separate RNA bound to the His tag-peptide. The washing can be performed according to an ordinary SELEX method or can be performed under milder conditions than the SELEX method, for example. Examples of the washing include a method in which a carrier or the like to which the complex is immobilized is precipitated and then the supernatant is removed and a method in which the carrier or the like to which the complex is immobilized is washed with a washing buffer of 100 times the volume of the carrier or the like after the supernatant is removed. There is no limitation how many times the washing by a washing buffer is performed, and the washing is performed, for example, once. The elution can be performed using imidazole having a predetermined concentration, for example, 100 to 300 mmol/L, as an elution solvent. Examples of the purification include phenol chloroform extraction and ethanol precipitation.

Next, the purified/separated RNA is subjected to RT-PCR to synthesize a DNA product (cDNA). For example, the RNA is added to a reaction solution that contains dNTP Mix, a predetermined primer, a reverse transcription enzyme, DNA polymerase, and the like and subjected to one-step RT-PCR. For RT-PCR, QIAGEN (registered trademark) OneStep RT-PCR Kit can be used, for example. The conditions for RT-PCR are as follows. For example, after treating at 50°C for 30 minutes and at 95°C for 10 minutes, one cycle of treatment at 94°C for 1 minute, 56°C for 1 minute, and at 72°C for 1 minute is repeated for 5 cycles. Further, the reaction solution is treated at 72°C for 5 minutes. Here, in the SELEX-T method, for example, in order to suppress a bias by PCR (sequence deviation of RNA pool), as compared to an ordinary SELEX method, preferably, the number of cycles of RT-PCR is, for example, 1 to 10 and is preferably 4 to 6.

Then, using the synthesized DNA product as a template, RNA (that is, RNA aptamer) is synthesized by RNA polymerase. There is no particular limitation on RNA polymerase and any RNA polymerase can be used and can be appropriately selected. An example of RNA, polymerase includes thermostable T7 RNA polymerase (ScriptMAX Thermo T7 Transcription Kit, produced by Toyobo Co., Ltd.). In the case where the thermostable T7 RNA polymerase is used as RNA polymerase, for example, at the time of producing an RNA pool, by coupling a T7 promoter to one end of the random sequence, RNA can be synthesized by the thermostable T7 RNA polymerase. There are no particular limitations on the conditions for the RNA synthesis by RNA polymerase. For example, the synthesis can be performed at 37°C to 50°C for 2 hours to 6 hours.

After the RNA synthesis, the obtained RNA is subjected to DNase I treatment, gel filtration, phenol chloroform extraction, ethanol precipitation, and the like to purify/separate it. In this manner, an aptamer that is bindable to a His tag can be obtained.

With respect to the obtained RNA, the procedures from the mixing step with the His tag-peptide to the RNA synthesis step can be performed repeatedly. There is no particular limitation on the number of repetitions (the number of rounds), and the number of repetitions is, for example, 5 to 10 and preferably 6 to 8. The binding affinity between the obtained RNA aptamer and the His tag-peptide can be determined by a surface plasmon resonance molecular interaction analysis or the like using BiacoreX (produced by GE Healthcare UK Ltd.), for example.

Preferably, the nucleic acid construct of the present invention is a screening nucleic acid construct for screening peptide that is bindable to a target or its encoding nucleic acid, for example.

### <Formation method of complex>

As described above, the complex formation method of the present invention is a complex formation method for forming a complex of a fusion transcript having a base sequence that includes an encoding nucleic acid of arbitrary peptide, an encoding nucleic acid of a peptide tag, and an encoding nucleic acid of an aptamer that is bindable to the peptide tag and a fusion translation that includes the arbitrary peptide and the peptide tag that includes the step of: expressing the nucleic acid construct of the present invention, the nucleic acid construct including:
a cloning region for being inserted with an encoding nucleic acid of arbitrary peptide;
an encoding nucleic acid of a peptide tag, wherein the peptide tag is a histidine tag; and
an encoding nucleic acid of an aptamer that is bindable to the peptide tag, the encoding nucleic acid of arbitrary peptide being inserted into the cloning region.

The complex formation method of the present invention is characterized by the use of the nucleic acid construct of the present invention in which the arbitrary encoding nucleic acid is inserted, and other steps and conditions are not limited at all. The complex formation method of the present invention will be described below with the screening method of the present invention.

### <Screening method>

As described above, the screening method of the present invention is a screening method for screening peptide that is bindable to a target or an encoding nucleic acid of the peptide that includes the steps of:
(A) forming a complex by the complex formation method of the present invention;
(B) bringing the complex into contact with the target; and
(C) recovering the complex that is bound to the target.

First, the step (A), i.e., the complex formation method of the present invention will be described. In the step (A), the nucleic acid construct of the present invention in which the arbitrary encoding nucleic acid is inserted into the cloning region is expressed. Thereby, a fusion transcript having a base sequence containing the arbitrary encoding nucleic acid, the peptide tag-encoding nucleic acid, and the aptamer-encoding nucleic acid is transcribed, and a fusion translation including the arbitrary peptide and the peptide tag is translated. Further, since the aptamer is bindable to the peptide tag, the aptamer in the fusion transcript binds to the peptide tag in the fusion translation. Thereby, a complex of the fusion transcript and the fusion translation is formed.

The complex may be formed *in vivo* or *in vitro,* for example. In the former case, for example, the nucleic acid construct is expressed *in vivo.* Specifically, preferably, the nucleic acid construct is expressed using a cell such as a living cell or the like to form the complex in the cell, for example. Further, in the latter case, for example, the nucleic acid construct is expressed *in vitro.* Specifically, preferably, the nucleic acid construct is expressed using a cell-free protein synthesizing system or the like, for example. The present invention is preferably the former case because of, for example, a simple operation.

In the case where the complex is formed *in vivo,* there are no particular limitations on the type of the cell, and examples of the cell include various hosts. Examples of the host include bacteria such as *Escherichia* such as *Escherichia coli, Bacillus* such as *Bacillus subtilis, Pseudomonas* such as *Pseudomonas putida,* and *Rhizobium* such as *Rhizobium meliloti;* and yeasts such as *Saccharomyces cerevisiae* and *Schizosaccharomyces pombe.* Further, as the host, for example, animal cells such as a COS cell and a CHO cell; insect cells such as Sf9 and Sf21; and the like can be used. The host can be determined suitably according to the type of the vector of the nucleic acid construct, for example. In the present invention, there is no particular limitation on the combination of the host and the vector. For example, the combination that achieves superior induction of expression of peptide such as protein or the like, superior efficiency of transfection, and the like is preferable. Specifically, for example, the host is preferably *Escherichia coli* and the vector is preferably a vector derived from *Escherichia coli* and is more preferably pCold, which is a cold shock expression vector, because pCold achieves the induction at a low temperature. Since the cold expression vector can prevent insolubilization and promote solubilization of peptide expressed in a cell such as *Escherichia coli* or the like as described above, for example, the expressed peptide can be recovered easily. Further, since the insolubilization of peptide is caused by formation of a peptide inclusion body, for example, there is a need for breaking the inclusion body to take out peptide. However, since such treatment is unnecessary if the aforementioned vector is used, for example, dissociation of the binding between the fusion transcript and the fusion translation in the complex due to breaking of the inclusion body can be prevented sufficiently. Further, since the cold shock expression vector can suppress the expression of a host-derived protein by an expression induction at a low temperature, for example, a protein derived from the nucleic acid construct can be synthesized efficiently.

The nucleic acid construct can be expressed *in vivo,* for example, by transfecting the nucleic acid construct into the cell and performing an induction of expression of peptide such as protein or the like with respect to the cell of after transfection.

There is no particular limitation on the transfection method of the nucleic acid construct, and, for example, the method can be determined suitably according to the type of the cell, type of the vector, and the like. Examples of the transfection method include a protoplast method, a lithium acetate method, the Hanahan method, an electroporation method, a transfection method by infection using a virus vector or the like, a calcium phosphate method, a lipofection method, a transfection method by infection using bacteriophage or the like, a transfection method by ultrasonic nucleic acid, a transfection method by a gene gun, and the DEAE-dextran method.

There is no particular limitation on the method of inducing peptide expression, and, for example, the induction of peptide expression can be performed by culturing the cell of after transfection. There are no particular limitations on the conditions for the culture, and the conditions can be determined suitably according to the type of the cell, the type of the vector, and the like. Specifically, in the case where the cell is *Escherichia coli,* the culture conditions are, for example, as follows. That is, preferably, the culture temperature is 20 to 40°C and the culture time is 0.5 to 6 hours; and more preferably, the culture temperature is 30 to 37°C and the culture time is 1 to 3 hours. Examples of the culture medium to be used include an LB culture medium, an NZYM culture medium, a Terrific Broth culture medium, an SOB culture medium, an SOC culture medium, and a 2 × YT culture medium.

Further, in the case where the basic vector in the nucleic acid construct is pCold, for example, expression induction at a low temperature is possible. Therefore, the culture conditions at the time of expression induction are, for example, as follows. That is, preferably, the culture temperature is 4 to 18°C and the culture time is 1 to 24 hours; and more preferably, the culture temperature is 10 to 16°C and the culture time is 12 to 24 hours. Further, at the time of culturing, an inducer for inducing expression may be added to a culture medium suitably according to the type of the cell and the type of the vector. There is no particular limitation on the inducer, and an example of the inducer includes isopropyl-1-thio-β-galactoside (IPTG). The concentration of the inducer in the culture medium is, for example, 0.1 to 2 mmol/L and preferably 0.5 to 1 mmol/L.

For example, the library of the nucleic acid construct may be transfected into the cell. In other words, the library containing plural nucleic acid constructs each including a different arbitrary encoding nucleic acid may be transfected into the cell. Specifically, for example, the library containing plural nucleic acid constructs each including a different random arbitrary encoding nucleic acid may be transfected into the cell.

The step (B) is a step of bringing the complex obtained in the step (A) into contact with a target. The complex is a complex of a fusion transcript and a fusion translation as described above. If arbitrary peptide in the fusion translation is bindable to the target, the complex bindes to the target via the arbitrary peptide, for example.

In the step (A), in the case where the complex is formed *in vivo,* for example, the complex is recovered from the inside of the cell and brought into contact with the target. There is no limitation at all on the method of recovering the complex from the cell, and the method can be selected suitably according to the type of the cell.

Further, in the step (A), in the case where the complex is formed *in vitro,* for example, the complex is recovered from the cell-free protein synthesizing system or the like and brought into contact with the target.

There is no limitation at all on the type of the target, and the target may be any of peptide such as protein; hormones; nucleic acids; low molecular compounds; organic compounds; inorganic compounds; saccharides; lipids; viruses; bacteria; cells; biological tissues; and the like. For example, the target is preferably an immobilized target that is immobilized on a solid phase because it can be handled easily, for example. There is no particular limitation on the solid phase, and examples of the solid phase include plates such as a well plate and a microplate; a chip; a bead such as microsphere; a gel; a resin; a membrane such as a cellulose membrane; a film; a test tube; a micro tube; a plastic container; a cell, a tissue or a fixed paraffin section containing the target; and a particle. The target is also referred to as a target substance or a target molecule, for example.

For example, the solid phase is preferably insoluble. There is no particular limitation on the insoluble material, and examples of the insoluble material include organic resin materials and inorganic materials. The organic resin material may be, for example, a natural material or a synthesized material. Specific examples of the organic resin material include agarose, crosslinking agarose, crosslinking dextran, polyacrylamide, crosslinking polyacrylamide, cellulose, microcrystalline cellulose, crosslinking agarose, polystyrene, polyester, polyethylene, polypropylene, an ABS resin, polyvinyl fluoride, a polyamine-methyl vinyl-ether-maleic acid copolymer, 6-nylon, 6,6-nylon, and latex. Examples of the inorganic material include a glass, a silica gel, diatomaceous earth, titanium dioxide, barium sulfate, zinc oxide, lead oxide, and silica sand. The solid phase may contain one of or more than one of the aforementioned insoluble materials.

In the case where the solid phase is a particle, for example, the particle is preferably a magnetic particle. If the solid phase is a magnetic particle, for example, the magnetic particle can be recovered easily by a magnetic force.

The target may be bound to the solid phase directly or indirectly, for example. The immobilization of the target to the solid phase may be physical bonding or chemical bonding, for example, and specific examples thereof include adsorption and chemical bonding such as covalent bonding.

There are no particular limitations on the conditions for the contact between the complex and the target, and the conditions can be determined suitably according to the type of the target, for example. The conditions are, for example, as follows. Preferably, the temperature is 4 to 37°C, pH is 4 to 10, and the time is 10 minutes to 60 minutes; and more preferably, the temperature is 4 to 20°C, pH is 6 to 9, and the time is 15 to 30 minutes. The complex and the target are brought into contact with each other preferably in a solvent, for example. As the solvent, for example, buffer solutions such as a HEPES buffer solution, a carbonate buffer solution, and a phosphate buffer can be used.

The step (C) is a step of recovering the complex that is bound to the target. The complex is bound to the target via arbitrary peptide in the fusion translation thereof. Therefore, by recovering the complex that is bound to the target, the peptide that is bindable to a target and the encoding nucleic acid of the peptide can be selected. It is considered that the arbitrary peptide in the complex recovered in the step (C) is bindable to the target. Therefore, the arbitrary peptide in the complex is also referred to as the "candidate peptide", the encoding nucleic acid of the candidate peptide is also referred to as the "candidate encoding nucleic acid", and the complex containing the candidate peptide is also referred to as the "candidate complex".

The complex that is bound to the target may be recovered in the state where it is bound to the target or the state where it is liberated from the target, for example.

The recovery of the complex that is bound to the target can be performed by washing the target, for example. In this manner, for example, the complex that is bound to the target can be exclusively recovered by removing the complex that is not bound to the target by washing the target. In this case, the target is preferably immobilized on the solid phase as described above. By washing the solid phase, for example, the complex that is not bound to the target that is immobilized on the solid phase is removed. Since the complex that is bound to the immobilized target remains on the solid phase, the complex can be recovered in the state where it is bound to the target.

The step (C) may further include a step of liberating the complex that is bound to the target from the target, for example. There is no particular limitation on the method of liberating the complex from the target.

Furthermore, the step (C) may further include a step of liberating the complex transcript composing the complex from the complex, for example. The complex transcript may be liberated after recovering the complex from the target or may be liberated from the complex that is bound to the target, for example. There is no limitation at all on the method of liberating the complex transcript from the complex. For example, an eluate that contains phenol or the like can be used. An example of the eluate that contains phenol includes Trizol (product name, produced by Invitrogen).

Preferably, the screening method of the present invention further includes the following step (D). (D) synthesizing the arbitrary encoding nucleic acid using a transcript of the arbitrary encoding nucleic acid in the complex recovered in the step (C) as a template.

In this manner, by further synthesizing the arbitrary encoding nucleic acid using the transcript of the arbitrary encoding nucleic acid in the complex that is bound to the target as a template, arbitrary peptide that is bindable to the target and its encoding nucleic acid can be identified. The synthesized arbitrary encoding nucleic acid may be identified after cloning, for example. With respect to the arbitrary encoding nucleic acid, for example, by identifying the base sequence, the amino acid sequence of the arbitrary peptide can be identified indirectly.

In the step (D), preferably, the synthesis of the arbitrary encoding nucleic acid is performed by the RT-PCR, for example. Specifically, preferably, the arbitrary encoding nucleic acid is synthesized by a reverse transcription reaction using the transcript of the arbitrary encoding nucleic acid as a template, and further the synthesized arbitrary encoding nucleic acid is amplified, for example.

The synthesis of the arbitrary encoding nucleic acid using a transcript of the arbitrary encoding nucleic acid as a template may be performed, for example, in the state where the transcript of the arbitrary encoding nucleic acid is contained in the complex or the state where the fusion transcript is liberated from the complex.

According to the screening method of the present invention, as described above, for example, by the steps (A), (B), and (C), arbitrary peptide that is bindable to the target and its encoding nucleic acid can be selected. Further, by the step (D), the arbitrary peptide and its encoding nucleic acid can be identified.

For example, in the screening method of the present invention, preferably, the arbitrary encoding nucleic acid obtained in the step (D) is again inserted into the cloning region of the nucleic acid construct of the present invention and the steps (A), (B), and (C) are performed again. More preferably, the steps (A), (B), (C), and (D) are repeated more than one time.

As described above, by transfecting the library of the nucleic acid construct into the cells, plural transformants in which different nucleic acid constructs are transfected are obtained. Further, by performing the culture in the state where the plural transformants are present, a complex mix in which complexes derived from the respective transformants are present can be obtained. By subjecting this complex mix to the steps (B) and (C), for example, plural complexes that are bound to the targets are recovered. Hence, in the step (D), with respect to the plural complexes recovered in the step (C), for example, the respective arbitrary encoding nucleic acids are synthesized. Then, preferably, the synthesized arbitrary encoding nucleic acids are again inserted into the cloning regions of the nucleic acid constructs of the present invention, plural libraries of the nucleic acid constructs are produced, and the steps (A), (B), and (C) are performed in the same manner as described above. Thereby, arbitrary peptide that is bound to the target and its encoding nucleic acid can be further concentrated and arbitrary peptide having a good binding affinity to the target and its encoding nucleic acid can be selected. When the steps (A), (B), (C), and (D) are regarded as 1 cycle, there is no particular limitation on the number of cycles, and the number of cycles is preferably at least 2, for example.

Further, in the case where the libraries of the nucleic acid construct are transfected into the cells, for example, the steps (A), (B), and (C) and further the step (D) can be performed after separating plural transformants into each clone or separating plural transformants into plural groups each including several clones. The separation of the transformants into each clone or groups may be performed at the stage in the first cycle or at the stage after the first cycle, for example.

There is no particular limitation on the method of evaluating the binding affinity of the complex to the target. A specific example thereof includes a method in which a labeled anti-peptide tag antibody that is labeled with a labeling substance is used. In this method, for example, after bringing the labeled peptide tag antibody into contact with the substance in which the target and the complex are bound, detection of the labeled anti-peptide tag antibody is performed. By detecting the labeled anti-peptide tag antibody, the presence or absence of the peptide tag can be determined. In other words, if the complex is bound to the target, the labeled anti-peptide tag antibody binds to the peptide tag in the complex. Therefore, by detecting the labeled anti-peptide tag antibody, it can be determined that the complex is indirectly bound to the target. On the other hand, if the complex is not bound to the target, the peptide tag is not present. Therefore, the labeled anti-peptide tag antibody cannot be detected and it can be determined that the complex is not bound to the target. An example of the labeling substance of the labeled anti-peptide tag antibody includes horseradish peroxidase (HRP), and an example of the detection reagent for detecting the HRP includes a coloring reagent such as 3,3',5,5'-tetramethylbenzidine (TMB). Further, for example, depending on whether or not the molecular weight of the target is increased, the binding of the complex can be determined.

In the screening method of the present invention, the evaluation of the binding affinity may be performed in the step (C) or the evaluation of the binding affinity may be performed after selecting the candidate peptide and the candidate encoding nucleic acid, for example.

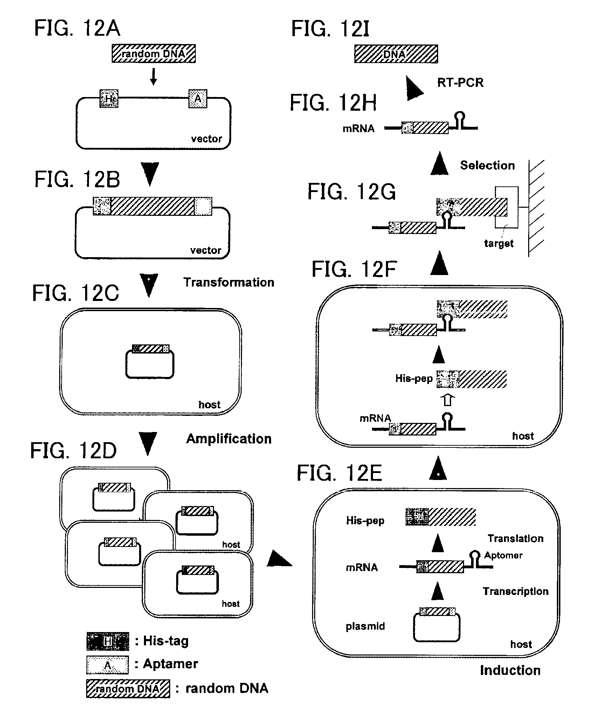

Hereinafter, an example of the screening method of the present invention will be described using FIGs. 12A to 12I. FIGs. 12A to 12I show the outline of the screening method in which a complex is formed *in vivo.* In this example, a vector is employed as the nucleic acid construct of the present invention and the peptide tag is a his tag.

First, as shown in FIG. 12A, random DNA that encodes random peptide as the arbitrary encoding nucleic acid is inserted into a vector including the His tag-encoding nucleic acid (H) and the aptamer-encoding nucleic acid (A) to produce a recombinant vector (FIG. 12B). At this time, the His tag-encoding nucleic acid (H) and the random DNA are arranged so as to be a correct reading frame.

Then, the recombinant vector is transfected into a host to conduct transformation (FIG. 12C). Subsequently, the obtained transformant is amplified (FIG. 12D), and induction of peptide expression is performed (FIG. 12E). As shown in FIG. 12E, by the expression induction, first, from the His tag-encoding nucleic acid (H), the random DNA, and the aptamer-encoding nucleic acid (A) in the recombinant vector, a fusion transcript (fusion mRNA) that contains the respective transcripts is formed, and further, on the basis of the fusion transcript, a fusion translation (fusion peptide: His-pep) that contains the His tag and the random peptide encoded with the random DNA is formed. Then, since an RNA aptamer in the fusion mRNA is bindable to the His tag, as shown in FIG. 12F, the RNA aptamer in the fusion mRNA binds to the fusion peptide to form a complex.

Subsequently, the complex and other proteins are taken out from the inside of the transformant and are brought into contact with a target immobilized on a solid phase. Here, if the random peptide in the complex is bindable to the target, the complex binds to the immobilized target via the random peptide (FIG. 12G). In this state, the His tag is bound to the random peptide that is bound to the immobilized target and the fusion mRNA is bound to the His tag via the aptamer. The transcript of the random DNA in this fusion mRNA is mRNA that encodes the random peptide that is bound to the immobilized target. Accordingly, by selecting the fusion transcript in the complex that is bound to the immobilized target (FIG 12H), the information on the random peptide that is bound to the immobilized target and on its encoding nucleic acid can be obtained. Specifically, RT-PCR is performed using the fusion transcript in the complex as a template, and cDNA is synthesized based on mRNA of the random DNA (FIG. 12I). Thereby, the information on the base sequence of the encoding nucleic acid of the peptide that is bindable to the target and on the amino acid sequence of the peptide can be obtained. Further, by transfecting the cDNA obtained by the RT-PCR into the vector again (FIG. 12A) and repeatedly performing a series of procedures, the encoding nucleic acid of the peptide that is bindable to the target can be further selected.

Next, the screening method of the present invention will be described with reference to the case in which the screening is performed by transfecting a plasmid library including random DNA as the nucleic acid construct into *Escherichia coli* as the cell as an example. Note here that the method described below is merely an example and the present invention is not limited thereto.

First, a plasmid library is transfected into *Escherichia coli* by electroporation or the like, and the resultant *Escherichia coli* is subjected to shaking culture. An example of the culture medium of the culture includes an LB culture medium containing ampicillin. For example, the culture of the *Escherichia coli* is preferably performed until the absorbance at the OD 600 nm becomes 0.5 to 0.6. Further, in the case where the vector of the plasmid library is the cold shock expression vector, for example, cold shock expression is preferably conducted at 15°C for 18 hours in the presence of 0.5 to 1 mmol/L IPTG.

Next, the cultured *Escherichia coli* is harvested by centrifugation, the harvested *Escherichia coli* is suspended in 50 mL of physiological saline containing 10 mmol/L EDTA, and the *Escherichia coli* is again harvested by centrifugation. The recovered *Escherichia coli* is suspended in 5 mL of 20 mmol/L HEPES buffer solution containing 20% sucrose and 1 mmol/L EDTA, 10 mg of lysozyme is added thereto, and the resultant is incubated on ice for 1 hour to dissolve a cell wall. Subsequently, Mg²⁺ is added thereto such that the final concentration becomes 2 mmol/L, and *Escherichia coli* is harvested by centrifugation. Then, the recovered *Escherichia coli* is suspended in 50 mL of physiological saline containing 0.1 mmol/L magnesium acetate, and centrifugation is again performed to recover spheroplast.

The recovered spheroplast is promptly suspended in 2.5 to 5 mL of 20 mmol/L HEPES buffer solution containing 0.05 to 0.5% Triton (registered trademark)-X100, 0.1 mmol/L magnesium acetate, 0.1mg/mL tRNA, 0.1% HSA or BSA (RNase free), and a protease inhibitor. After causing lysis, genomic DNA of *Escherichia coli* is shredded by mechanical shearing or DNase I. Then, NaCl is added thereto such that the final concentration becomes 150 mmol/L, the resultant is allowed to stand for 5 minutes, and the supernatant containing the complex is obtained by centrifugation. The supernatant can be stored at -80°C until the evaluation for the binding to the target is made, for example.

The supernatant containing the complex is brought into contact with the target and then incubated at 4°C for 10 to 30 minutes. As described above, the target is preferably immobilized on a solid phase. Examples of the solid phase on which the target is immobilized include a gel on which the target is immobilized, a plastic container on which the target is immobilized, a cell containing the target, and a tissue containing the target. Preferably, the solid phase on which the target is immobilized is preliminarily applied with blocking by the same HSA or BSA as that added at the time of lysis, for example.

Subsequently, the solid phase is washed with a washing liquid to remove the complex that is not bound to the target. An example of the washing liquid includes 20 mmol/L HEPES buffer solution containing 0.05 to 0.5% Triton (registered trademark)-X100, 0.1 mmol/L magnesium acetate, and 100 to 150 mmol/L NaCl.

Next, from the target immobilized on the solid phase, the complex is liberated and recovered by the eluate. Examples of the eluate include a buffer solution containing a denaturant such as Trizol (invitrogen), Isogen (Wako), 8 mol/L urea, 6 mol/L guanidine, or 1% SDS and a buffer solution further containing 0.05 to 0.5% Triton (registered trademark)-X100 and 1 to 10 mmol/L EDTA in addition to the denaturant. There is no particular limitation on the buffer solution, and an example of the buffer solution includes a Tris buffer solution.

Then, from the obtained complex, a fusion transcript (RNA) is purified. For the purification of RNA, for example, Trizol (product name, produced by invitrogen) or the like can be used. Further, in the case of performing precipitation of ethanol, for example, a precipitation aid such as tRNA, glycogen, or Ethatinmate (product name, produced by Nippongene) is preferably used. In the purification of RNA, preferably, after incubating at 37°C for 30 minutes using RNase free DNase, extraction of phenol chloroform and precipitation of ethanol are performed, for example.

Subsequently, using the purified RNA as a template, cDNA is synthesized by RT-PCR. Preferably, the synthesized cDNA is subjected to PCR for forming a complementary double-stranded cDNA, for example. In the case where plural random DNAs are used as arbitrary encoding nucleic acids, for example, there is a possibility that plural cDNAs each having the 5' side region and the 3' side region in the arbitrary encoding nucleic acid that are in common among the cDNAs and having a sequence of an intermediate region that differs among the cDNAs are synthesized by the RT-PCR and heteroduplex cDNA is formed. Hence, preferably, PCR with respect to the cDNA synthesized by RT-PCR is further performed to elongate a complementary strand and to amplify complementary double-stranded cDNA. There is no particular limitation on the method of amplifying the complementary double-stranded cDNA. For example, the amplification of the complementary double-stranded cDNA can be performed by a method in which a forward primer and a reverse primer are further added to the reaction solution of the RT-PCR and the annealing reaction and the elongation reaction are repeatedly performed after denaturation. There is no particular limitation on the amount of the primer added to the reaction solution. Preferably, each primer is added to the reaction solution such that the final concentration becomes 10 mmol/L, for example. Further, preferably, the thermal denaturation is performed, for example, by treating at 95°C for 30 seconds and then treating at 94°C for 3 minutes; the annealing reaction is performed, for example, at 63.2°C for 3 minutes; the elongation reaction is performed, for example, at 72°C for 3 minutes. Preferably, the annealing reaction and the elongation reaction are repeated 5 times, for example. In this manner, the complementary double-stranded cDNA can be obtained.

Preferably, the obtained double-stranded cDNA is again inserted into a vector such as the aforementioned plasmid and a series of procedures described above is performed repeatedly. Thereby, arbitrary plasmid that is bindable to a target can be selected.

Further, for improving selection efficiency, for example, after transfecting the nucleic acid construct into *Escherichia coli,* the *Escherichia coli* may be dispensed to a multiwell plate to limit clones. Specifically, the *Escherichia coli* is amplified in the plate, some of the cultured *Escherichia coli* are stored, and then an expression induction is conducted and lysis is caused. The lysis can be caused, for example, by adding 20 mmol/L HEPES buffer solution containing 0.05 to 0.5% Triton (registered trademark)-X100, 1 mmol/L EDTA, 2 mg/mL, lysozyme, and 1 mg/mL DNase after harvesting the *Escherichia coli* in the plate. The thus prepared lysate is added to a plate on which the target is immobilized, and the complex in the lysate is allowed to bind to the target. Thereafter, the plate is washed with 20 mmol/L HEPES buffer solution containing 0.05 to 0.5% Triton (registered trademark)-X100 and 1 mmol/L EDTA, and then the complex that is bound to the target is detected using an HRP-labeled-anti-His tag antibody or the like as described above. Thereby, the well containing a plenty of clones that form complexes that bind to the targets is specified. Further, from the preliminarily stored *Escherichia coli,* the *Escherichia coli* of the corresponding well is selected and amplified, and selection is subsequently performed by a series of procedures.

### <Kit>

The complex formation kit of the present invention is a kit used for the complex formation method of the present invention and includes the nucleic acid construct of the present invention. The complex formation kit of the present invention is characterized by including the nucleic acid construct of the present invention, and other configurations and the like are not limited at all.

The screening kit of the present invention is a kit used for the screening method of the present invention and includes the nucleic acid construct of the present invention. The screening kit of the present invention is characterized by including the nucleic acid construct of the present invention, and other configurations and the like are not limited at all.

Each of the kits of the present invention may further include a living cell to be transfected with the nucleic acid construct. Further, each of the kits of the present invention may include a reagent, an instruction manual, and the like for transfecting the nucleic acid construct into the living cell, for example.

### Examples

Next, Examples of the present invention will be described. However, the present invention is not limited by the following Examples. Commercially available reagents were used based on the protocols thereof unless otherwise noted.

### [Example 1]

An aptamer was produced and the binding affinity thereof was confirmed.

### 1. Materials and methods

### (1) Reagent

As for monoclonal antibodies, an anti-GFP antibody (JL-8) was purchased from TAKARA BIO INC., an anti-His-tag antibody was purchased from QIAGEN GmbH, and an anti-MIF antibody (MAB289) was purchased from R&D Systems Inc. An HRP-anti-MIF antibody was purchased from R&D systems Inc.

### (2) RNA aptamer

The RNA aptamers represented by SEQ ID NOs: 1 to 12 and 26 to 47 shown in Tables 2 and 5 were synthesized. In Tables 2 and 5, lower-case sequences each refer to the consensus sequence and capital sequences each refer to a random sequence.

FIGs. 3 and 4 are schematic views showing the secondary structures predicted with respect to the aforementioned RNA aptamers. FIG. 3 shows the predicted secondary structures of the RNA aptamer shot 47 (SEQ ID NO: 2), the RNA aptamer #701 (SEQ ID NO: 1), the RNA aptamer #714 (SEQ ID NO: 10), the RNA aptamer #716 (SEQ ID NO: 3), and the RNA aptamer #746 (SEQ ID NO: 9); and FIG. 4 shows the predicted secondary structures of the miniaturized aptamer #47s (SEQ ID NO: 12). These secondary structures are predicted using GENETYX-MAC software. In FIGs. 3 and 4, the sequences in white letters enclosed in the black boxes each refer to the consensus sequence represented by SEQ ID NO: 17. As shown in FIGs. 3 and 4, it was predicted that each of these RNA aptamers includes the consensus sequence at the site in which the stem is bent.

Further, on the basis of the information on the secondary structures of the respective aptamers, especially on the basis of the information on the secondary structures of the RNA aptamer shot 47 and the RNA aptamer #714, the miniaturized RNA aptamers represented by SEQ ID NOs: 12 to 16, 54 to 56, and 65 to 68 (hereinafter, referred to as the "miniaturized RNA aptamer") were synthesized.

### (3) Target protein

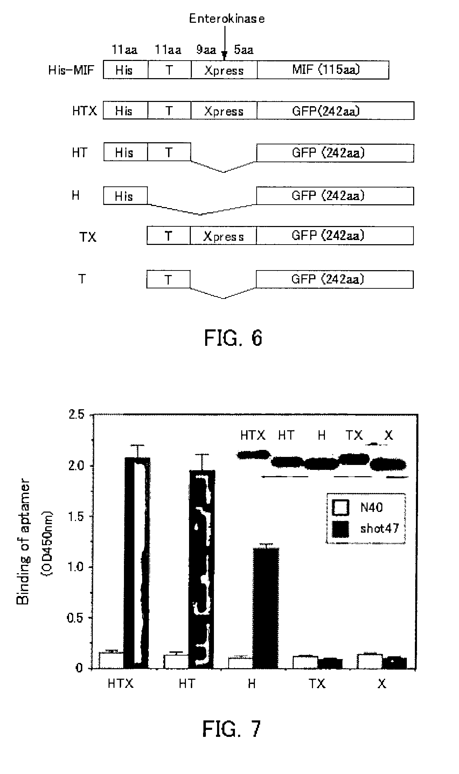

As target proteins, a fusion protein containing a tag region and a macrophage migration inhibitory factor (MIF) and fusion proteins each containing a tag region and GFP shown in FIG. 6 were provided. FIG. 6 is a schematic view showing the structures of the prepared six fusion proteins, namely His-MIF, HTX, HT, H, TX (not part of the invention), and T (not part of the invention). In FIG. 6, the "His" refers to the His tag (11 amino acid residues) that contains His peptide in which six His are coupled, the "T" refers to the peptide tag (11 amino acid residues) that contains the T7 gene 10 leader of 10 amino acid residues, and the "Xpress" refers to the Xpress™ Epitope of 14 amino acid residue (hereinafter, referred to as the "Xpress tag"), and three of them in total refer to the tag region. Further, in FIG. 6, the "MIF" is MIF of 115 amino acid residues and the "GFP," is GFP of 242 amino acid residues. The sequence of the "Xpress" can be cleaved by enterokinase between the 9^{th} amino acid from the N-terminal and the 10^{th} amino acid from the N-terminal. The amino acid sequences of the N-terminal regions of the respective fusion proteins shown in FIG. 6 and the corresponding base sequences, specifically, the base sequences and the amino acid sequences of the tag regions and MIF or GFP are shown in the following Table 8. In Table 8, with respect to each base sequence and each amino acid sequence of MIF and GFP, 9 bases at the 5' end and 3 amino acid residues at the N-terminal are exclusively described.

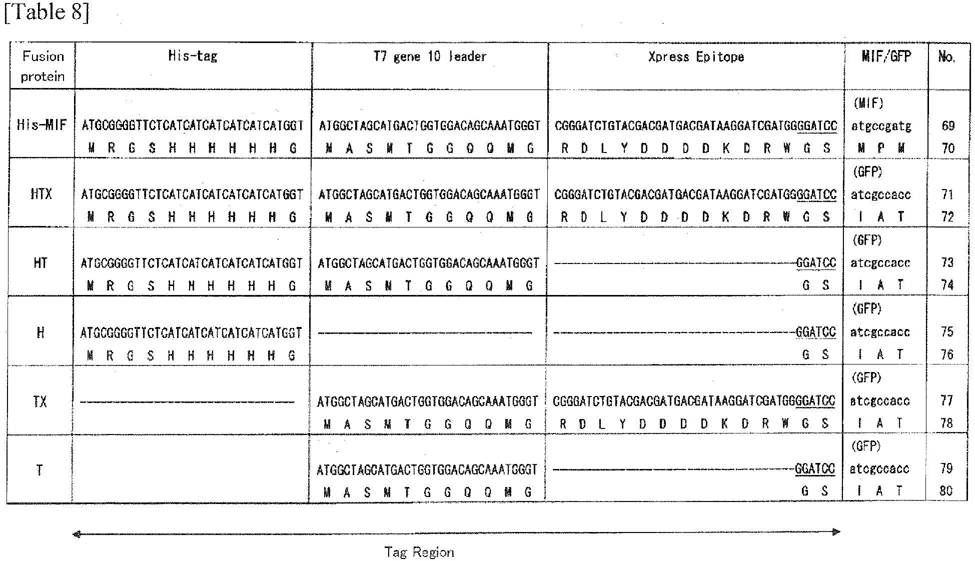

His-MIF, which is a fusion protein of a His tag and MIF, was purchased from ATGen Co., Ltd. (Gyeonggi-do, South Korea). The MIF that does not include a His tag was produced by treating the His-MIF with enterokinase (Novagen, EMD Chemicals, Inc., USA) to cleave the His tag (not part of the invention).

The fusion proteins (HTX, HT, H, TX, and T) each containing GFP were respectively prepared by the following methods. First, using a pRSETexpression vector (Invitrogen Corporation, USA) that includes the encoding DNA of the His tag, the encoding DNA of the T7 gene 10 leader, and the encoding DNA of the Xpress tag as a template, the DNA fragment of the tag region of each of the fusion proteins shown in Table 8 was amplified by PCR using a primer set. Each of the obtained DNA fragments and a GFP gene (TAKARA BIO INC., Japan) were integrated into a pCold IVexpression vector (TAKARA BIO INC., Japan), and this recombinant vector was transfected into *Escherichia coli* BL21 Star (DE3) (Invitrogen Corporation) to conduct transformation. Then, according to a standard method for using the pCold IVexpression vector, the transformant of the *Escherichia coli* was cultured in a culture medium containing 1 mmol/L isopropyl β-D-1-thiogalactopyranoside at 15°C for 18 hours to express the fusion protein. After cultivation, a fungus body was recovered by centrifugal separation (5,000 × g, 10 minutes), and the recovered fungus body was suspended it in 20 mmol/L HEPES (pH7.2) containing 1% Triton (registered trademark)-X100. After conducting freeze-thawing twice with respect to this suspension, the equal amount of 20 mmol/L HEPES (pH7.2) containing 300 mmol/L sodium chloride and 0.2 mmol/L magnesium acetate was added thereto, and the resultant was subjected to centrifugal separation (14,000 × g, 10 minutes). Then, the obtained supernatant was used as a fusion protein solution. The concentration of the fusion protein in the fusion protein solution was estimated from the result of the western blotting using a sample of after serial dilution and an anti-GFP antibody.

### (4) Molecular interaction analysis

The molecular interaction between each of the RNA aptamers and each of the fusion proteins, i.e., the binding affinity of each of the RNA aptamers to each of the fusion proteins was analyzed by a surface plasmon resonance. The analysis of the binding affinity was performed using BiacoreX (GE Healthcare UK Ltd.) according to the instruction manual. Specifically, first, poly A having a length of 20 bases was added to the 3' end of the RNA aptamer to prepare a poly A-added RNA aptamer, and the obtained poly A-added RNA aptamer was heated at 95°C for 5 minutes and then rapidly cooled on ice. Then, using a running buffer, the poly A-added RNA aptamer was introduced into a flow cell of a streptavidin chip (Sensor chip SA, GE Healthcare UK Ltd.) on which biotinylated poly-T having a length of 20 bases whose 5' end is biotinylated was bound. At this time, by the complementary binding between the poly A of the poly A-added RNA aptamer and the poly-T of the biotinylated poly-T, the poly A-added RNA aptamer was immobilized to the chip via the biotinylated poly-T. The poly A-added RNA aptamer was allowed to be bound until the resonance unit (RU; 1RU = 1pg/mm²) becomes 700 RU. Subsequently, a HEPES buffered saline (HBS) containing a fusion protein of a predetermined concentration was introduced into the chip using the running buffer, and the signal (RU) was measured. Here, the composition of the running buffer was as follows: 10 mmol/L HEPES (4-(2-hydroxyethyl)-1-piperazineethanesulfonic acid), 150 mmol/L sodium chloride, 0.1 mmol/L magnesium acetate, and 0.01 % Tween (registered trademark) 20 (pH7.2). Further, as a control, using the chip on which the poly A-added RNA aptamer was not immobilized and the biotinylated poly-T was bound, in the same manner as described above, the transfection of the fusion protein and the measurement of the signal were performed.

### (5) ELISA improvement method using RNA aptamer

Various antibodies (anti-GFP antibody, anti-His-tag antibody, and anti-MIF antibody) were each allowed to adsorb to a 96-hole plate (Iwaki, AGC TECHNO GLASS CO., LTD., Japan), and blocking was conducted using 1% bovine blood serum albumin. Next, 50µL of fusion protein (1 µg/mL), 20 mmol/L HEPES, 150 mmol/L sodium chloride, 0.1 mmol/L magnesium acetate, and 0.5% Triton (registered trademark)-X100 were added to the plate, and the plate was incubated at room temperature for 3 hours to bind the fusion protein to the plate. After incubation, the plate was washed with HBS-T three times. With respect to the control, the incubation and washing were performed in the same manner as described above except that 50 µL of HBS-T was added in place of 50 µL of fusion protein.

Next, poly A having a length of 20 bases was added to the 3' end of the RNA aptamer to prepare a poly A-added RNA aptamer. After denaturing the poly A-added RNA aptamer, it was mixed with biotinylated poly-T having a length of 20 bases (740 nmol/L) whose 5' end is biotinylated, tRNA (100 µg/mL), and an RNase inhibitor (0.16 units/mL), and then a biotin-labeled RNA aptamer was produced by the complementary binding between the poly A of the poly A-added RNA aptamer and the poly-T of the biotinylated poly-T. This biotin-labeled RNA aptamer was added to the plate and incubated at 4°C for 30 minutes. Subsequently, the plate was washed and then 0.1 µg/mL HRP-streptavidin (Thermo Fisher Scientific Inc., USA) was added to the plate. Further, after washing the plate, a 1-Step Ultra TMB substrate (Thermo Fisher Scientific Inc., USA) was added to the plate to develop colors, and the absorbance at 450 nm was measured.

### (6) Pull-down assay

In the same manner as in (5), a biotin-labeled RNA aptamer was produced. The biotin-labeled RNA aptamer (50 µL) and a solution containing a fusion protein were mixed in equal amount, and the mixture was incubated at 4°C for 15 minutes. Thereby, the biotin-labeled RNA aptamer was allowed to bind to a sample containing the fusion protein. As the samples each containing the fusion protein, the HBS-T (containing 200 µg/mL tRNA) to which His-MIF was added such that the final concentration becomes 10 µg/mL, a culture supernatant (containing 5% fetal bovine serum) of rabbit renal cell line (RK-13 cell line) to which His-MIF was added such that the final concentration becomes 10 µg/mL, and the extract of *Escherichia coli* in which His-GFP (HT) is expressed were used. Next, 5 µL of streptavidin-Sepharose (GE Healthcare) was added to the mixture, and the mixture was incubated at 4°C for 1 hour to allow the biotin-labeled RNA aptamer to bind to the Sepharose. After incubation, the Sepharose was washed with the HBS-T 3 times, a sample buffer for SDS-polyacrylamide electrophoresis was added thereto, and the resultant was subjected to heat treatment at 95°C for 5 minutes. Thereby, the fusion protein that was bound to the Sepharose via streptavidin and biotin was eluted. The eluted protein was subjected to 15% SDS-polyacrylamide electrophoresis, and the protein was transcribed onto a PVDF membrane (Immobilon-P, Millipore). The membrane of after transcription was applied with the blocking with 5% skim milk, and 1 µg/mL antibody was added and allowed to bind thereto at room temperature for 3 hours. As the antibody, an anti-MIF antibody or an anti-His-tag antibody was used. Further, after washing the membrane, an HRP-anti-mouse IgG antibody (GE Healthcare) was allowed to bind thereto. Then, after washing, the presence of the fusion protein was checked using an ECL chemiluminescent reagent (GE Healthcare).

### (7) Northwestern blotting

The fusion protein of after serial dilution was subjected to non-reducing SDS-polyacrylamide electrophoresis, the blotting of the fusion protein to a PVDF membrane was performed in the same manner as in (6), and then the blocking of the membrane was conducted. Then, the biotin-labeled RNA aptamer prepared in the same manner as in (6) was added to the membrane in place of the antibody (anti-MIF antibody or anti-His-tag antibody). Further, HRP-streptavidin was added thereto, and the presence of the fusion protein was checked using the ECL chemiluminescent reagent (GE Healthcare) in the same manner as in (6).

### 2. Result

### (1) Molecular interaction analysis

### (1-1) Binding affinity of RNA aptamers to His-MIF

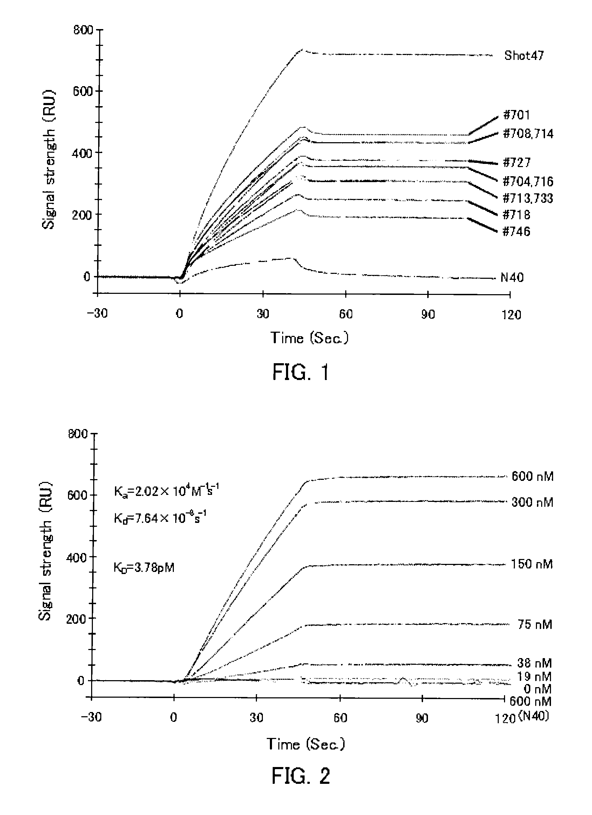

The binding affinity of each of the RNA aptamers to the fusion protein His-MIF was analyzed by the molecular interaction analysis except that the His-MIF concentration in the HBS-T to be introduced into the chip was 600 nmol/L. As the RNA aptamers, the nucleic acids represented by SEQ ID NOs: 1 to 11 and 26 to 47 each having 20-base-long consensus sequences at the 5' side and the 3' side were used. Among them, the results obtained by using the RNA aptamer #701 (SEQ ID NO: 1), the RNA aptamer shot 47 (SEQ ID NO: 2), the RNA aptamer #716 (SEQ ID NO: 3), the RNA aptamer #727 (SEQ ID NO: 4), the RNA aptamer #704 (SEQ ID NO: 5), the RNA aptamer #713 (SEQ ID NO: 6), the RNA aptamer #708 (SEQ ID NO: 7), the RNA aptamer #718 (SEQ ID NO: 8), the RNA aptamer #746 (SEQ ID NO: 9), the RNA aptamer #714 (SEQ ID NO: 10), and the RNA aptamer #733 (SEQ ID NO: 11) are shown in FIG. 1. FIG. 1 is a sensorgram of the signals detected using Biacore. In FIG. 1, the vertical axis indicates the signal strength (RU) measured by the BIACORE X and the horizontal axis indicates the analysis time (sec). In the horizontal axis, the period between 0 second and 45 seconds is the period in which the fusion proteins are introduced. Further, FIG. 1 also shows the result obtained by using RNA (hereinafter, N40) that is not bindable to the fusion protein His-MIF instead of the RNA aptamers as Comparative Example. The N40 was RNA having 20-base long consensus sequences, which are the same as those in the RNA aptamer, at the 5' side and the 3' side and having a 40-base-long random sequence between the consensus sequences.

As shown in FIG. 1, all of the RNA aptamers showed the binding affinity to the His-MIF. Among them, the RNA aptamer shot 47 showed an excellent binding affinity. Although it is not shown in FIG. 1, in addition to them, the binding affinity of each of the RNA aptamers represented by SEQ ID NOs: 26 to 47 to the His-MIF was also confirmed.

### (1-2) Binding affinity of shot 47

The molecular interaction analysis was performed with respect to the RNA aptamer shot 47 (SEQ ID NO: 2) in the same manner as described above except that the concentrations of His-MIF in the HBS-T to be introduced into the chip were set at 0, 19, 38, 75, 150, 300, and 600 nmol/L. Further, as Comparative Example, the molecular interaction analysis was performed in the same manner as described above except that the N40 was used in place of the RNA aptamer. Then, on the basis of the results thereof, with respect to the RNA aptamer shot 47, the binding rate constant (Kₐ), the dissociation rate constant (K_{d}), and the dissociation constant (K_{D} = K_{d}/Kₐ) were obtained. The results are shown in FIG. 2. FIG. 2 is a sensorgram of the signals detected using Biacore. In FIG. 2, the vertical axis and the horizontal axis are the same as those in FIG. 1.

As shown in FIG. 2, according to the molecular interaction analysis, it was found that the binding rate constant (Kₐ) of the RNA aptamer shot 47 was 2.02 × 10⁴ mol/L⁻¹s⁻¹, the dissociation rate constant (K_{d}) of the RNA aptamer shot 47 was 7.64 × 10⁻⁸s⁻¹, and the dissociation constant (K_{D}) of the RNA aptamer shot 47 was 3.78 × 10⁻¹² mol/L. With respect to the binding force of the RNA aptamer shot 47, since the dissociation constant of the antibody to a commercially available His tag is 1 × 10⁻⁹ mol/L (QIAexpress Detection and Assay Handbook, QIAGEN GmbH, Hilden, Germany, Oct, 2002, p.15.), it was found that the RNA aptamer shot 47 is an aptamer excellent in the binding force.

### (1-3) Binding affinity of miniaturized RNA aptamers to His-MIF

By the molecular interaction analysis, the binding affinity of each of the miniaturized RNA aptamers to the fusion protein His-MIF was analyzed. As the miniaturized RNA aptamers, the miniaturized RNA aptamer #47s (SEQ ID NO: 12), the miniaturized RNA aptamer #47sT (SEQ ID NO: 13), the miniaturized RNA aptamer shot 47sss (SEQ ID NO: 14), and the miniaturized RNA aptamer #47sssT (SEQ ID NO: 16), which are obtained by miniaturizing the RNA aptamer shot 47 (SEQ ID NO: 2), were used. The results are shown in FIG. 5. FIG. 5 is a sensorgram of the signals detected using Biacore. In FIG. 5, the vertical axis and the horizontal axis are the same as those in FIG. 1. FIG. 5 also shows the result obtained by using the RNA aptamer shot 47, which is not miniaturized.

As shown in FIG. 5, all of the miniaturized RNA aptamers showed the binding affinity to the His-MIF. Among them; the miniaturized RNA aptamer #47s and the miniaturized RNA aptamer shot 47sss, which are miniaturized aptamers designed so as not to break the stem-loop structure of the RNA aptamershot 47 shown in FIG. 4, showed the equivalent effect to the RNA aptamer shot 47. From this, it can be predicted that the stem-loop structure is an important structure for aptamers. Further, the miniaturized RNA aptamer #47s and the miniaturized RNA aptamer shot 47sss each showed a binding affinity that is superior to other miniaturized RNA aptamers. As shown in Table 7, the other miniaturized RNA aptamers, the miniaturized RNA aptamer #47sT (SEQ ID NO: 13) and the miniaturized RNA aptamer #47sssT (SEQ ID NO: 16), are aptamers in each of which any of the 7^{th} U, the 11^{th} U, and the 15^{th} A in the sequence of SEQ ID NO: 18 enclosed in the box in the base sequence of the #47s is deleted or substituted. Therefore, in SEQ ID NO: 18, it can be predicted that it is important to maintain the 7^{th} U and the 11^{th} U in the loop structure and, as shown in FIG. 4, the 15^{th} A at the site in which the stem is bent.

### (2) ELISA improvement method

### (2-1) Binding site of shot 47 in His-GFP

It was checked by the aforementioned ELISA improvement method to which site of the fusion protein the RNA aptamer shot 47 is bound. As fusion proteins, among the fusion proteins shown in FIG. 6, five fusion proteins, namely HTX, HT, H, TX (not part of the invention), and T (not part of the invention) each containing

GFP were used. Further, an anti-GFP antibody was immobilized on the plate in the ELISA improvement method. Furthermore, as Comparative Example, the binding affinity was confirmed in the same manner as described above except that the N40 was used instead of the RNA aptamer shot 47.

The results arc shown in FIG. 7. FIG. 7 is a graph showing the binding affinity of the RNA aptamer shot 47 to each of the respective fusion proteins. In FIG. 7, the vertical axis indicates the absorbance at 450 nm that shows the binding affinity (binding of RNA aptamer) and indicates the average value ± deviation (SD) based on three-time measurements. The horizontal axis indicates the type of the fusion protein. The white bars indicate the results obtained using the N40 and the black bars indicate the results obtained using the RNA aptamer shot 47. Further, the photograph on the upper right in FIG. 7 shows the results of the western blotting with respect to the fusion proteins used. It has been already confirmed that the respective proteins were obtained by preparation from the transformants described above.

As shown in the graph of FIG. 7, the RNA aptamer shot 47 showed the binding affinity to the fusion proteins (HTX, HT, and H) each including a His tag but did not bind to the fusion proteins (TX and T) each not including a His tag (not part of the invention). Since all of the fusion proteins include GFP, it became evident that the RNA aptamer shot 47 does not bind to GFP. Among the fusion proteins, since HT is the fusion protein in which Xpress (Xpress^{™}Epitope) is deleted and H is the fusion protein in which T (T7 gene 10 leader) is deleted, it became evident that the RNA aptamer shot 47 binds to the His tag. Further, since the binding affinity of the RNA aptamer shot 47 to each of the respective fusion proteins showed the following relationship: HTX ≈ HT > H, it was found that the binding affinity increases if the aptamer further includes a tag such as Xpress (Xpress^{™}Epitope) or T (T7 gene 10 leader) in addition to the His tag.

### (2-2) Binding affinity of shot 47 to His-MIF

The binding affinity of the RNA aptamer shot 47 to the fusion protein His-MIF and to MIF that does not include a His tag (not part of the invention) was confirmed by the aforementioned ELISA improvement method. An anti-MIF antibody and an anti-His-tag antibody were immobilized to the plates in the ELISA improvement method. As Comparative Example, the binding affinity was confirmed in the same manner as described above except that the N40 was used in place of the RNA aptamer shot 47. As a control, for confirming the binding of the His-MIF to the plate, the absorbance at 450 nm was measured by adding an HRP-labeled anti-MIF polyclonal antibody (anti-MIFpAb) in place of the RNA aptamer and also adding a substrate.

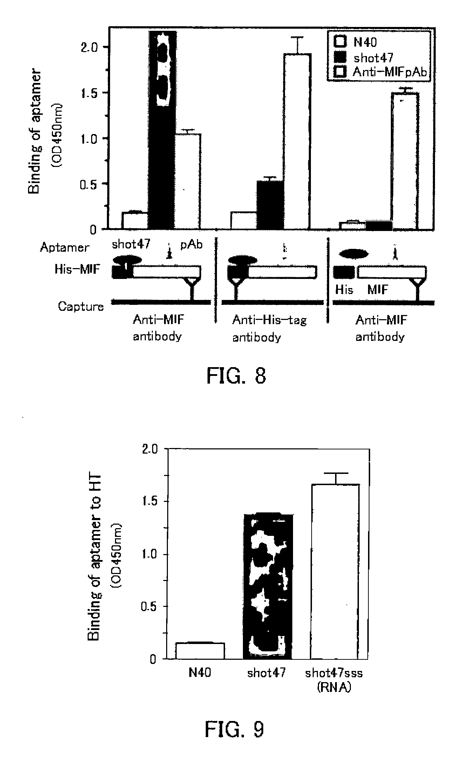

The results are shown in FIG. 8. FIG. 8 is a graph showing the binding affinity of the RNA aptamer shot 47 to the fusion protein His-MIF. In FIG. 8, the vertical axis indicates the absorbance at 450 nm that shows the binding affinity of the RNA aptamer and indicates the average value ± deviation (SD) based on three-time measurements. In FIG. 8, the white bars indicate the results obtained using the N40, the black bars indicate the results obtained using the RNA aptamer shot 47, and the gray bars indicate the results obtained using the HRP-labeled anti-MIF polyclonal antibody. The bars on the left side indicate the result of His-MIF in the plate on which the anti-MIF antibody was immobilized, the bars in the middle indicate the result of His-MIF in the plate on which the anti-His-tag antibody was immobilized, and the bars at the right side indicate the result of MIF in the plate on which the anti-MIF antibody was immobilized. Further, in FIG. 8, a schematic view of binding modes is shown under the graph.

As shown in FIG. 8, as a result of binding the immobilized anti-MIF antibody and MIF that does not include a His tag (not part of the invention), MIF that does not include a His tag was detected in the anti-MIF polyclonal antibody. From this, it could be confirmed that MIF was bound to the immobilized MIF antibody but the binding of the RNA aptamer shot 47 was not confirmed. On the other hand, as a result of binding the immobilized anti-MIF antibody and the fusion protein His-MIF, the binding of the RNA aptamer shot 47 was confirmed. From this result, it was found that the RNA aptamer shot 47 recognizes a His tag and therefore the fusion protein can be detected by the RNA aptamer shot 47 if it includes a His tag. Further, as a result of binding the immobilized anti-His-tag antibody and the fusion protein His-MIF, as compared to the case in which the immobilized anti-MIF antibody was used, the binding affinity of the RNA aptamer shot 47 was decreased. This is considered to be due to as follows. That is, since the immobilized anti-His-tag antibody was bound to the His tag, which is the target to be bound with the shot 47, it was hard for the RNA aptamer shot 47 to bind thereto.

### (2-3) Binding affinity of shot 47 and shot 47sss to HT

With respect to the RNA aptamer shot 47 and the miniaturized RNA aptamer shot 47sss, the binding affinity to the fusion protein HT consisting of His (His tag), T (T7 gene 10 leader), and GFP was confirmed by the aforementioned ELISA improvement method. An anti-GFP antibody was immobilized to the plate in the ELISA improvement method. Further, as Comparative Example, the binding affinity was confirmed in the same manner as described above except that the N40 was used in place of the shot 47.

The results are shown in FIG. 9. FIG. 9 is a graph showing the binding affinity of the RNA aptamer shot 47 and the miniaturized RNA aptamer shot 47sss to the fusion protein HT. In FIG. 9, the vertical axis indicates the absorbance at 450 nm that shows the binding affinity (binding of RNA aptamer) and indicates the average value ± deviation (SD) based on three-time measurements. The horizontal axis indicates the type of the RNA aptamer. The white bar indicates the result obtained using the N40, the black bar indicates the result obtained using the shot 47, and the gray bar indicates the result obtained using the shot 47sss.

As shown in the graph of FIG. 9, both of the RNA aptamer shot 47 and the miniaturized RNA aptamer shot 47sss showed the binding affinity to the fusion protein HT including a His tag.

### (3) Pull-down assay

### (3-1) Binding affinity of shot 47 to fusion protein

With respect to the RNA aptamer shot 47, the binding affinity to each of the fusion proteins His-MIF and His-GFP are confirmed by the aforementioned pull-down assay and northwestern blotting. Further, as Comparative Example, the binding affinity was confirmed in the same manner as described above except that the N40 was used in place of the shot 47.

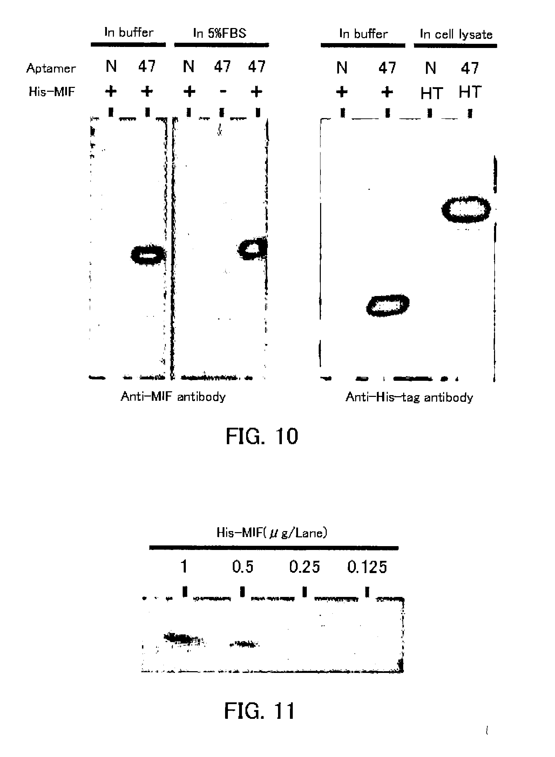

The result of the pull-down assay is shown in FIG. 10. FIG. 10 is a photograph of the result of the pull-down assay showing the binding between the RNA aptamer shot 47 and the fusion proteins His-MIF and HT. In FIG. 10, "In buffer" indicates the result obtained using the His-MIF-containing HBS-T, "In 5% FBS" indicates the result obtained using the His-MIF-containing culture supernatant, and "In cell lysate" indicates the result obtained using the extract of *Escherichia coli* in which HT is expressed. Further, in the "Aptamer" column of FIG. 10, "N" indicates the result using the N40 and "47" indicates the result using the RNA aptamer shot 47. In the "His-MIF" column, "+" indicates that the fusion protein in the sample is His-MIF, "HT" indicates that the fusion protein in the sample is HT, and "-" indicates that the sample contains neither His-MIF nor HT. As shown in FIG. 10, the fusion proteins His-MIF and HT could be pulled down by using the RNA aptamer shot 47. Further, as can be seen from the result of "In cell lysate" of FIG. 10, the fusion protein HT could be pulled down even from the homogenate of *Escherichia coli* by the RNA aptamer shot 47.

The result of the northwestern blotting is shown in FIG. 11. In FIG. 11, the numerical value in each lane indicates the concentration of His-MIF per lane (µg/lane). As shown in FIG. 11, also with respect to the fusion protein subjected to blotting, the detection could be performed by the northwestern blotting using the RNA aptamer shot 47.

### [Example 2]

An HTMCSshot/pCold vector or an HTBSNshot/pCold v3 vector was constructed as a nucleic acid construct, and arbitrary encoding DNA including random DNA was inserted thereto to produce a plasmid library.

### (HTMCSshot/pCold vector)

Double-stranded DNA consisting of His tag-encoding DNA, T-tag-encoding DNA, a stuffer sequence, and aptamer-encoding DNA was inserted into the NdeI-XbaI site of pCold (registered trademark) IV (TAKARA BIO INC.) to produce an HTMCSshot/pCold vector. The sequence of the sense strand of the double-stranded DNA is shown in SEQ ID NO: 57 in the following Table 9. In the following sequence, the His tag-encoding DNA and the T7 gene leader are derived from pRSET (Invitrogen Corporation). In the following sequence, the stuffer sequence is derived from the multicloning site of pFLAG-CMV1 (Sigma Chemical Co.), the arrow at the 5' side indicates the cleavage site of BamHI and the arrow at the 3' side indicates the cleavage site of NotI. The aptamer-encoding DNA in the following sequence has a sequence that includes the DNA sequence corresponding to the miniaturized RNA aptamer shot 47sss (SEQ ID NO: 102: gguauauuggcgccuucguggaaug) having a length of 25 bases shown in Table 1.

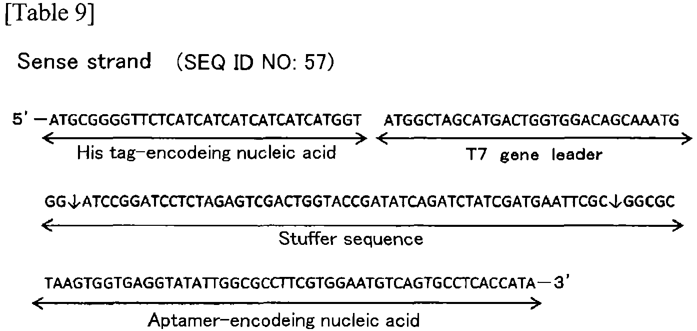

### (HTBSNshot/pcold v3 vector)

His tag-encoding DNA, T-tag-encoding DNA, a stuffer sequence, and aptamer-encoding DNA were inserted into the NdeI-transcription termination site of pCold (registered trademark) IV (TAKARA BIO INC.) to produce an HTBSNshot/pCold v3 vector. The aptamer-encoding DNA was inserted into the inside of the transcription termination site sequence of pCold IV.

The sequence adjacent to 3'UTR of pCold IV is shown in the following SEQ ID NO: 58. In the sequence of SEQ ID NO: 58, the underlined part at the 5' side refers to a stop codon and the underlined part at the 3' side refers to a transcription termination site sequence (transcription terminator). The lower-case "tt" site was substituted with the aptamer-encoding DNA.

partial sequence of pCold IV (SEQ ID NO: 58)

5'-CATATGCGGGGTTCTCATCATCATCATCATCATGGTATGGCTAGCATGACTGGTG
GACAGCAAATGGGATCCCCCGGGAGCGGCCGCTAATCTAGATAGGTAATCTCTGCT
TAAAAGCACAGAATCTAAGATCCCTGCCAttTGGCGGGGATT-3'

The partial sequence of the sense strand of the pCold IV after insertion of the respective sequences is shown in the following SEQ ID NO: 59. In the following sequence, the His tag-encoding DNA and the T7 gene leader are derived from pRSET (Invitrogen Corporation). In the following sequence, the arrow at the 5' side of the stuffer sequence indicates the cleavage site of BamHI, the arrow in the middle indicates the cleavage site of SmaI, and the arrow at the 3' side indicates the cleavage site of NotI. Further, in the following sequence, the underlined part of the pCold IV-derived sequence refers to a transcription termination site sequence. The aptamer-encoding DNA in the following sequence has a sequence that includes the DNA sequence corresponding to the miniaturized RNA aptamer shot 47sss (SEQ ID NO: 102: gguauauuggcgccuucguggaaug) having a length of 25 bases shown in Table 1.

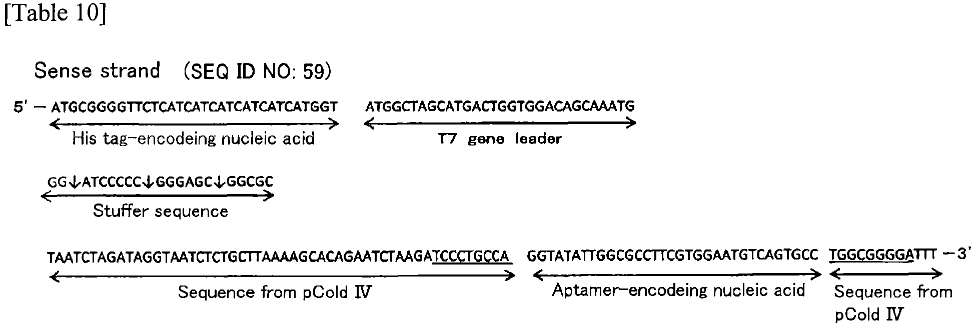

### (Plasmid library 1)

Polynucleotide (Lib60F) that includes a random sequence N₆₀ having a length of 60 bases and polynucleotide (Lib60R) that is capable of annealing to the 3' region of the Lib60F were synthesized. The sequence of the Lib60F is shown in the following SEQ ID NO: 60 and the sequence of Lib60R is shown in the following SEQ ID NO: 61. In the following sequences, the underlined parts respectively refer to the complementary sequences in the respective polynucleotides.

Lib60F (SEQ ID NO: 60)
   CACGGATCC(N₆₀)GGTGGAGGCGGGTCTGGGGGCGGAGGTTCAG
Lib60R (SEQ ID NO: 61)
   CGTCTAGCGGCCGCCTGAACCTCCGCCCCCAGA

Further, 1 µL of 100 µmol/L Lib60F and 10 µL of 100 µmol/L Lib60R were mixed and an elongation reaction was performed with a reaction solution of 100 µL in total using HotStarTaq (Qiagen). The conditions for the elongation reaction were as follows. That is, after treating at 95°C for 15 minutes, one cycle of treatment at 60°C for 3 minutes and 72°C for 3 minutes was repeated for 5 cycles, and finally the reaction was performed at 72°C for 10 minutes. An amplification product was purified from the reaction solution by ethanol precipitation, digested with BamHI and NotI, and then purified by phenol chloroform extraction and ethanol precipitation. The resultant was used as double-stranded arbitrary encoding DNA that includes a random sequence N₆₀ having a length of 60 bases. Further, the arbitrary encoding DNA was allowed to bind to the HTMCSshot/pCold vector using T4 DNA ligase. As the HTMCSshot/pCold vector, the one preliminarily digested with BamHI and NotI and then purified by agarose gel electrophoresis and gel extraction was used. The resultant library was used as a plasmid library 1.

### (Plasmid library 2)

Polynucleotide (Lib787R, Lib747R, or Lib727R) that includes random sequences (MNN)₇ each having a length of 21 bases at two sites or polynucleotide (Lib707R) that includes a random sequence (MNN)₁₄ having a length of 42 bases at one site was synthesized with polynucleotide (Lib707F) that is capable of annealing to the 3' region of each of the polynucleotides. The sequences thereof are respectively shown in the following SEQ ID NOs: 62 to 64, 81, and 82. In the following sequences, the underlined parts respectively refer to the complementary sequences in the respective polynucleotides.

Lib707F (SEQ ID NO: 62)
   CAAATGGGATCCGAATCTGGT
Lib787R (SEQ ID NO: 63)
   CTTAGCGGCCGCT(MNN)₇AGAAGCTTTACCGTTAATGCTACC(MNN)₇ACCAGATTCG GATCCCATTTG
   (M = A or C)
Lib747R (SEQ ID NO: 64)
   CTTAGCGGCCGCT(MNN)₇TTTACCGTTAAT(MNN)₇ACCAGATTCGGATCCCATTTG
   (M = A or C)
Lib727R (SEQ ID NO: 81)
   CTTAGCGGCCGCT(MNN)₇ACCCGG(MNN)₇ACCAGATTCGGATCCCATTTG
   (M = A or C)
Lib707R (SEQ ID NO: 82)
   CTTAGCGGCCGCT(MNN)₁₄ACCAGATTCGGATCCCATTTG
   (M = A or C)

Further, PCR and purification of an amplification product were performed in the same manner as the plasmid library 1 except that 2.5 µL of 100 µmol/L Lib707F and 1 µL of 100 µmol/L Lib787R, 1 µL of 100 µmol/L Lib747R, 1 µL of 100 µmol/L Lib727R, or 1 µL of 100 µmol/L Lib707R were used. After digesting the amplification product with BamHI and NotI, purification was performed by phenol chloroform extraction and an ultrafilter (product name: Amicon YM-30). The resultant was used as double-stranded arbitrary encoding DNA that includes a random sequence (MNN)₇ having a length of 21 bases in the antisense strand. Note here that the double-stranded arbitrary encoding DNA includes, in the sense strand, random sequences (NNK)₇ each having a length of 21 bases at two sites or a random sequence (NNK)₁₄ having a length of 42 bases at one site (K = G, T, or U). Further, the arbitrary encoding DNA was allowed to bind to the HTBSNshot/pCold v3 vector using T4 DNA ligase. As the HTBSNshot/pCold v3 vector, the one preliminarily digested with BamHI, NotI, and SmaI and then purified by phenol chloroform extraction and an ultrafilter (product name Amicon YM-50) was used. The respective plasmids were mixed in equal amount and the resultant was used as a plasmid library 2.

### (Plasmid library3)

Polynucleotide (sFBG762R) that includes a random sequence (MNN)₂ having a length of 6 bases, a random sequence (MNN)₆ having a length of 18 bases, and a random sequence (MNN)₇ having a length of 21 bases at three sites from the 5' side was synthesized with polynucleotide (sFBG762F) that is capable of annealing to the 3' region of the sFBG762R. The sequence of the sFBG762F is shown in the following SEQ ID NO: 83 and the sequence of sFBG762R is shown in the following SEQ ID NO: 84. In the following sequences, the underlined parts respectively refer to the complementary sequences in the respective polynucleotides.

sFBG762F (SEQ ID NO: 83)
   CAAATGGGATCCGAAATCAAA
sFBG762R (SEQ ID NO: 84)
   TTAGCGGCCGCTCTGATA(MNN)₂GCC(MNN)₆ATACAGGCCATC(MNN)₇TTTGATTTC GGATCCCATTTG
   (M = A or C)

Further, PCR and the restriction enzyme treatment and purification of the amplification product were performed in the same manner as the plasmid library 1 except that 2.5 µL of 100 µmol/L sFBG762F and 1 µL of 100 µmol/L sFBG762R were used. The resultant was used as double-stranded arbitrary encoding DNA that includes random sequences (MNN)₂, (MNN)₆, and (MNN)₇ at three sites in the antisense strand. Note here that the double-stranded arbitrary encoding DNA includes, in the sense strand, a random sequence (NNK)₇ having a length of 21 bases, a random sequence (NNK)₆ having a length of 18 bases, and a random sequence (NNK)₂ having a length of 6 bases at three sites from the 5' side (K = G, T or U). Further, the arbitrary encoding DNA was allowed to bind to the HTBSNshot/pCold v3 vector in the same manner as the plasmid library 2. The resultant was used as a plasmid library 3.

### [Example 3]

Each of the plasmid libraries obtained in Example 2 was transfected into an *Escherichia coli* DH5α line. This *Escherichia coli* was subjected to shaking culture in 100 mL of LB culture medium containing 100 µg/mL ampicillin until the absorbance at OD 600 nm becomes 0.5 to 0.6. Thereafter, IPTG was added to the culture solution such that the concentration becomes 0.5 mmol/L and culture was conducted at 15°C for 18 hours to conduct induction of cold shock expression.

The culture solution was subjected to centrifugation to recover *Escherichia coli,* the recovered *Escherichia coli* was suspended in 50 mL of physiological saline containing 10 mmol/L EDTA, and the resultant was again subjected to centrifugation to harvest *Escherichia coli.* The *Escherichia coli* was suspended in 5 mL of 20 mmol/L HEPES buffer solution containing 20% sucrose and 1 mmol/L EDTA, 10 mg of lysozyme was added thereto, and the resultant was cultured on ice for 1 hour. Further, Mg²⁺ was added thereto such that the final concentration becomes 2 mmol/L, precipitates were recovered by centrifugation and the recovered precipitates were suspended in 50 mL of physiological saline containing 0.1 mmol/L magnesium acetate, and the resultant was again subjected to centrifugation to recover spheroplast.

The recovered spheroplast was promptly suspended in 4 mL of 20 mmol/L HEPES buffer solution containing 0.1% Triton (registered trademark)-X100, 0.1 mmol/L Mg (OAc)₂, 0.1 mg/mL tRNA, 0.1 % HSA (RNase free), and a protease inhibitor (product name complete mini EDTA free, Roche Ltd). After causing lysis, *Escherichia coli* genomic DNA was shredded by mechanical shearing. Then, NaCl was added thereto such that the final concentration becomes 150 mmol/L, the resultant was allowed to stand for 5 minutes, and the supernatant was recovered by centrifugation. These operations were conducted at 4°C.

The obtained lysate was added to the solid phase (96-hole microtiter plate) on which rabbit IgG or mouse IgG was immobilized as a target and the resultant was incubated at 4°C for 30 minutes to bind the complex contained in the lysate to the target. As the solid phase, the one preliminarily applied with the blocking with HSA added to the lysate was used.

Subsequently, the solid phase was washed with a washing liquid to remove the complex that is not bound to the target. As the washing liquid, 20 mmol/L HEPES buffer solution containing 0.05 to 0.5% Triton (registered trademark)-X100, 0.1 mmol/L magnesium acetate, and 100 to 150 mmol/L NaCl was used. Then, eluate was added to the solid phase of after washing, RNA and peptide of the complex that is bound to the target were separated from the solid phase and recovered. As the eluate, an RNA separation reagent (product name: Trizol (registered trademark), invitrogen) was used.

Next, RNA was purified from the eluate, the obtained RNA was treated with RNase free DNase I (Promega) at 37°C for 30 minutes, and phenol chloroform extraction and ethanol precipitation were performed to conduct purification. With respect to purified RNA, cDNA was synthesized by RT-PCR using OneStep RT-PCR Kit (product name, QIAGEN). The annealing conditions of the primer in the PCR were as follows. That is, the temperature was 63.2°C and the number of cycles of PCR was 26 or 34. Further, as primers, the following forward primer shotPCR F2 and the following reverse primer shotPCR R2 were used.

shotPCR F2 (SEQ ID NO: 85)
   GGCTAGCATGACTGGTGGACAGCAAA
shotPCR R2 (SEQ ID NO: 86)
   CTGACATTCCACGAAGGCGCCAATA

Further, with respect to the respective RT-PCR reaction solutions of 34 PCR cycles, the reaction for forming a complementary double strand was performed. First, the forward primer and the reverse primer were added to each of the RT-PCR reaction solutions such that the final concentration becomes 10 µmol/L. Then, after performing thermal denaturation at 95°C for 5 minutes, one cycle of an annealing reaction at 63.2°C for 3 minutes and an elongation reaction at 72°C for 3 minutes was repeated for 5 cycles, and finally the reaction was performed at 72°C for 7 minutes. Thereby, with respect to the cDNA synthesized by RT-PCR, the complementary double strand was formed.

Subsequently, from the RT-PCR reaction solution applied only with the RT-PCR and the RT-PCR reaction solution applied with the reaction for forming a complementary double strand, amplification products were respectively recovered by ethanol precipitation and the recovered amplification products were subjected to electrophoresis.

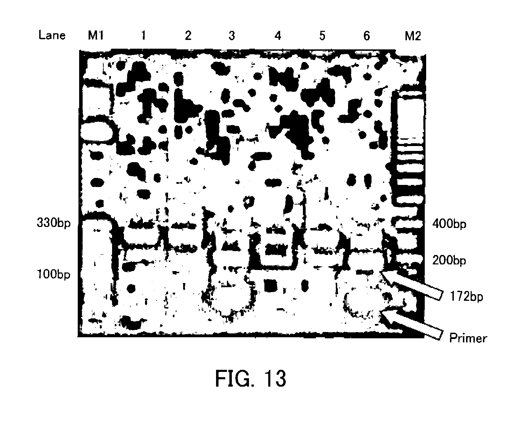

The results of the electrophoresis are shown in FIG. 13. In FIG. 13, each lane indicates as follows.

### M1: molecular weight marker (100bp ladder marker)

Lane 1: mouse IgG
   RT-PCR reaction solution of 26 cycles
Lane 2: mouse IgG
   RT-PCR reaction solution of 34 cycles
Lane 3: mouse IgG
   Reaction solution applied with reaction for forming complementary double strand after RT-PCR reaction of 34 cycles
Lane 4: rabbit IgG
   RT-PCR reaction solution of 26 cycles
Lane 5: rabbit IgG
   RT-PCR reaction solution of 34 cycles
Lane 6: rabbit IgG
   Reaction solution applied with reaction for forming complementary double strand after RT-PCR reaction of 34 cycles

### M2: molecular weight marker (200bp ladder marker)

As shown in FIG. 13, as a result of subjecting the RT-PCR reaction solution to electrophoresis, the electrophoresis band was shifted to a high molecular side relative to the actual correct molecular size (172bp). However, by applying the reaction for forming a complementary double strand to the RT-PCR reaction solution, as shown in the lanes 3 and 6, the band was observed at 172bp. This shows that the aforementioned heteroduplex cDNA is decreased and the complementary double-stranded cDNA is obtained.

### [Example 4]

The amplification product prepared in Example 3 was digested with BamHI and NotI, and then purified by phenol chloroform extraction and ethanol precipitation. The resultant was inserted into the HTMCSshot/pCold vector in the same manner as in Example 3. Thereafter, in the same manner as in Example 3, culture of *Escherichia coli* and induction of cold shock expression were conducted, lysis of *Escherichia coli* was caused, and a lysate was again obtained. The first lysate obtained in Example 3 was used as a lysate of round 1 in which selection was not performed; and the second lysate obtained in Example 4 was used as a lysate of round 2 in which selection was performed.

Rabbit IgG (Target 01) or mouse IgG (Target 02) as a target was immobilized to a 96-hole microtiter plate by adsorption, and the blocking was applied thereto with BSA. Then, the round 1 lysate and the round 2 lysate were added to each of the wells and reacted at room temperature for 2 hours. Next, the plate was washed with 20 mmol/L Tris buffer solution (pH7.5) (hereinafter, referred to as "TBST") containing 0.1% Tween (registered trademark) 20 and 0.9% NaCl, an HRP-labeled-anti-His-tag antibody (Quagen) diluted with TBST containing 0.2% BSA was added thereto, and the resultant was reacted at room temperature for 2 hours. After the reaction, the plate was washed with the TBST, a 1-step Ultra TMB substrate (Thermo Fisher Scientific Inc., USA) was added to develop colors, and the absorbance at 450 nm was measured. Further, as a blank, the reaction and absorbance measurement were performed using the plate on which only BSA was immobilized and each lysate in the same manner as described above.

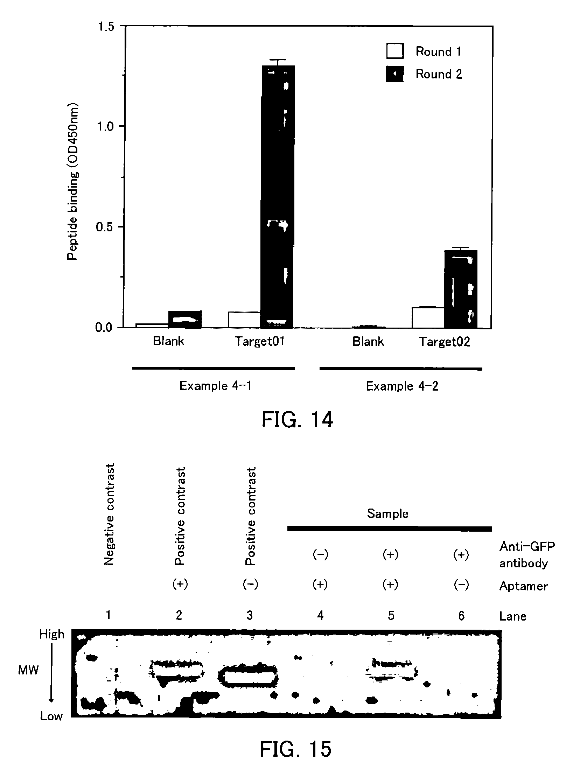

The result obtained using Target 01 as Example 4-1 and the result obtained using Target 02 as Example 4-2 are shown in FIG. 14. FIG. 14 is a graph showing the binding affinity of the lysates to the targets. In FIG. 14, the vertical axis indicates the absorbance at 450 nm that shows the binding affinity of peptide to the target, and the value is increased as the amount of the peptide that is bound to the target is increased. In FIG. 14, the white bars indicate the result obtained using the round 1 lysate and the black bars indicate the result obtained using the round 2 lysate.

As shown in FIG. 14, the both cases of using rabbit IgG and mouse IgG as targets showed the absorbance higher than the blank, and it was found that each of the round 1 lysate and the round 2 lysate contains peptide that binds to each of the targets.

Further, the round 2 lysate showed a higher absorbance than the round 1 lysate, and it was found that the amount of the peptide that binds to each of the targets contained in the round 2 lysate is greater than that in the round 1 lysate. This shows that the amount of the peptide that specifically binds to the target is increased in the lysate by selection. It means that the clone of the peptide that specifically binds to the target is concentrated.

### [Example 5]

The plasmid (HTGFP/pCold) that expresses the fusion protein HT produced in "1.(3)" of Example 1 was provided. As described above, this plasmid is the plasmid into which the encoding DNA of a His tag, the encoding DNA of T7 gene 10 leader, and the encoding DNA of GFP are inserted and does not contain the encoding DNA of an aptamer. Further, the plasmid (HTGFPshot/pCold) into which the encoding DNA of a His tag, the encoding DNA of T7 gene 10 leader, the encoding DNA of GFP, and the encoding DNA of an aptamer are inserted at the NdeI-XbaI site of pCold IV in this order was produced. Then, these plasmids each were transfected into an *Escherichia coli* DH5α line, and culture of *Escherichia coli* and induction of cold shock expression were performed and lysis of *Escherichia coli* was caused in the same manner as in Example 3 to obtain lysates.

An anti-GFP antibody as a target was immobilized to a solid phase (96-hole microtiter plate) by adsorption, and the blocking was applied thereto with HSA. Then, the round 1 lysate and the round 2 lysate each were added to a well and incubated at 4°C for 30 minutes to bind the complex contained in the lysate to the target. Thereafter, in the same manner as in Example 3, washing, elution, RNA purification, and RT-PCR were performed to synthesize cDNA. The annealing conditions of the primer in the PCR were as follows. That is, the temperature was 60°C and the number of cycles of PCR was 30. Further, as primers, the following forward primer pCold F2 and the following reverse primer pCold R were used.

pCold F2 (SEQ ID NO: 87)
   GTAAGGCAAGTCCCTTCAAGAG
pCold R (SEQ ID NO: 88)
   GGCAGGGATCTTAGATTCTG

Then, the reaction solution of the RT-PCR as a sample was subjected to electrophoresis. As a negative contrast, the reaction solution with which the RT-PCR was performed in the same manner as described above except that RNA was not added as a sample was subjected to electrophoresis. Further, as a positive contrast, RNA was purified from the lysate before it was brought into contact with the anti-GFP antibody, DNA was synthesized by RT-PCR in the same manner as described above using the purified RNA as a template, and the reaction solution of the RT-PCR was subjected to electrophoresis.

The results are shown in FIG. 15. FIG. 15 is an electrophoresis photograph of the reaction solution of the RT-PCR that shows the binding between the mRNA-GFP complex contained in the lysate and the anti-GFP antibody. In FIG. 15, the "Negative contrast" shows the result obtained using the reaction solution with which RT-PCR was performed without adding RNA and each "Positive contrast" shows the result obtained using the reaction solution with which RT-PCR was performed using RNA contained in each lysate as a template. Among the samples, the "anti-GFP antibody (-)" shows the result obtained using a solid phase on which HSA was immobilized in place of the anti-GFP antibody and each "anti-GFP antibody (+)" shows the result obtained using a solid phase on which the anti-GFP antibody was immobilized. Each "Aptamer (+)" shows the result of the case in which mRNA to which an aptamer was added was expressed using HTGFPshot/pCold and the "Aptamer (-)" shows the result of the case in which mRNA to which an aptamer was not added was expressed using HTGFP/pCold.

As shown in the lane 4 of FIG. 15, in the case where mRNA to which an aptamer was added was expressed, few mRNA-GFP complexes that were non-specifically adsorbed were recovered from the solid phase on which HSA was immobilized. Further, as shown in the lane 6, in the case where mRNA to which an aptamer was not added was expressed, the mRNA-GFP complex was not formed, and few mRNAs that were non-specifically adsorbed were recovered even from the solid phase on which an anti-GFP antibody was immobilized. In contrast, as shown in the lane 5, in the case where mRNA to which an aptamer was added was expressed and a solid phase on which an anti-GFP antibody was immobilized was used, a large number of mRNA-GFP complexes that are specifically adsorbed to an anti-GFP antibody could be recovered. This shows that mRNA containing the transcript of the encoding nucleic acid of an aptamer and His tag-GFP translated from the mRNA formed a complex, the complex was bonded to the anti-GFP antibody immobilized on the solid phase, and RNA of the encoding nucleic acid of peptide that was bound to the anti-GFP antibody was specifically recovered. Therefore, it is obvious that, by analyzing the sequence of the recovered RNA or the sequence of DNA corresponding thereto by a conventionally known technique such as sequencing, the sequence of the encoding nucleic acid of the candidate peptide that binds to the anti-GFP antibody and the amino acid sequence of the candidate peptide can be analyzed.

As described above, according to the present invention, by utilizing the binding between the peptide tag and the aptamer corresponding thereto, the information on the encoding nucleic acid of the candidate peptide that binds to the target can be easily analyzed, and the amino acid sequence of the candidate peptide can be determined based on the analysis result. Therefore, according to the present invention, for example, the selection of the candidate peptide can be performed without causing enormous time and efforts unlike the obtainment of the antibody by immunization or the like.

The invention of the present application was described above with reference to the embodiments. However, the invention of the present application is not limited to the above-described embodiments. Various changes that can be understood by those skilled in the art can be made in the configurations and details of the invention of the present application within the scope of the invention of the present application.

### Industrial Applicability

According to the nucleic acid construct of the present invention, the complex of the fusion transcript and the fusion translation can be formed by utlizing the binding between the transcribed aptamer and the translated peptide tag. In the complex, the fusion translation includes arbitrary peptide and the fusion transcript includes the transcript of the arbitrary peptide. Therefore, in the case where the complex binds to a target, for example, by the identification of the transcript in the complex, the arbitrary peptide that is bindable to the target can be identified. In this manner, according to the present invention, simply by inserting the encoding nucleic acid of the arbitrary peptide into the nucleic acid construct of the present invention to form the complex and recovering the complex that is bound to the target, the peptide that is bindable to the target and its encoding nucleic acid can be identified easily. Accordingly, the present invention provides a very useful tool and method for screening a binding molecule to a target, for example, in medical fields.

## Claims

1. A nucleic acid construct, comprising:
a cloning region for being inserted with an encoding nucleic acid of arbitrary peptide;
an encoding nucleic acid of a peptide tag, wherein the peptide tag is a histidine tag; and
an encoding nucleic acid of an aptamer that is bindable to the peptide tag;
wherein the nucleic acid construct is for forming a complex of a fusion transcript having a base sequence that includes the encoding nucleic acid of arbitrary peptide, the encoding nucleic acid of a peptide tag, and the encoding nucleic acid of the aptamer and a fusion translation that includes the arbitrary peptide and the peptide tag.

2. The nucleic acid construct according to claim 1, wherein the nucleic acid construct is capable of:
transcribing the fusion transcript having a base sequence that includes the encoding nucleic acid of arbitrary peptide to be inserted into the cloning region,
the encoding nucleic acid of a peptide tag, and the encoding nucleic acid of the aptamer; and
translating the fusion translation that includes the arbitrary peptide and the peptide tag.

3. The nucleic acid construct according to claim 1 or 2, wherein the nucleic acid construct is a vector.

4. The nucleic acid construct according to claim 3, wherein the vector is a cold shock expression vector.

5. The nucleic acid construct according to any one of claims 1 to 3, wherein each of the encoding nucleic acid of a peptide tag and the encoding nucleic acid of
the aptamer is DNA.

6. The nucleic acid construct according to any one of claims 1 to 5, wherein the aptamer is any of the following nucleic acids (a), (b), (c), and (d):
(a) a nucleic acid that includes a base sequence represented by SEQ ID NO: 17:
GGUNnAYUmGGH (SEQ ID NO: 17),
wherein N represents A, G, C, U, or T, n of Nn represents the number of N, which is an integer of 1 to 3, Y represents U, T, or C, m of Um represents the number of U, which is an integer of 1 to 3, and H represents U, T, C, or A;
(b) a nucleic acid that includes a base sequence obtained by substitution, deletion, addition, or insertion of one or more bases in the base sequence shown in (a) and is bindable to the histidine tag;
(c) a nucleic acid that includes a base sequence represented by SEQ ID NO: 18:
GGCGCCUUCGUGGAAUGUC (SEQ ID NO: 18);
(d) a nucleic acid that includes a base sequence obtained by substitution, deletion, addition, or insertion of one or more bases in the base sequence shown in (c) and is bindable to the histidine tag.

7. The nucleic acid construct according to claim 6, wherein the nucleic acid (a) is:
a nucleic acid that includes a base sequence represented by any of SEQ ID NOs: 89 to 104;
a nucleic acid that includes a base sequence represented by any of SEQ ID NOs: 1 to 16;
a nucleic acid that includes a base sequence represented by any of SEQ ID NOs: 105 to 114, 116 to 124, and 127 to 146;
a nucleic acid that includes a base sequence represented by any of SEQ ID NOs: 26 to 35, 37 to 45, 65 to 68, 19 to 25, and 48 to 56;
a nucleic acid that includes a base sequence represented by any of SEQ ID NOs: 2, 12, 14, 15, and 55; or
a nucleic acid that includes a base sequence represented by any of SEQ ID NOs: 158 to 2302 and 2303 to 2312.

8. The nucleic acid construct according to claim 6, wherein the nucleic acid (a) is a nucleic acid that includes a base sequence represented by SEQ ID NO: 147: GGUNnAYUmGGHGCCUUCGUGGAAUGUC (SEQ ID NO: 147), wherein N represents A, G, C, U, or T, n of Nn represents the number of N, which is an integer of 1 to 3, Y represents U, T, or C, m of Um represents the number of U, which is an integer of 1 to 3, and H represents U, T, C, or A.

9. The nucleic acid construct according to claim 8, wherein the base sequence represented by SEQ ID NO: 147 is a base sequence represented by SEQ ID NO: 148:
GGUAUAUUGGCGCCUUCGUGGAAUGUC (SEQ ID NO: 148).

10. The nucleic acid construct according to claim 6, wherein the nucleic acid (c) is a nucleic acid that includes a base sequence represented by any of SEQ ID NOs: 2, 12, 14, 15, and 55.

11. The nucleic acid construct according to any one of claims 1 to 10, wherein the number of histidines in the histidine tag is 6 to 10.

12. The nucleic acid construct according to any one of claims 1 to 11, wherein the nucleic acid construct comprises the encoding nucleic acid of a peptide tag at the 5' side of the cloning region and comprises the encoding nucleic acid of the aptamer at the 3' side of the cloning region.

13. The nucleic acid construct according to any one of claims 1 to 12, further comprising an encoding nucleic acid of T7 gene leader.

14. The nucleic acid construct according to any one of claims 1 to 13, wherein the encoding nucleic acid of arbitrary peptide is inserted into the cloning region.

15. The nucleic acid construct according to any one of claims 1 to 14, wherein the nucleic acid construct is a screening nucleic acid construct for screening peptide that is bindable to a target or its encoding nucleic acid.

16. A complex formation method for forming a complex of a fusion transcript having a base sequence that includes an encoding nucleic acid of arbitrary peptide, an encoding nucleic acid of a peptide tag, and an encoding nucleic acid of an aptamer that is bindable to the peptide tag and a fusion translation that includes the arbitrary peptide and the peptide tag, comprising the step of:
expressing the nucleic acid construct according to any one of claims 1 to 15, the nucleic acid construct comprising:
a cloning region for being inserted with an encoding nucleic acid of arbitrary peptide;
an encoding nucleic acid of a peptide tag, wherein the peptide tag is a histidine tag; and
an encoding nucleic acid of an aptamer that is bindable to the peptide tag, the encoding nucleic acid of arbitrary peptide being inserted into the cloning region.

17. The complex formation method according to claim 16, wherein the complex is formed *in vivo.*

18. The complex formation method according to claim 16 or 17, wherein the nucleic acid construct is expressed *in vivo.*

19. The complex formation method according to any one of claims 16 to 18, wherein the nucleic acid construct is expressed in a living cell.

20. The complex formation method according to claim 19, wherein the living cell is *Escherichia coli.*

21. The complex formation method according to claim 16, wherein the complex is formed *in vitro.*

22. The complex formation method according to claim 16 or 21, wherein the nucleic acid construct is expressed *in vitro.*

23. The complex formation method according to any one of claims 16 to 22, wherein the nucleic acid construct is a vector.

24. A screening method for screening peptide that is bindable to a target or an encoding nucleic acid of the peptide, comprising the steps of:
(A) forming a complex by the complex formation method according to any one of claims 16 to 23;
(B) contacting the complex with the target; and
(C) recovering the complex that is bound to the target.

25. The screening method according to claim 24, wherein, in the step (B), the target is a target that is immobilized on a solid phase.

26. The screening method according to claim 24 or 25, further comprising the step of:
(D) synthesizing the encoding nucleic acid of arbitrary peptide using a transcript of the encoding nucleic acid of arbitrary peptide in the complex recovered in the step (C) as a template.

27. The screening method according to claim 26, wherein, in the step (D), the encoding nucleic acid of arbitrary peptide is synthesized by a reverse transcription-polymerase chain reaction.

28. The screening method according to claim 26 or 27, wherein the encoding nucleic acid of arbitrary peptide obtained in the step (D) is inserted into the cloning region of the nucleic acid construct according to any one of claims 1 to 15, and the steps (A), (B), and (C) are performed again.

29. The screening method according to claim 28, wherein the steps (A), (B), (C), and (D) are performed repeatedly.

30. The screening method according to any one of claims 26 to 29, wherein a base sequence of the encoding nucleic acid of arbitrary peptide obtained in the step (D) is determined.

31. The screening method according to claim 30, wherein an amino acid sequence of the arbitrary peptide that is bindable to the target is determined based on the base sequence of the encoding nucleic acid of arbitrary peptide.

32. A complex formation kit used for the complex formation method according to any one of claims 16 to 23, comprising:
the nucleic acid construct according to any one of claims 1 to -15.

33. The complex formation kit according to claim 32, further comprising a living cell to be transfected with the nucleic acid construct.

34. A screening kit used for the screening method according to any one of claims 24 to 31, comprising:
the nucleic acid construct according to any one of claims 1 to 15.

35. The screening kit according to claim 34, further comprising a living cell to be transfected with the nucleic acid construct.

## Patentansprüche

1. Nukleinsäurekonstrukt, umfassend:
eine Klonierungsregion, in die eine kodierende Nukleinsäure eines beliebigen Peptids insertiert werden kann;
eine kodierende Nukleinsäure eines Peptid-Tag, wobei das Peptid-Tag ein Histidin-Tag ist; und
eine kodierende Nukleinsäure eines Aptamers, das an das Peptid-Tag binden kann;
wobei das Nukleinsäurekonstrukt zur Bildung eines Komplexes aus einem Fusionstranskript mit einer Basensequenz, die die kodierende Nukleinsäure des beliebigen Peptids, die codierende Nukleinsäure des Peptid-Tag und die kodierende Nukleinsäure des Aptamers umfaßt, und einem Fusionstranslationsprodukt, das das willkürliche Peptid und das Peptid-Tag umfaßt, dient.

2. Nukleinsäurekonstrukt nach Anspruch 1, wobei das Nukleinsäurekonstrukt fähig ist zur:
Transkription des Fusionstranskripts mit einer Basensequenz, die die kodierende Nukleinsäure des in die Klonierungsregion zu insertierenden beliebigen Peptids, die codierende Nukleinsäure des Peptid-Tag und die kodierende Nukleinsäure des Aptamers umfaßt; und
Translation des Fusionstranslationsprodukts, das das willkürliche Peptid und das Peptid-Tag umfaßt.

3. Nukleinsäurekonstrukt nach Anspruch 1 oder 2, wobei das Nukleinsäurekonstrukt ein Vektor ist.

4. Nukleinsäurekonstrukt nach Anspruch 3, wobei der Vektor ein Kälteschock-Expressionsvektor ist.

5. Nukleinsäurekonstrukt nach einem der Ansprüche 1 bis 3, wobei jede kodierende Nukleinsäure eines Peptid-Tag und jede kodierende Nukleinsäure der Aptamers DNA ist.

6. Nukleinsäurekonstrukt nach einem der Ansprüche 1 bis 5, wobei das Aptamer eine der folgenden Nukleinsäuren (a), (b), (c) und (d) ist:
(a) eine Nukleinsäure, die eine Basensequenz, dargestellt durch SEQ ID NO: 17, umfaßt:
GGUNnAYUmGGH (SEQ ID NO: 17),
wobei N A, G, C, U oder T darstellt, n von Nn die Anzahl von N darstellt, die eine ganze Zahl von 1 bis 3 ist, Y U, T oder C darstellt, m von Um die Anzahl von U darstellt, die eine ganze Zahl von 1 bis 3 ist, und H U, T, C oder A darstellt;
(b) einer Nukleinsäure, die eine Basensequenz umfaßt, die durch Substitution, Deletion, Addition oder Insertion von einer oder mehreren Basen in der in (a) gezeigten Basensequenz erhalten ist, und an das Histidin-Tag binden kann;
(c) einer Nukleinsäure, die eine Basensequenz, dargestellt durch SEQ ID NO: 18, umfaßt:
GGCGCCUUCGUGGAAUGUC (SEQ ID NO: 18);
(d) eine Nukleinsäure, die eine Basensequenz umfaßt, die durch Substitution, Deletion, Addition oder Insertion von einer oder mehreren Basen in der in (c) gezeigten Basensequenz erhalten ist, und an das Histidin-Tag binden kann.

7. Nukleinsäurekonstrukt nach Anspruch 6, wobei die Nukleinsäure (a) ist:
eine Nukleinsäure, die eine Basensequenz, dargestellt durch eine der SEQ ID NOs: 89 bis 104, umfaßt;
eine Nukleinsäure, die eine Basensequenz, dargestellt durch eine der SEQ ID NOs: 1 bis 16, umfaßt;
eine Nukleinsäure, die eine Basensequenz, dargestellt durch eine der SEQ ID NOs: 105 bis 114, 116 bis 124 und 127 bis 146, umfaßt;
eine Nukleinsäure, die eine Basensequenz, dargestellt durch eine der SEQ ID NOs: 26 bis 35, 37 bis 45, 65 bis 68, 19 bis 25 und 48 bis 56, umfaßt;
eine Nukleinsäure, die eine Basensequenz, dargestellt durch eine der SEQ ID NOs: 2, 12, 14, 15 und 55, umfaßt; oder
eine Nukleinsäure, die eine Basensequenz, dargestellt durch eine der SEQ ID NOs: 158 bis 2302 und 2303 bis 2312, umfaßt.

8. Nukleinsäurekonstrukt nach Anspruch 6, wobei die Nukleinsäure (a) eine Nukleinsäure ist, die eine Basensequenz, dargestellt durch SEQ ID NO: 147, umfaßt:
GGUNnAYUmGGHGCCUUCGUGGAAUGUC (SEQ ID NO: 147),
wobei N A, G, C, U oder T darstellt, n von Nn die Anzahl N darstellt, die eine ganze Zahl von 1 bis 3 ist, Y U, T oder C darstellt, m von Um die Anzahl von U darstellt, die eine ganze Zahl von 1 bis 3 ist, und H U, T, C oder A darstellt.

9. Nukleinsäurekonstrukt nach Anspruch 8, wobei die Basensequenz, darstellt durch SEQ ID NO: 147, eine Basensequenz, darstellt durch SEQ ID NO: 148, ist:
GGUAUAUUGGCGCCUUCGUGGAAUGUC (SEQ ID NO: 148).

10. Nukleinsäurekonstrukt nach Anspruch 6, wobei die Nukleinsäure (c) eine Nukleinsäure ist, die eine Basensequenz, darstellt durch eine der SEQ ID NOs: 2, 12, 14, 15 und 55, umfaßt.

11. Nukleinsäurekonstrukt nach einem der Ansprüche 1 bis 10, wobei die Anzahl der Histidine in dem Histidin-Tag 6 bis 10 beträgt.

12. Nukleinsäurekonstrukt nach einem der Ansprüche 1 bis 11, wobei das Nukleinsäurekonstrukt die kodierende Nukleinsäure eines Peptid-Tag an der 5'-Seite der Klonierungsregion umfaßt und die kodierende Nukleinsäure des Aptamers an der 3'-Seite der Klonierungsregion umfaßt.

13. Nukleinsäurekonstrukt nach einem der Ansprüche 1 bis 12, ferner umfassend eine kodierende Nukleinsäure des T7-Gen-Leader.

14. Nukleinsäurekonstrukt nach einem der Ansprüche 1 bis 13, wobei die kodierende Nukleinsäure des beliebigen Peptids in die Klonierungsregion insertiert ist.

15. Nukleinsäurekonstrukt nach einem der Ansprüche 1 bis 14, wobei das Nukleinsäurekonstrukt ein Screeningnukleinsäurekonstrukt zum Screenen eines Peptids ist, das an ein Ziel oder dessen kodierende Nukleinsäure binden kann.

16. Komplexbildungsverfahren zur Bildung eines Komplexes aus einem Fusionstranskript mit einer Basensequenz, die eine kodierende Nukleinsäure eines beliebigen Peptids, eine kodierende Nukleinsäure eines Peptid-Tag und eine kodierende Nukleinsäure eines Aptamers, das an das Peptid-Tag binden kann, umfaßt, und einem Fusionstranslationsprodukt, das das beliebige Peptid und das Peptid-Tag umfaßt, umfassend den Schritt des:
Exprimierens des Nukleinsäurekonstrukts nach einem der Ansprüche 1 bis 15, wobei das Nukleinsäurekonstrukt umfaßt:
eine Klonierungsregion, in die eine kodierenden Nukleinsäure eines beliebigen Peptids insertiert werden kann;
eine kodierende Nukleinsäure eines Peptid-Tag, wobei das Peptid-Tag ein Histidin-Tag ist; und
eine kodierende Nukleinsäure eines Aptamers, das an das Peptid-Tag binden kann;
wobei die kodierende Nukleinsäure des beliebigen Peptids in die Klonierungsregion insertiert ist.

17. Komplexbildungsverfahren nach Anspruch 16, wobei der Komplex *in vivo* gebildet wird.

18. Komplexbildungsverfahren nach Anspruch 16 oder 17, wobei das Nukleinsäurekonstrukt *in vivo* exprimiert wird.

19. Komplexbildungsverfahren nach einem der Ansprüche 16 bis 18, wobei das Nukleinsäurekonstrukt in einer lebenden Zelle exprimiert wird.

20. Komplexbildungsverfahren nach Anspruch 19, wobei die lebende Zelle *Escherichia coli* ist.

21. Komplexbildungsverfahren nach Anspruch 16, wobei der Komplex *in vitro* gebildet wird.

22. Komplexbildungsverfahren nach Anspruch 16 oder 21, wobei das Nukleinsäurekonstrukt *in vitro* exprimiert wird.

23. Komplexbildungsverfahren nach einem der Ansprüche 16 bis 22, wobei das Nukleinsäurekonstrukt ein Vektor ist.

24. Screeningverfahren zum Screenen eines Peptids, das an ein Ziel oder eine kodierende Nukleinsäure des Peptids bindet, umfassend die Schritte des:
(A) Bildens eines Komplexes durch das Komplexbildungsverfahren nach einem der Ansprüche 16 bis 23;
(B) Inkontaktbringens des Komplexes mit dem Ziel; und
(C) Gewinnens des Komplexes, der an das Target gebunden ist.

25. Screeningverfahren nach Anspruch 24, wobei in dem Schritt (B), das Ziel ein Ziel ist, das auf einer festen Phase immobilisiert ist.

26. Screeningverfahren nach Anspruch 24 oder 25, ferner umfassend den Schritt des:
(D) Synthetisierens der kodierenden Nukleinsäure des beliebigen Peptids unter Verwendung eines Transkripts der kodierenden Nukleinsäure des beliebigen Peptids in dem in Schritt (C) gewonnenen Komplexes als Templat.

27. Screeningverfahren nach Anspruch 26, wobei in dem Schritt (D) die kodierende Nukleinsäure des beliebigen Peptids durch eine Reverse Transkriptase-Polymerase-Kettenreaktion synthetisiert wird.

28. Screeningverfahren nach Anspruch 26 oder 27, wobei die in dem Schritt (D) erhaltene kodierende Nukleinsäure des beliebigen Peptids in die Klonierungsregion des Nukleinsäurekonstrukts nach einem der Ansprüche 1 bis 15 insertiert wird und die Schritte (A), (B) und (C) erneut durchgeführt werden.

29. Screeningverfahren nach Anspruch 28, wobei die Schritte (A), (B), (C) und (D) wiederholt durchgeführt werden.

30. Screeningverfahren nach einem der Ansprüche 26 bis 29, wobei eine Basensequenz der kodierenden Nukleinsäure des in Schritt (D) erhaltenen beliebigen Peptids bestimmt wird.

31. Screeningverfahren nach Anspruch 30, wobei eine Aminosäuresequenz des beliebigen Peptids, das an das Ziel binden kann, basierend auf der Basensequenz der kodierenden Nukleinsäure des beliebigen Peptids bestimmt wird.

32. Komplexbildungskit, das für das Komplexbildungsverfahren nach einem der Ansprüche 16 bis 23 verwendet wird, umfassend:
das Nukleinsäurekonstrukt nach einem der Ansprüche 1 bis 15.

33. Komplexbildungskit nach Anspruch 32, ferner umfassend eine lebende Zelle, die mit dem Nukleinsäurekonstrukt transfiziert wird.

34. Screeningkit, das für das Screeningverfahren nach einem der Ansprüche 24 bis 31 verwendet wird, umfassend:
das Nukleinsäurekonstrukt nach einem der Ansprüche 1 bis 15.

35. Screeningkit nach Anspruch 34, ferner umfassend eine lebende Zelle, die mit dem Nukleinsäurekonstrukt transfiziert wird.

## Revendications

1. Construction d'acide nucléique, comprenant :
une région de clonage destinée à être insérée avec un acide nucléique codant d'un peptide arbitraire ;
un acide nucléique codant d'une étiquette peptidique, l'étiquette peptidique étant une étiquette histidine ; et
un acide nucléique codant d'un aptamère qui peut être lié à l'étiquette peptidique ;
dans laquelle la construction d'acide nucléique est destinée à former un complexe d'un transcrit de fusion ayant une séquence de bases qui inclut l'acide nucléique codant d'un peptide arbitraire, l'acide nucléique codant d'une étiquette peptidique, et l'acide nucléique codant de l'aptamère et une traduction de fusion qui inclut le peptide arbitraire et l'étiquette peptidique.

2. Construction d'acide nucléique selon la revendication 1, dans laquelle la construction d'acide nucléique est capable de :
transcrire le transcrit de fusion ayant une séquence de bases qui inclut l'acide nucléique codant d'un peptide arbitraire pour être inséré dans la région de clonage, l'acide nucléique codant d'une étiquette peptidique, et l'acide nucléique codant de l'aptamère ; et
traduire la traduction de fusion qui inclut le peptide arbitraire et l'étiquette peptidique.

3. Construction d'acide nucléique selon la revendication 1 ou 2, dans laquelle la construction d'acide nucléique est un vecteur.

4. Construction d'acide nucléique selon la revendication 3, dans laquelle le vecteur est un vecteur d'expression de choc froid.

5. Construction d'acide nucléique selon l'une quelconque des revendications 1 à 3, dans laquelle chacun de l'acide nucléique codant d'une étiquette peptidique et de l'acide nucléique codant de l'aptamère est de l'ADN.

6. Construction d'acide nucléique selon l'une quelconque des revendications 1 à 5, dans laquelle l'aptamère est l'un quelconque des acides nucléiques (a), (b), (c) et (d) suivants :
(a) un acide nucléique qui inclut une séquence de bases représentée par SEQ ID NO: 17 :
GGUNnAYUmGGH (SEQ ID NO: 17),
dans laquelle N représente A, G, C, U ou T, n de Nn représente le nombre de N, qui est un entier de 1 à 3, Y représente U, T ou C, m de Um représente le nombre de U, qui est un entier de 1 à 3, et H représente U, T, C ou A ;
(b) un acide nucléique qui inclut une séquence de bases obtenue par substitution, délétion, addition ou insertion d'une ou plusieurs bases dans la séquence de bases montrée dans (a) et peut être lié à l'étiquette histidine ;
(c) un acide nucléique qui inclut une séquence de bases représentée par SEQ ID NO: 18 :
GGCGCCUUCGUGGAAUGUC (SEQ ID NO: 18) ;
(d) un acide nucléique qui inclut une séquence de bases obtenue par substitution, délétion, addition ou insertion d'une ou plusieurs bases dans la séquence de bases montrée dans (c) et peut être lié à l'étiquette histidine.

7. Construction d'acide nucléique selon la revendication 6, dans laquelle l'acide nucléique (a) est :
un acide nucléique qui inclut une séquence de bases représentée par l'une quelconque de SEQ ID NO: 89 à 104 ;
un acide nucléique qui inclut une séquence de bases représentée par l'une quelconque de SEQ ID NO: 1 à 16 ;
un acide nucléique qui inclut une séquence de bases représentée par l'une quelconque de SEQ ID NO: 105 à 114, 116 à 124 et 127 à 146 ;
un acide nucléique qui inclut une séquence de bases représentée par l'une quelconque de SEQ ID NO: 26 à 35, 37 à 45, 65 à 68, 19 à 25 et 48 à 56 ;
un acide nucléique qui inclut une séquence de bases représentée par l'une quelconque de SEQ ID NO: 2, 12, 14, 15 et 55 ; ou
un acide nucléique qui inclut une séquence de bases représentée par l'une quelconque de SEQ ID NO: 158 à 2302 et 2303 à 2312.

8. Construction d'acide nucléique selon la revendication 6, dans laquelle l'acide nucléique (a) est un acide nucléique qui inclut une séquence de bases représentée par SEQ ID NO: 147 :
GGUNnAYUmGGHGCCUUCGUGGAAUGUC (SEQ ID NO: 147),
dans laquelle N représente A, G, C, U ou T, n de Nn représente le nombre de N, qui est un entier de 1 à 3, Y représente U, T ou C, m de Um représente le nombre de U, qui est un entier de 1 à 3, et H représente U, T, C ou A.

9. Construction d'acide nucléique selon la revendication 8, dans laquelle la séquence de bases représentée par SEQ ID NO: 147 est une séquence de bases représentée par SEQ ID NO: 148 :
GGUAUAUUGGCGCCUUCGUGGAAUGUC (SEQ ID NO : 148).

10. Construction d'acide nucléique selon la revendication 6, dans laquelle l'acide nucléique (c) est un acide nucléique qui inclut une séquence de bases représentée par l'une quelconque de SEQ ID NO: 2, 12, 14, 15 et 55.

11. Construction d'acide nucléique selon l'une quelconque des revendications 1 à 10, dans laquelle le nombre d'histidines dans l'étiquette histidine est de 6 à 10.

12. Construction d'acide nucléique selon l'une quelconque des revendications 1 à 11, dans laquelle la construction d'acide nucléique comprend l'acide nucléique codant d'une étiquette peptidique du côté 5' de la région de clonage et comprend l'acide nucléique codant de l'aptamère du côté 3' de la région de clonage.

13. Construction d'acide nucléique selon l'une quelconque des revendications 1 à 12, comprenant en outre un acide nucléique codant de séquence de tête du gène T7.

14. Construction d'acide nucléique selon l'une quelconque des revendications 1 à 13, dans laquelle l'acide nucléique codant d'un peptide arbitraire est inséré dans la région de clonage.

15. Construction d'acide nucléique selon l'une quelconque des revendications 1 à 14, dans laquelle la construction d'acide nucléique est une construction d'acide nucléique de criblage permettant de cribler un peptide qui peut être lié à une cible ou son acide nucléique codant.

16. Procédé de formation d'un complexe destiné à former un complexe d'un transcrit de fusion ayant une séquence de bases qui inclut un acide nucléique codant d'un peptide arbitraire, un acide nucléique codant d'une étiquette peptidique, et un acide nucléique codant d'un aptamère qui peut être lié à l'étiquette peptidique et une traduction de fusion qui inclut le peptide arbitraire et l'étiquette peptidique, comprenant l'étape:
d'expression de la construction d'acide nucléique selon l'une quelconque des revendications 1 à 15, la construction d'acide nucléique comprenant :
une région de clonage destinée à être insérée avec un acide nucléique codant d'un peptide arbitraire ;
un acide nucléique codant d'une étiquette peptidique, dans lequel l'étiquette peptidique est une étiquette histidine ; et
un acide nucléique codant d'un aptamère qui peut être lié à l'étiquette peptide, l'acide nucléique codant d'un peptide arbitraire étant inséré dans la région de clonage.

17. Procédé de formation d'un complexe selon la revendication 16, dans lequel le complexe est formé *in vivo*.

18. Procédé de formation d'un complexe selon la revendication 16 ou 17, dans lequel la construction d'acide nucléique est exprimée *in vivo.*

19. Procédé de formation d'un complexe selon l'une quelconque des revendications 16 à 18, dans lequel la construction d'acide nucléique est exprimée dans une cellule vivante.

20. Procédé de formation d'un complexe selon la revendication 19, dans lequel la cellule vivante est *Escherichia coli.*

21. Procédé de formation d'un complexe selon la revendication 16, dans lequel le complexe est formé *in vitro.*

22. Procédé de formation d'un complexe selon la revendication 16 ou 21, dans lequel la construction d'acide nucléique est exprimée *in vitro.*

23. Procédé de formation d'un complexe selon l'une quelconque des revendications 16 à 22, dans lequel la construction d'acide nucléique est un vecteur.

24. Procédé de criblage destiné à cribler un peptide qui peut être lié à une cible ou un acide nucléique codant du peptide, comprenant les étapes de :
(A) formation d'un complexe par le procédé de formation d'un complexe selon l'une quelconque des revendications 16 à 23 ;
(B) mise en contact du complexe avec la cible ; et
(C) récupération du complexe qui est lié à la cible.

25. Procédé de criblage selon la revendication 24, dans lequel, à l'étape (B), la cible est une cible qui est immobilisée sur une phase solide.

26. Procédé de criblage selon la revendication 24 ou 25, comprenant en outre l'étape de :
(D) synthèse de l'acide nucléique codant d'un peptide arbitraire à l'aide d'un transcrit de l'acide nucléique codant d'un peptide arbitraire dans le complexe récupéré à l'étape (C) en tant que matrice.

27. Procédé de criblage selon la revendication 26, dans lequel, à l'étape (D), l'acide nucléique codant d'un peptide arbitraire est synthétisé par une réaction de polymérisation en chaîne après transcription inverse.

28. Procédé de criblage selon la revendication 26 ou 27, dans lequel l'acide nucléique codant d'un peptide arbitraire obtenu à l'étape (D) est inséré dans la région de clonage de la construction d'acide nucléique selon l'une quelconque des revendications 1 à 15, et les étapes (A), (B) et (C) sont à nouveau réalisées.

29. Procédé de criblage selon la revendication 28, dans lequel les étapes (A), (B), (C) et (D) sont réalisées de façon répétée.

30. Procédé de criblage selon l'une quelconque des revendications 26 à 29, dans lequel une séquence de bases de l'acide nucléique codant d'un peptide arbitraire obtenu à l'étape (D) est déterminée.

31. Procédé de criblage selon la revendication 30, dans lequel une séquence d'acides aminés du peptide arbitraire qui peut être lié à la cible est déterminée en se basant sur la séquence de bases de l'acide nucléique codant d'un peptide arbitraire.

32. Kit de formation de complexe utilisé pour le procédé de formation d'un complexe selon l'une quelconque des revendications 16 à 23, comprenant :
la construction d'acide nucléique selon l'une quelconque des revendications 1 à 15.

33. Kit de formation de complexe selon la revendication 32, comprenant en outre une cellule vivante à transfecter avec la construction d'acide nucléique.

34. Kit de criblage utilisé pour le procédé de criblage selon l'une quelconque des revendications 24 à 31, comprenant :
la construction d'acide nucléique selon l'une quelconque des revendications 1 à 15.

35. Kit de criblage selon la revendication 34, comprenant en outre une cellule vivante à transfecter avec la construction d'acide nucléique.


### SEQUENCE LISTING

<110> SVA - Swedish National Veterinary Institute
<120> Genomic typing
<130> P8716PC00
<150> SE 0950824-3
   <151> 2009-11-03
<160> 61
<170> PatentIn version 3.3
<210> 1
   <211> 53
   <212> DNA
   <213> Avian influenza virus
<400> 1
   cgggaactat caccaaacaa caccccgcaa ggagaragaa raagaaaaaa gag 53
<210> 2
   <211> 52
   <212> DNA
   <213> Avian influenza virus
<400> 2
   cgggaactat caccaaacaa caccccgaag gggagagaag aagaaaaaag ag 52
<210> 3
   <211> 53
   <212> DNA
   <213> Avian influenza virus
<400> 3
   cgggaactat caccaaacaa caccccgccc tcaaagaaaa agaaaaacaa gag 53
<210> 4
   <211> 53
   <212> DNA
   <213> Avian influenza virus
<400> 4
   cgggaactat caccaaacaa caccccgcaa agagaragaa gaarraaaag arg 53
<210> 5
   <211> 51
   <212> DNA
   <213> Avian influenza virus
<400> 5
   cgggaactat caccaaacaa caccccgaag agaragaaga arraagcgar g 51
<210> 6
   <211> 49
   <212> DNA
   <213> Avian influenza virus
<400> 6
   cgggaactat caccaaacaa caccccggtc cctcaaagga agaaaagag 49
<210> 7
   <211> 52
   <212> DNA
   <213> Avian influenza virus
<400> 7
   cgggaactat caccaaacaa caccccggag akaraagaag aaaaaagmga gg 52
<210> 8-
   <211> 52
   <212> DNA
   <213> Avian influenza virus
<400> 8
   cgggaactat caccaaacaa caccccggag akaraagaag aaaaaagagr gg 52
<210> 9
   <211> 53
   <212> DNA
   <213> Avian influenza virus
<400> 9
   cgggaactat caccaaacaa caccccgrga gakaraagaa gaaaraarag agg 53
<210> 10
   <211> 53
   <212> DNA
   <213> Avian influenza virus
<400> 10
   cgggaactat caccaaacaa caccccgrga gakaraagaa gaaaaargar agg 53
<210> 11
   <211> 53
   <212> DNA
   <213> Avian influenza virus
<400> 11
   cgggaactat caccaaacaa caccccggga gagagaagaa gaaaaaagag agg 53
<210> 12
   <211> 51
   <212> DNA
   <213> Avian influenza virus
<400> 12
   cgggaactat caccaaacaa caccccgccc tcaaagaaga aaaaaaagag g 51
<210> 13
   <211> 53
   <212> DNA
   <213> Avian influenza virus
<400> 13
   cgggaactat caccaaacaa caccccgcct caaagaaraa gaaaaaarag agg 53
<210> 14
   <211> 44
   <212> DNA
   <213> Avian influenza virus
<400> 14
   cgggaactat caccaaacaa caccccggga tcgcgtgtga ggag 44
<210> 15
   <211> 49
   <212> DNA
   <213> Avian influenza virus
<400> 15
   cgggaactat caccaaacaa caccccgcca aaaaaaggaa aaaaagagg 49
<210> 16
   <211> 43
   <212> DNA
   <213> Avian influenza virus
<400> 16
   cgggaactat caccaaacaa caccccggat cgcgsgtgag gag 43
<210> 17
   <211> 47
   <212> DNA
   <213> Avian influenza virus
<400> 17
   cgggaactat caccaaacaa caccccgcca aagaggagga ggagagg 47
<210> 18
   <211> 50
   <212> DNA
   <213> Avian influenza virus
<400> 18
   cgggaactat caccaaacaa caccccgcca aaaaagagga aaaagagagg 50
<210> 19
   <211> 49
   <212> DNA
   <213> Avian influenza virus
<400> 19
   cgggaactat caccaaacaa caccccgttc caaaaaggaa aaagagagg 49
<210> 20
   <211> 52
   <212> DNA
   <213> Avian influenza virus
<400> 20
   cgggaactat caccaaacaa caccccgaaa ctccaaaaag aagaarmaga gg 52
<210> 21
   <211> 53
   <212> DNA
   <213> Avian influenza virus
<400> 21
   cgggaactat caccaaacaa caccccgcca aaaaagaaaa agaaaaagag agg 53
<210> 22
   <211> 50
   <212> DNA
   <213> Avian influenza virus
<400> 22
   cgggaactat caccaaacaa caccccgccc aaaaaaagaa gaaaragagg 50
<210> 23
   <211> 53
   <212> DNA
   <213> Avian influenza virus
<400> 23
   cgggaactat caccaaacaa caccccggaa attccaaaaa agaaaaagag agg 53
<210> 24
   <211> 53
   <212> DNA
   <213> Avian influenza virus
<400> 24
   cgggaactat caccaaacaa caccccgaga gagggaggaa gaagaaaaag agg 53
<210> 25
   <211> 52
   <212> DNA
   <213> Avian influenza virus
<400> 25
   cgggaactat caccaaacaa caccccgaaa gagagagaag gaaaaagaga gg 52
<210> 26
   <211> 51
   <212> DNA
   <213> Avian influenza virus
<400> 26
   cgggaactat caccaaacaa caccccgaaa aaagaaaaag aaaaagaggc c 51
<210> 27
   <211> 48
   <212> DNA
   <213> Avian influenza virus
<400> 27
   cgggaactat caccaaacaa caccccgccc gagaargaga aagagagg 48
<210> 28
   <211> 51
   <212> DNA
   <213> Avian influenza virus
<400> 28
   cgggaactat caccaaacaa caccccgttc caaaaagaga acaaaaagag g 51
<210> 29
   <211> 52
   <212> DNA
   <213> Avian influenza virus
<400> 29
   cgggaactat caccaaacaa caccccgaaa tcccaaagag aaagaaaaga gg 52
<210> 30
   <211> 50
   <212> DNA
   <213> Avian influenza virus
<400> 30
   cgggaactat caccaaacaa caccccgccc raagaagaga gagaagagag 50
<210> 31
   <211> 45
   <212> DNA
   <213> Avian influenza virus
<400> 31
   cgggaactat caccaaacaa caccccggcg caggcagaaa agagg 45
<210> 32
   <211> 46
   <212> DNA
   <213> Avian influenza virus
<400> 32
   cgggaactat caccaaacaa caccccgaca tgcgcaaaaa aagagg 46
<210> 33
   <211> 50
   <212> DNA
   <213> Avian influenza virus
<400> 33
   cgggacaaca aaccactatc aaccccgaty cctcararag raacaaragg 50
<210> 34
   <211> 49
   <212> DNA
   <213> Avian influenza virus
<400> 34
   cgggacaaca aaccactatc aaccccgtyc ctcararagr aacaaragg 49
<210> 35
   <211> 50
   <212> DNA
   <213> Avian influenza virus
<400> 35
   cgggacaaca aaccactatc aaccccggty cctcararag agacaaragg 50
<210> 36
   <211> 50
   <212> DNA
   <213> Avian influenza virus
<400> 36
   cgggacaaca aaccactatc aaccccggty cctcararag rtacaaragg 50
<210> 37
   <211> 48
   <212> DNA
   <213> Avian influenza virus
<400> 37
   cgggacaaca aaccactatc aaccccgccc tcagagagag acgagagg 48
<210> 38
   <211> 48
   <212> DNA
   <213> Avian influenza virus
<400> 38
   cgggacaaca aaccactatc aaccccgccc tcaacgagaa acaagagg 48
<210> 39
   <211> 48
   <212> DNA
   <213> Avian influenza virus
<400> 39
   cgggacaaca aaccactatc aaccccgycc tcaaarggag acaagagg 48
<210> 40
   <211> 48
   <212> DNA
   <213> Avian influenza virus
<400> 40
   cgggacaaca aaccactatc aaccccgcct gaaaytccaa aagraagg 48
<210> 41
   <211> 50
   <212> DNA
   <213> Avian influenza virus
<400> 41
   cgggacaaca aaccactatc aaccccgcct gaaaytccaa aaggaaaagg 50
<210> 42
   <211> 48
   <212> DNA
   <213> Avian influenza virus
<400> 42
   cgggacaaca aaccactatc aaccccgyga aattccaaar ggaagagg 48
<210> 43
   <211> 47
   <212> DNA
   <213> Avian influenza virus
<400> 43
   cgggacaaca aaccactatc aaccccgcga artyccaaar ggragag 47
<210> 44
   <211> 47
   <212> DNA
   <213> Avian influenza virus
<400> 44
   cgggacaaca aaccactatc aaccccggaa aycccaaakg gaagagg 47
<210> 45
   <211> 46
   <212> DNA
   <213> Avian influenza virus
<400> 45
   cgggacaaca aaccactatc aaccccgcyg aartcccraa kggaar 46
<210> 46
   <211> 49
   <212> DNA
   <213> Avian influenza virus
<400> 46
   cgggacaaca aaccactatc aaccccgcct garaytccaa aaggragag 49
<210> 47
   <211> 48
   <212> DNA
   <213> Avian influenza virus
<400> 47
   cgggacaaca aaccactatc aaccccgctg araytccaaa gggragag 48
<210> 48
   <211> 49
   <212> DNA
   <213> Avian influenza virus
<400> 48
   cgggacaaca aaccactatc aaccccgctg aaattccaaa aggaagagg 49
<210> 49
   <211> 52
   <212> DNA
   <213> Avian influenza virus
<400> 49
   cgggacaaca aaccactatc aaccccgaat gtycctcaaa ragaaacaag ag 52
<210> 50
   <211> 49
   <212> DNA
   <213> Avian influenza virus
<400> 50
   cgggacaaca aaccactatc aaccccggtt cctcaaagag acacaaggg 49
<210> 51
   <211> 49
   <212> DNA
   <213> Avian influenza virus
<400> 51
   cgggacaaca aaccactatc aaccccgttc cccaaagaga aaaaagagg 49
<210> 52
   <211> 48
   <212> DNA
   <213> Avian influenza virus
<400> 52
   cgggacaaca aaccactatc aaccccgtcc tcaaagggaa acaagagg 48
<210> 53
   <211> 48
   <212> DNA
   <213> Avian influenza virus
<400> 53
   cgggacaaca aaccactatc aaccccgtgg agtcccaaga aaaagagg 48
<210> 54
   <211> 49
   <212> DNA
   <213> Avian influenza virus
<400> 54
   cgggacaaca aaccactatc aaccccgcgg aaaatccraa gamkagagg 49
<210> 55
   <211> 49
   <212> DNA
   <213> Avian influenza virus
<400> 55
   cgggacaaca aaccactatc aaccccgctg aaatcccaaa gaaaagagg 49
<210> 56
   <211> 21
   <212> DNA
   <213> Avian influenza virus
<400> 56
   atgtccgaga tatgttaagc a 21
<210> 57
   <211> 20
   <212> DNA
   <213> Avian influenza virus
<400> 57
   tttgtaatct gcagcagttc 20
<210> 58
   <211> 27
   <212> DNA
   <213> Avian influenza virus
<400> 58
   cctccagart atgcmtayaa aattgtc 27
<210> 59
   <211> 23
   <212> DNA
   <213> Avian influenza virus
<400> 59
   taccaaccgt ctaccatkcc ytg 23
<210> 60
   <211> 27
   <212> DNA
   <213> Avian influenza virus
<400> 60
   cgggaactat caccaaacaa caccccg 27
<210> 61
   <211> 27
   <212> DNA
   <213> Avian influenza virus
<400> 61
   cgggacaaca aaccactatc aaccccg 27